(19) Europäisches Patentamt
European Patent Office
Office européen des brevets

(11) EP 3 115 067 A1

(12) # EUROPEAN PATENT APPLICATION

(43) Date of publication:
**11.01.2017 Bulletin 2017/02**

(21) Application number: **16001593.9**

(22) Date of filing: **15.05.2014**

(51) Int Cl.:
***A61L 2/18*** *(2006.01)* ***A01N 25/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.05.2013 JP 2013106191**
**11.11.2013 JP 2013232949**
**11.11.2013 JP 2013232951**
**24.04.2014 JP 2014090209**

(62) Document number(s) of the earlier application(s) in accordance with Art. 76 EPC:
**14728654.6 / 2 999 490**

(71) Applicant: **Honbu Sankei Co., Ltd.**
**Osaka-shi Osaka 540-0001 (JP)**

(72) Inventor: **GODA, Hisataka**
**Osaka-shi Osaka, 541-0048 (JP)**

(74) Representative: **Müller-Boré & Partner Patentanwälte PartG mbB**
**Friedenheimer Brücke 21**
**80639 München (DE)**

Remarks:
This application was filed on 19-07-2016 as a divisional application to the application mentioned under INID code 62.

(54) **LONG-TERM PRESERVATION AND APPLICATION OF CHLOROUS ACID AQUEOUS SOLUTION FORMULATION**

(57) The present invention provides a method of preserving a chlorous acid aqueous solution for a long period of time, comprising maintaining the chlorous acid aqueous solution at 10°C or lower.

Fig. 11

30th Day
**Sterilizing Effect Examination Chart (pH3.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 500ppm | − | − | − | − |
| 0.010% | 400ppm | − | − | − | − |
| 0.008% | 350ppm | + | + | − | − |
| 0.007% | 300ppm | + | + | + | + |
| 0.006% | 200ppm | + | + | + | + |
| 0.005% | 250ppm | + | + | + | + |
| 0.004% | 200ppm | + | + | + | + |
| 0.003% | 150ppm | + | + | + | + |
| 0.002% | 100ppm | + | + | + | + |
| 0.001% | 50ppm | + | + | + | + |
| 0.000% | 0ppm | + | + | + | + |

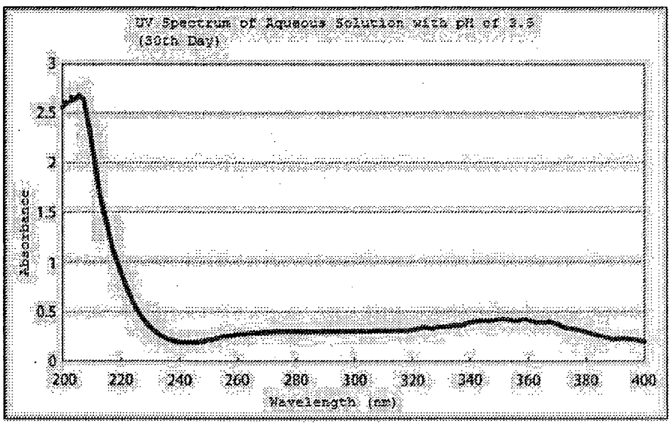


EP 3 115 067 A1

**Sterilizing Effect Examination Chart (pH6.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | − | − | − | − |
| 0.008% | 80ppm | − | − | − | − |
| 0.007% | 70ppm | − | − | − | − |
| 0.006% | 60ppm | − | − | − | − |
| 0.005% | 50ppm | − | − | − | − |
| 0.004% | 40ppm | − | − | − | − |
| 0.003% | 30ppm | + | + | + | − |
| 0.002% | 20ppm | + | + | + | + |
| 0.001% | 10ppm | + | + | + | + |
| 0.000% | 0ppm | + | + | + | + |

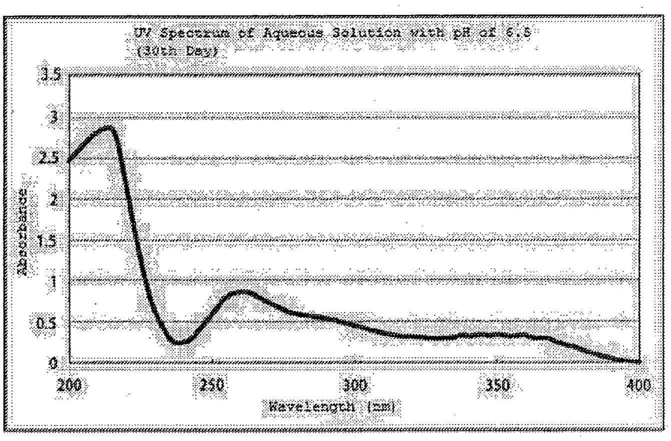


**Sterilizing Effect Examination Chart (pH8.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | + | − | − | − |
| 0.008% | 80ppm | + | + | − | − |
| 0.007% | 70ppm | + | + | + | + |
| 0.006% | 60ppm | + | + | + | + |
| 0.005% | 50ppm | + | + | + | + |
| 0.004% | 40ppm | + | + | + | + |
| 0.003% | 30ppm | + | + | + | + |
| 0.002% | 20ppm | + | + | + | + |
| 0.001% | 10ppm | + | + | + | + |
| 0.000% | 0ppm | + | + | + | + |

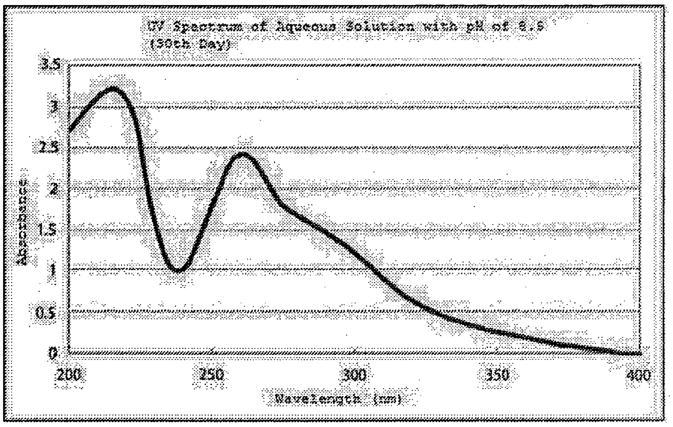

## Description

The present invention relates to a chlorous acid aqueous solution that can be preserved for a long period of time and a sterilizing agent that can be preserved for a long period of time using the same. Further, the present invention relates to a novel application of a chlorous acid aqueous solution.

[Background Art]

A chlorous acid aqueous solution has drawn attention as a food additive. However, it is an issue that a chlorous acid aqueous solution is difficult to manufacture and, even if the manufacture was possible, preservation under normal conditions is not possible.

The inventor has discovered a chlorous acid aqueous solution and a manufacturing method thereof. A sterilizing effect against E. coli was verified and a patent application therefor was filed (Patent Literature 1).

[Citation List]

[Patent Literature]

[PTL 1]

Patent Literature 1: International Publication No. WO 2008-026607

[Summary of Invention]

[Solution to Problem]

In the present invention, a technique that enables unexpected and significant long termpreservation of a chlorous acid aqueous solution has been found and provided. Further, the inventor has found a novel application of a chlorous acid aqueous solution and provides the same. Thus, the present invention provides the following.

(1) A sterilizing agent comprising a chlorous acid aqueous solution, metal hydroxide, and metal phosphate.
(2) The sterilizing agent of (1), wherein the metals comprise potassium.
(3) The sterilizing agent of (1) or (2), wherein the metal hydroxide comprises sodiumhydroxide and/or potassiumhydroxide and the metal phosphate comprises sodium phosphate and/or potassium phosphate.
(4) The sterilizing agent according to any one of (1) to (3), wherein pH is 3.2 or higher and lower than 7.0.
(5) The sterilizing agent according to anyone of (1) - (4), wherein pH is 5.0 to 7.0.
(6) The sterilizing agent according to anyone of (1) - (5), wherein pH is about 5.5.
(7) The sterilizing agent according to any one of (1) - (6), wherein the chlorous acid aqueous solution is 0.25%-75% (when converted into chlorous acid, chlorous acid concentration of 72.0% chlorous acid aqueous solution is 30000 ppm), potassium dihydrogen phosphate is 0.70% to 13.90%, and potassium hydroxide is 0.10% to 5.60%.
(8) The sterilizing agent according to any one of (1) - (7), wherein sodium hydroxide and potassium hydroxide are 0.1 N to 1.0 N and buffer pH of sodium phosphate and potassium phosphate is 3.2 or higher and lower than 7.0.
(9) An article impregnated with the sterilizing agent according to any one of (1) to (8).
(10) The article of (9), wherein the article is selected from a sheet, film, patch, brush, nonwoven fabric, paper, fabric, absorbent cotton, and sponge.
(11) The article of (9) or (10), wherein the article is an article in a form of a sheet with one layer or two or more layers, wherein a chlorous acid aqueous solution is impregnated at a concentration of 3000 ppm or higher in the sterilizing agent.
(12) The article of (11), wherein the article in a form of a sheet is made of cotton.
(13) The article according to any one of (9) - (12), wherein the article is an article in a form of a sheet with three or more layers.
(14) A method of preserving a chlorous acid aqueous solution for a long period of time, comprising maintaining the chlorous acid aqueous solution at 10°C or lower.

The inventor has found a novel application of a chlorous acid aqueous solution and provides the same. Further, the inventor has found a technique that enables unexpected and significant long term preservation of a chlorous acid aqueous solution and provides the same. The present invention also provides the following.

<Application in preventing secondary contamination>

(1) A sterilizing agent for preventing secondary contamination, comprising a chlorous acid aqueous solution.
(2) An article impregnated with a chlorous acid aqueous solution for preventing secondary contamination.
(3) The article of (2), wherein the article is selected from a sheet, film, patch, brush, nonwoven fabric, paper, fabric, absorbent cotton, and sponge.
(4) The article of (2) or (3), wherein the article is a sheet with one layer or two or more layers, and chlorous acid is impregnated at a concentration of 3000 ppm or higher.
(5) The article of (4), wherein the sheet is made of cotton.
(6) The article according to any one of (2) to (5), wherein the article is a sheet with three or more layers.

<Application in sterilizing a floor surface>

(7) An article impregnated with a chlorous acid aqueous solution for sterilizing a floor surface.
(8) The article of (7), wherein the article is selected from a sheet, film, patch, brush, nonwoven fabric, paper, fabric, absorbent cotton, and sponge.
(9) The article of (7) or (8), wherein chlorus acid is impregnated at a concentration of 1000 ppm or higher.
(10) The article of (8) or (9), wherein the sheet is made of cotton.

<Application in handling environment (odor)>

(11) An agent for removing odor comprising a chlorous acid aqueous solution.
(12) The agent for removing odor of (11), wherein the chlorous acid aqueous solution is comprised at at least 400 ppm.
(13) The agent for removing odor of (11) or (12), wherein the odor comprises at least one odor selected from the group consisting of ammonium odor, amine odor and sulfide odor.
(14) The agent for removing odor according to any one of (11) to (13) having an odor removing effect even when 3 hours or more has elapsed after odor removing treatment.
(15) The agent for removing odor according to any one of (11) to (14) having an odor removing effect even when 9 hours or more has elapsed after odor removing treatment.
(16) An article impregnated with a chlorous acid aqueous solution for removing odor.
(17) The article of (16), wherein the odor comprises at least one odor selected from the group consisting of ammonium odor, amine odor and sulfide odor.
(18) The article of (16) or (17) having an odor removing effect even when 3 hours or more has elapsed after odor removing treatment.
(19) The article according to any one of (16) to (18) having an odor removing effect even when 9 hours or more has elapsed after odor removing treatment.
(20) The article according to any one of (16) to (19), wherein the article is selected froma sheet, film, patch, brush, nonwoven fabric, paper, fabric, absorbent cotton, and sponge.
(21) The article according to any one of (16) to (20), wherein the article is a sheet and chlorous acid is impregnated at a concentration of 500 ppm or higher.
(22) The article of (20) or (21), wherein the sheet is made of cotton.

<Application in washing and removing microbes from the body (hands and fingers)>

(23) An agent for removingmicrobes adhering to abody, comprising a chlorous acid aqueous solution.
(24) An article impregnated with a chlorous acid aqueous solution for removing microbes adhering to the body.
(25) The article of (24), wherein the article is selected from a sheet, film, patch, brush, nonwoven fabric, paper, fabric, absorbent cotton, and sponge.
(26) The article of (24) or (25), wherein the article is a sheet and chlorous acid is impregnated at a concentration of 3000 ppm or higher.

(27) The article of (25) or (26), wherein the sheet is made of cotton.

Additional embodiments and advantages of the present invention are recognized by those skilled in the art if the following Detailed Description is read and understood as needed. In the present invention, one or more features described above are intended to be able to provide combinations that were explicitly described as well as combinations thereof. The additional embodiments and advantages of the present invention are recognized by those skilled in the art if the following Detailed Description is read and understood as needed.

[Advantageous Effects of Invention]

According to the present invention, a technique for long term preservation and a novel application of a useful agent, a chlorous acid aqueous solution, are provided, whereby the potential for extensive utilization in the food industry, clinical practice and the like was further increased.

[Brief Description of Drawings]

[fig. 1]
Figure 1 shows a schematic diagram of a manufacturing plant that was used in the Examples. Each of the symbols is as follow. 1: sulfuric acid input port, 2: hydrogen peroxide input port, 3: reaction bath, 4: collection bath, 5: gas adsorption bath, 6: gas washing bath, 7: air pump, 8: aeration value/chloric acid input port, 9: agitation motor, A: valve for the sulfuric acid input port, B: valve for hydrogen peroxide input port, C: air pump faucet, D: valve for discharging a reaction fluid, E: trifurcated faucet, G: aeration valve.
[fig. 2]
Figure 2 is a spectrograph of confirmation test (2) in analyzing components of the chlorous acid aqueous solution with pH of 8.5 in Example 1.
[fig. 3]
Figure 3 is a spectrograph of confirmation test (2) in analyzing components of the chlorous acid aqueous solution with pH of 6.5 in Example 1.
[fig. 4]
Figure 4 is a spectrograph of confirmation test (2) in analyzing components of the chlorous acid aqueous solution with pH of 3.5 in Example 1.
[fig. 5]
Figure 5 shows UV spectrums immediately after the manufacture of the chlorous acid aqueous solution (pH 3.0) manufactured in Example 1.
[fig. 6]
Figure 6 shows UV spectrums 30 days after the manufacture of the chlorous acid aqueous solution (pH 3.0) manufactured in Example 1.
[fig. 7]
Figure 7 shows UV spectrums immediately after the manufacture of the chlorous acid aqueous solution (pH 9.0) manufactured in Example 1.
[fig. 8]
Figure 8 shows UV spectrums 30 days after the manufacture of the chlorous acid aqueous solution (pH 3.0) manufactured in Example 1.
[fig. 9]
Figure 9 shows sterilizing effect examination charts (phenol and chlorous acid concentrations) together with UV spectrums for pH of 3.5, 6.5, and 8.5 immediately after manufacture.
[fig. 10]
Figure 10 shows sterilizing effect examination charts (phenol and chlorous acid concentrations) together with UV spectrums for pH of 3.5, 6.5, and 8.5 after 10 days from manufacture.
[fig. 11]
Figure 11 shows sterilizing effect examination charts (phenol and chlorous acid concentrations) together with UV spectrums for pH of 3.5, 6.5, and 8.5 after 30 days from manufacture.
[fig. 12]
Figure 12 shows a phenol coefficient immediately after manufacture, a phenol coefficient 10 days after manufacture, and a phenol coefficient 30 days after manufacture at pH of 2.0 to 9.0 for every 0.5.
[fig. 13]

Figure 13 is a spectrograph of confirmation test (2) in analyzing components of the chlorous acid aqueous solution formulation manufactured with the chlorous acid aqueous solution having pH of 3.5 in Example 1.
[fig. 14]
Figure 14 shows chronological changes in available chlorine after impregnating a treatment sheet with each diluent of Example 4.
[fig. 15]
Figure 15 shows pictures (from left: rubber mattress, tatami mattress, and carpet) of the procedure in Example 6.
[fig. 16]
Figure 16 shows a representative structure that is used in the method of measuring odor used in Example 11.
[fig. 17]
Figure 17 shows a glass container that was used in place of an air bag in the Examples.
[fig. 18]
Figure 18 shows results of examining deodorizing effects on ammonia odor. The rhombuses indicate the control (Cont), white squares indicate distilled water, white triangles indicate sodium hypochlorite (400 ppm), and black squares indicate chlorous acid aqueous solution (400 ppm). The vertical axis indicates the concentration of ammonium remaining in the air (ppm) after correction, and the horizontal axis indicates the elapsed time (before spraying; time after spraying, 0 hour, 3 hours, 6 hours, 9 hours, 24 hours).
[fig. 19]
Figure 19 shows results of examining deodorizing effects on amine odor (methyl mercaptan) (first time). The rhombuses indicate the control (Cont), white squares indicate distilled water, white triangles indicate sodium hypochlorite (400 ppm), and black squares indicate chlorous acid aqueous solution (400 ppm). The vertical axis indicates the concentration of methyl mercaptan remaining in the air (ppm) after correction, and the horizontal axis indicates the elapsed time (before spraying; time after spraying 0 hour, 3 hours, 6 hours, 9 hours, 24 hours) .
[fig. 20]
Figure 20 shows results of examining deodorizing effects on amine odor (methyl mercaptan) (second time). The rhombuses indicate the control (Cont), white squares indicate distilled water, white triangles indicate sodium hypochlorite (400 ppm), and black squares indicate chlorous acid aqueous solution (400 ppm). The vertical axis indicates the concentration of methyl mercaptan remaining in the air (ppm) after correction, and the horizontal axis indicates the elapsed time (before spraying; time after spraying 0 hour, 3 hours, 6 hours, 9 hours, 24 hours) .
[fig. 21]
Figure 21 shows results of examining deodorizing effects on hydrogen sulfide odor (first time). The rhombuses indicate the control (Cont), white squares indicate distilledwater, white triangles indicate sodium hypochlorite (400 ppm), and black squares indicate chlorous acid aqueous solution (400 ppm) . The vertical axis indicates the concentration of hydrogen sulfide remaining in the air (ppm) after correction, and the horizontal axis indicates the elapsed time (before spraying; time after spraying, 0 hour, 3 hours, 6 hours, 9 hours, 24 hours).
[fig. 22]
Figure 22 shows results of examining deodorizing effects on hydrogen sulfide odor (second time). The rhombuses indicate the control (Cont), white squares indicate distilledwater, white triangles indicate sodium hypochlorite (400 ppm), and black squares indicate chlorous acid aqueous solution (400 ppm) . The vertical axis indicates the concentration of hydrogen sulfide remaining in the air (ppm) after correction, and the horizontal axis indicates the elapsed time (before spraying; time after spraying, 0 hour, 3 hours, 6 hours, 9 hours, 24 hours).

[Description of Embodiments]

The present invention is described below. Throughout the entire specification, a singular expression shouldbe understood as encompassing the concept thereof in a plural form unless specifically noted otherwise. Thus, singular articles (e.g., "a", "an", "the" and the like in case of English) should be understood as encompassing the concept thereof in a plural form unless specifically noted otherwise. Further, the terms used herein should be understood as being used in the meaning that is commonly used in the art, unless specifically noted otherwise. Thus, unlessdefinedotherwise, all terminologies and scientific technical terms that are used herein have the same meaning as the terms commonly understood by those skilled in the art to which the present invention belongs. In case of a contradiction, the present specification (including the definitions) takes precedence.
Herein, "stability" of a chlorous acid aqueous solution refers to a property of retaining a sterilizing or disinfecting effect after being preserved.
Herein, "antimicrobial (action)" refers to suppression of growth of microorganisms such as mold, microbes, or viruses that are pathogenic or harmful. A substance having antimicrobial action is referred to as an antimicrobial agent.
Herein, narrowly-defined "sterilizing (action)" refers to killing of microorganisms such as mold, microbes, or viruses that are pathogenic or harmful. A substance having sterilizing action is referred to as a narrowly-defined sterilizing

agent.

Antimicrobial action and sterilizing action are together referred to as disinfecting (action), which is used herein in a broad concept including antimicrobial action and sterilizing action unless specifically noted otherwise. Thus, a substance having antimicrobial action and sterilizing action is generally referred herein as a "sterilizing agent", which is understood as an agent having both antimicrobial action and a narrowly defined sterilizing action in general use herein.

Herein, an article is any article that can be impregnated with a chlorous acid aqueous solution to be used in sterilization or the like, including medical devices. A sheet, film, patch, brush, nonwoven fabric, paper, fabric, absorbent cotton, sponge and the like are also examples thereof, but the article is not limited thereto. Further, any material may be used as long as a chlorous acid aqueous solution can be impregnated therein.

(Chlorous Acid Aqueous Solution and Manufacturing Example Thereof)

The chlorous acid aqueous solution used in the present invention has a feature that was discovered by the inventors. A chlorous acid aqueous solution manufactured by any method, such as known manufacturing methods described in Patent Literature 1, can be used. It is possible to mix and use, for example, an aquesous solution with 72.00% in chlorous acid aqueous solution (30000 ppm as chlorous acid concentration), 1.70% in potassium dihydrogen phosphate, 0.50% in potassium hydroxide, and 25.80% in purified water, as a typical constitution (scheduled to be sold under the name "AUTOLOC Super" by the Applicant), but the constitution is not limited thereto. The chlorous acid aqueous solution may be 0.25%-75%, potassium dihydrogen phosphate may be 0.70%-13.90%, and potassium hydroxide may be 0.10%-5.60%. It is possible to use sodium dihydrogen phosphate instead of potassium dihydrogen phosphate, and sodium hydroxide instead of potassium hydroxide. This agent reduces the decrease of chlorous acid due to contact with an organic matter under acidic conditions. However, the sterilizing effect is retained. In addition, very little chlorine gas is generated. Further, the agent also has a feature of inhibiting amplification of odor frommixing chlorine and an organic matter.

In one embodiment, the chlorous acid aqueous solution that is used in the present invention can be produced by adding and reacting sulfuric acid or an aqueous solution thereof to a sodium chlorate aqueous solution in an amount and concentration at which the pH value of the sodium chlorate aqueous solution can be maintained at 3.4 or lower to generate chloric acid, and subsequently adding hydrogen peroxide in an amount equivalent to or greater than the amount required for a reduction reaction of the chloric acid.

Further, in another embodiment, the chlorous acid aqueous solution that is used in the present invention can be produced from adding one compound from inorganic acids or inorganic acid salts, two or more types of compounds therefrom, or a combination thereof to an aqueous solution, inwhich chlorous acid is produced by adding and reacting sulfuric acid or an aqueous solution thereof to a sodium chlorate aqueous solution in an amount and concentration at which the pH value of the sodium chlorate aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Furthermore, in another embodiment, the chlorous acid aqueous solution that is used in the present invention can be produced by adding one compound from inorganic acids or inorganic acid salts or organic acids or organic acid salts, two or more types of compounds therefrom, or a combination thereof to an aqueous solution, in which chlorous acid is produced by adding and reacting sulfuric acid or an aqueous solution thereof to a sodium chlorate aqueous solution in an amount and concentration at which the pH value of the sodium chlorate aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Further still, in another embodiment, the chlorous acid aqueous solution that is used the present invention can be produced by adding one compound from inorganic acids or inorganic acid salts or organic acids or organic salts, two or more types of compounds therefrom, or a combination thereof after adding one compound from inorganic acids or inorganic acid salts, two or more types of compounds therefrom or a combination thereof to an aqueous solution, in which chlorous acid is produced by adding and reacting sulfuric acid or an aqueous solution thereof to a sodium chlorate aqueous solution in an amount and concentration at which the pH value of the sodium chlorate aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Further, in another embodiment, carbonic acid, phosphoric acid, boric acid, or sulfuric acid can be used as the inorganic acid in the above-described method, but phosphoric acid is preferred. Although it is not desired to be constrained by theory, the present invention has demonstrated that a condition of chlorous acid can be maintained while retaining a sterilizing effect with a high buffering effect within a suitable pH range by use of phosphoric acid in particular.

Further still, in another embodiment, carbonate, hydroxy salt, phosphate or borate can be used as the inorganic

acid salt, but phosphate is preferred. Although it is not desired to be constrained by theory, the present invention has demonstrated that a condition of chlorous acid can be maintained while retaining a sterilizing effect with a high buffering effect within a suitable pH range by the use of phosphate in particular.

Further, in another embodiment, sodium carbonate, potassium carbonate, sodium bicarbonate or potassium bicarbonate can be used as the carbonate.

Furthermore, in another embodiment, sodium hydroxide, potassium hydroxide, calcium hydroxide, or barium hydroxide can be used as the hydroxy salt.

Further still, in another embodiment, disodium hydrogen phosphate, sodium dihydrogen phosphate, trisodium phosphate, tripotassium phosphate, dipotassium hydrogen phosphate, or potassium dihydrogen phosphate can be used as the phosphate.

Further, in another embodiment, sodium borate or potassium borate can be used as the borate.

Furthermore, in another embodiment, succinic acid, citric acid, malic acid, acetic acid, or lactic acid can be used as the organic acid.

Further still, in another embodiment, sodium succinate, potassium succinate, sodium citrate, potassium citrate, sodium malate, potassium malate, sodium acetate, potassium acetate, sodium lactate, potassium lactate, or calcium lactate can be used as the organic acid salt.

In a method of manufacturing an aqueous solution comprising chlorous acid ($HClO_2$) that can be used as a sterilizing agent (chlorous acid aqueous solution), chlorous acid ($HClO_2$) is produced by adding hydrogen peroxide ($H_2O_2$) in an amount required to produce chlorous acid by a reducing reaction of chloric acid ($HClO_3$) obtained by adding sulfuric acid ($H_2SO_4$) or an aqueous solution thereof to an aqueous solution of sodium chlorate ($NaClO_3$) so that the aqueous solution of sodium chlorate is in an acidic condition. The basic chemical reaction of this method of manufacturing is represented by the following formula A and formula B.

[Chemical 1]

$$2NaclO_3 + H_2SO_4 \rightarrow 2HClO_3 + Na_2SO_4 \qquad \text{(formula A)}$$

$$HClO_3 + K_2O_2 \rightarrow HClO_2 + H_2O + O2\uparrow \qquad \text{(formula B)}$$

Formula A indicates that chloric acid is obtained by adding sulfuric acid ($H_2SO_4$) or an aqueous solution thereof in an amount and concentration at which the pH value of a sodium chlorate ($NaClO_3$) aqueous solution can be maintained with in acidity, while sodium ions are removed. Next, formula B indicates that chloric acid ($HClO_3$) is reduced by hydrogen peroxide ($H_2O_2$) to produce chlorous acid ($HClO_2$).

[Chemical 2]

$$HClO_3 + H_2O_2 \rightarrow 2ClO_2+H_2O+O_2\uparrow \qquad \text{(formula C)}$$

$$2\ ClO_2+H_2O_2 \rightarrow 2HClO_2+O_2\uparrow \qquad \text{(formula D)}$$

$$2\ ClO_2+H_2O \Leftrightarrow HClO_2+HClO_3 \qquad \text{(formula E)}$$

$$2\ HClO_2 \Leftrightarrow H_2O+Cl_2O_3 \qquad \text{(formula F)}$$

At this time, chlorine dioxide gas ($ClO_2$) is generated (formula C). However, from coexisting with hydrogen peroxide ($H_2O_2$), chlorous acid ($HClO_2$) is produced through the reactions in formulae D-F.

Meanwhile, the produced chlorous acid ($HClO_2$) has a property such that it is decomposed early into chlorine dioxide gas or chlorine gas due to the presence of chloride ion ($Cl^-$), hypochlorous acid (HClO) and other reduction substances or a decomposition reaction occurring among a plurality of chlorous acid molecules. Thus, it is necessary to prepare chlorous acid ($HClO_2$) so that the state of being chlorous acid ($HClO_2$) can be retained for a long period of time in order to be useful as a sterilizing.

In this regard, chlorous acid ($HClO_2$) can be stably retained over a long period of time from creating a transition state to delay a decomposition reaction by adding one compound from inorganic acids, inorganic acid salts, organic acids or organic acid salts, two or more types of compounds therefrom, or a combination thereof to the chlorous acid

($HClO_2$) or chlorine dioxide gas ($ClO_2$) obtained by the above-described method or an aqueous solution containing them. Although it is not desired to be constrained by theory, it was further demonstrated in the present invention that chlorous acid ($HClO_2$) can be stably retained over a long period of time from creating a transition state to delay a decomposition reaction by using a phosphoric acid buffer. Further still, although it is not desired to be constrained by theory, it was demonstrated in the present invention that chlorous acid ($HClO_2$) can be more stably retained over a long period of time from creating a transition state to delay a decomposition reaction by using potassium (potassium hydroxide, potassium phosphate (e.g., disodium hydrogen phosphate, sodium dihydrogen phosphate, trisodium phosphate, tripotassium phosphate, dipotassium hydrogen phosphate, or potassium dihydrogenphosphate)) asmetal, in comparison to cases using sodium or the like.

In one embodiment, it is possible to utilize a mixture in which one compound from inorganic acids or inorganic acid salts, specifically phosphate, carbonate or hydroxy salt, particularly phosphate and hydroxy salt, two or more types of compounds therefrom or a combination thereof is added to chlorous acid ($HClO_2$) or chlorine dioxide gas ($ClO_2$) obtained by the above-described method or an aqueous solution containing them.

In another embodiment, it is possible to utilize a mixture in which one compound from inorganic acids, inorganic acid salts, organic acids, or organic acid salts, two or more types of compounds therefrom, or a combination thereof is added to an aqueous solution to which one compound from inorganic acids or inorganic acid salts, specifically phosphate, carbonate, or hydroxy salt, particularly phosphate and hydroxy salt, two or more types of compounds therefrom, or a combination thereof is added.

Additionally, in another embodiment, it is possible to utilize a mixture in which one compound from inorganic acids or inorganic acid salts or organic acids or organic acid salts, two or more types of compounds therefrom, or a combination thereof is added to the aqueous solution manufactured by the above-described method.

Carbonic acid, phosphoric acid, boric acid, or sulfuric acid can be used as the above-described inorganic acid, but the inorganic acidis not limited thereto. Further, besides carbonate and hydroxy salt, phosphate or borate can be used as the inorganic acid salt, where phosphate is preferable, but the inorganic acid salt is not limited thereto. More specifically, sodiumcarbonate, potassium carbonate, sodium bicarbonate, potassium bicarbonate, or the like works well in use as the carbonate; sodium hydroxide, potassium hydroxide, calcium hydroxide, barium hydroxide, or the like works well in use as the hydroxy salt; disodium hydrogen phosphate, sodium dihydrogen phosphate, trisodium phosphate, tripotassium phosphate, dipotassium hydrogen phosphate, potassium dihydrogen phosphate, or the like works well in use as the phosphate; and sodium borate, potassium borate, or the like works well in use as the borate. Potassium salt is preferable but not limited thereto. Furthermore, succinic acid, citric acid, malic acid, acetic acid, or lactic acid can be used as the above-described organic acid. Further, sodium succinate, potassium succinate, sodium citrate, potassium citrate, sodium malate, potassium malate, sodium acetate, potassium acetate, sodium lactate, potassium lactate, calcium lactate or the like is suitable as the organic acid salt.

When an acid and/or a salt thereof is added, a transition state, such as $Na^+ + ClO_2^- \leftrightarrow Na\text{-}ClO_2$, $K^+ + ClO_2^- \leftrightarrow K\text{-}ClO_2$, or $H^+ + ClO_2^- \leftrightarrow H\text{-}ClO_2$ can be temporarily created to delay the progression of chlorous acid ($HClO_2$) to chlorine dioxide ($ClO_2$), which enables the manufacture of an aqueous solution comprising chlorous acid ($HClO_2$) that retains chlorous acid ($HClO_2$) for a long period of time and generates a reduced amount of chlorine dioxide ($ClO_2$). Although it is not desired to be constrained by theory, it was demonstrated in the present invention that such an effect of retaining is enhanced by using a phosphoric acid buffer. Although it is not desired to be constrained by theory, it was further demonstrated in the present invention that such an effect of retaining is enhanced more by using potassium salt in comparison to a case of using sodium salt or the like.

The following represents the decomposition of sodium chlorite in an acidic aqueous solution in the above-described formulae C, D, E and F.

[Chemical 3]

$$5ClO_2^- + 4H^+ \rightarrow 4ClO_2 + 5Cl^- + 2H_2O \qquad (a)$$

$$(5Na\ ClO_2 + 4CH_3COOH \rightarrow 4ClO_2 + 4CH_3COONa + NaCl + 2H_2O)\ 3\ ClO_2^- \rightarrow 2ClO_3^- + Cl^- \qquad (b)$$

$$(3NaClO_2 \rightarrow 2NaC\ lO_3 + NaCl\ \text{Autodecoposition}\ ClO_2^- \rightarrow Cl^- + 2O \qquad (c)$$

As represented in the formula, the rate of decomposition of a sodium chlorite aqueous solution is greater when pH is lower, i.e., when acidity is stronger. That is, the absolute rates of the reactions (a), (b), and (c) in the above-described formula increase. For example, although the ratio accounted for by reaction (a) decreases when pH is lower,

the total decomposition rate changes significantly, i.e., to a larger value. Thus, the amount of generated chlorine dioxide ($ClO_2$) increases with the decrease in pH. Thus, the lower the pH value, sooner the sterilization or bleaching takes effect. However, stimulatory and harmful chlorine dioxide gas ($ClO_2$) renders an operation more difficult and negatively affects the health of a human being. Further, a reaction from chlorous acid to chlorine dioxide progresses quicker, rendering the chlorous acid unstable. In addition, the time a sterilization power can be retained is very short.

In this regard, when the above-described inorganic acids, inorganic acid salts, organic acids or organic acid salts are added to an aqueous solution comprising chlorous acid (HClO), pH values are adjusted within the range of 3.2-8.5, especially within the preferred range of pH 3.2-7.0, pH 5.0-7.0 or the like that is explained in other portions of the present specification, from the viewpoint of balancing suppression of chlorine dioxide generation and sterilizing power.

When a spectrometric measurement of a sample can simultaneously identify two absorption sections between the wavelengths 248 and 420 nm: an absorption section comprising acidic chlorous acid ions ($H^+ + ClO_2^-$) representing a peak in the vicinity of 260 nm and an absorption section comprising chlorine dioxide ($ClO_2$) representing a peak near 350 nm, it is possible to recognize that the chlorous acid aqueous solution of the present invention is abundantly present, i.e., the presence of chlorus acid ($HClO_2$) can be recognized. This is because the cyclic reactions involving chlorous acid ($HClO_2$) as the active agent, chlorine dioxide ($ClO_2$), and acidic chlorous acid ion ($H^+$+$ClO_2^-$) are concurrently ongoing shown in the following Chemical Formula.

Cyclic Reactions of Chlorus Acid, Chlorine Dioxide, and Acidic, Chlorous Acid Ion

[Chemical 4]

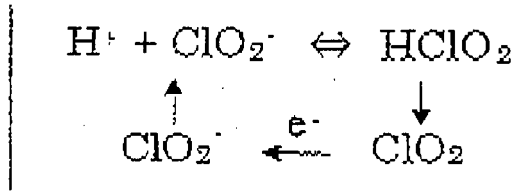

Conversion of chlorus acid ($HClO_2$) to chlorine dioxide ($ClO_2$) results in a single peak only near 350 nm.

It has already been found that pH can be further stabilized at this time by directly adding a buffer or by adding another buffer after first adjusting the pH with sodium carbonate or the like.

Thus, in one aspect, the present invention provides a sterilizing agent comprising a chlorous acid aqueous solution, metal hydroxide, and metal phosphate.

Although it is not desired to be constrained by theory, it was discovered that in the present invention, a sterilizing agent comprising a chlorous acid aqueous solution, metal hydroxide, and metalphosphate,particularly preferablysterilizing agents with pH configured to 3.5-7.5, unexpectedly retained a sterilizing effect while achieving a long-term stability effect (sterilizing effect does not decrease over 30 days or longer, i.e., over 240 days or longer converted in terms of normal temperature, in an accelerated test at 40°C in the range of pH 5.0 to 7.5 in particular). Preferable ranges of pH include, but not limited to, 3.2 or higher to less than 7.0, about 5.0 to about 7.5, about 5.0 to about 7.0, about 5.5 to about 7.0, about 5.0 to about 6.0, and the like. The lower limit includes, but not limited to, about 5.0, about 5.1, about 5. 2, about 5.3, about 5.4, about 5.5, and the like, and the upper limit includes, but not limited to, about 7.5, about 7.4, about 7.3, about 7.2, about 7.1, about 7.0, about 6.9, about 6. 8, about 6.7, about 6.5, about 6.4, about 6.3, about 6.2, about 6.1, about 6.0, about 5.9, about 5.8, about 5.7, about 5.6, about 5.5, and the like. The optimal pH includes, but not limited to, about 5.5. When "about" is used for a pH value herein, when the significant digit is the first decimal point, the range is intended to span 0.05 in both directions. For example, about 5.5 is understood as referring to 5.45 to 5.55. For the purpose of distinguishing from sodium chlorite, pH is preferably less than 7.0 in the present invention, but the pH is not limited thereto.

In another aspect, although it is not desired to be constrained by theory, the property of being readily dissociable in an aqueous solution was found in the present invention to be effective in retaining chlorous acid by using potassium as metal in a phosphoric acid buffer, in comparison to sodium or the like. In addition, the use was found to enhance an effect of retaining the created transition state for a long period of time and delaying the progression from chlorous acid ($HClO_2$) to chlorine dioxide ($ClO_2$). From the above, an effect of retaining chlorous acid ($HClO_2$) for a long period of time and reducing chlorine dioxide ($ClO_2$) generation was achieved. In addition, it was possible to achieve long-term preservation of chlorous acid (30 days or longer, i.e., 240 days or longer converted in terms of normal temperature, in an accelerated test at 40°C).

Preferable metal hydroxide includes sodium hydroxide and/or potassiumhydroxide. Preferable metal phosphate

includes sodium phosphate (e.g., disodium hydrogen phosphate, sodium dihydrogen phosphate, trisodium phosphate) and/or potassium phosphate (e.g., tripotassium phosphate, dipotassium hydrogen phosphate, or potassium dihydrogen phosphate; especially potassium dihydrogen phosphate), and still preferably, potassium hydroxide and potassium phosphate (e.g., tripotassium phosphate, dipotassium hydrogen phosphate, or potassium dihydrogen phosphate; especially potassium dihydrogen phosphate), where the above are non-limiting examples.

In a preferred embodiment, sodium hydroxide and potassium hydroxide are 0.1 N-1. 0 N and buffer pH of sodium phosphate and potassium phosphate is 5.0 to 7.5, especially pH of 5.0-7.0. This is because the effect of longer-termpreservation stability is unexpectedly more enhanced in comparison to the previously-expected levels at these constitution and pH.

In one aspect, the present invention provides an article impregnated with the sterilizing agent of the present invention. An article that can be used as the article of the present invention is any article that canbe impregnated with a chlorous acid aqueous solution to be used in sterilization or the like, including medical devices. A sheet, film, patch, brush, nonwoven fabric, paper, fabric, absorbent cotton, sponge and the like are examples thereof, but the article is not limited thereto. In a preferred embodiment, an article of the present invention is a sheet with one layer or two or more layers. In another preferred embodiment, chlorous acid is impregnated at a concentration of 3000 ppm or higher. When a sheet is impregnated at a concentration of 3000 ppm or higher, a sufficient sterilizing effect was observed against a wide variety of microbes, such as Staphylococcus aureus, Bacillus cereus, and microbes of the genus Salmonella, even with a single layer sheet. Such a sterilizing agent previously did not exist and is therefore recognized as a significant effect. In another preferred embodiment, the article of the present invention is a sheet with three or more layers. A sufficient sterilizing effect was observed against a wide variety of microbes, such as Staphylococcus aureus, Bacillus cereus, and microbes of the genus Salmonella, even at a low concentration of 1000 ppm, by providing a sheet with three layers or more. Thus, it can be seen that the number of layers of sheet should be increased when the concentration is low. The material of an article is not limited. Any material may be used as long as the material is capable of absorbing and retaining a chlorous acid aqueous solution and is capable of being applied. In one embodiment, a sheet may be made of cotton, but the material is not limited thereto.

In another aspect, the present invention provides a method of preserving a chlorous acid aqueous solution for a long period of time, comprising maintaining the chlorous acid aqueous solution at 10°C or lower. Although it is not desired to be constrained by theory, this is because a chlorous acid aqueous solution can be preserved over a long period over 30 days or longer by preserving at a low temperature.

Accordingly to the present invention, chlorous acid with high sterilizing power can be stabilized for a long period of time. Thus, an aqueous solution comprising chlorous acid, which generally could not be readily distributed as a product, can be distributed in a distribution channel. Thus, it is possible to make chlorous acid that is useful as a sterilizing agent prevalent in society.

In one aspect, the present invention provides a sterilizing agent for preventing secondary contamination, comprising a chlorous acid aqueous solution.

In another aspect, the present invention provides an article impregnated with a chlorous acid aqueous solution for preventing secondary contamination.

Herein, when used in regard to food products or the like, "cross-contamination" or "secondary contamination" refers to a certain microorganism contaminating cooking utensils, a hand or the like from a target such as a food product to contaminate another target such as a food product therefrom. This is a concept in contrast to primary contamination, which

An article that can be used as the article for preventing secondary contamination of the present invention is any article that can be impregnated with a chlorous acid aqueous solution for use in sterilization or the like, including medical devices. A sheet, film, patch, brush, nonwoven fabric, paper, fabric, absorbent cotton, sponge and the like are examples thereof, but the article is not limited thereto. In a preferred embodiment, an article of the present invention is a sheet with one layer or two or more layers. In another preferred embodiment, chlorous acid is impregnated at a concentration of 3000 ppm or higher. When a sheet is impregnated at a concentration of 3000 ppm or higher, a sufficient sterilizing effect was observed against a wide variety of microbes, such as Staphylococcus aureus, Bacillus cereus, and microbes of the genus Salmonella, even with a single layer sheet. Such a sterilizing agent previously did not exist and is therefore recognized as a significant effect. In another preferred embodiment, the article of the present invention is an article in a form of a sheet with three or more layers. A sufficient sterilizing effect was observed against a wide variety of microbes, such as Staphylococcus aureus, Bacillus cereus, and microbes of the genus Salmonella, even at a low concentration of 1000 ppm, by providing a sheet with three or more layers. Thus, it can be seen that the number of layers of sheet should be increased when the concentration is low. The material of an article is not limited. Any material may be used as long as the material is capable of absorbing and retaining a chlorous acid aqueous solution and is capable of being applied. In one embodiment, a sheet can be made of cotton, but the material is not limited thereto.

In another aspect, the present invention provides an article impregnated with a chlorous acid aqueous solution for sterilizing a floor surface. There are not that many sterilizing agents capable of sterilizing a floor surface. In addition,

there is no residual odor. Thus, the article is preferred for use in treating a floor surface or the like that requires maintenance of environment.

An article that can be used as the article for sterilizing a floor surface of the present invention is any article that can be impregnated with a chlorous acid aqueous solution for sterilization or the like, including medical devices. A sheet, film, patch, brush, nonwoven fabric, paper, fabric, absorbent cotton, sponge and the like are examples thereof, but the article is not limited thereto. In a preferred embodiment, chlorous acid is impregnated at a concentration of 1000 ppm or higher, but the concentration is not limited thereto. For sterilization of a floor surface, a sufficient disinfection effect was observed at 1000 ppm. Thus, it is expected that a sufficient sterilizing effect can be observed even at a concentration less than 1000 ppm. The material of an article is not limited. Any material may be used as long as the material is capable of absorbing and retaining a chlorous acid aqueous solution and is capable of being applied. In one embodiment, the sheet of the present invention is made of cotton.

In another aspect, the present invention provides an application of a chlorous acid aqueous solution in handling the environment or odor. That is, in one aspect, the present invention provides an agent for removing odor (also referred to as a deodorizer) comprising a chlorous acid aqueous solution. In another aspect, the present invention provides an article impregnated with a chlorous acid aqueous solution for removing odor.

The "odor" targeted by the present invention herein refers to any odor caused by a chemical substance (also referred to as an odorous substance, e.g., vomit). An odor index can express an odor concentration in index scale levels. The current Japanese Offensive Odor Control Law incorporates restrictions in terms of concentration of specific offensive odor causing substances (22 types) and in terms of odor index. The method of measurement is stipulated as being carried out according to a sensory testing method (determined by human olfactory sense) using three-point-comparative odor bag method by a government-approved odor judgment technician. Thus, it is understood that an evaluation by the human olfactory sensory system as in the present Example can be employed in the present invention. Such an effect on odor could not be achieved with sodium hypochlorite (chlorine odor) or ethyl alcohol (alcohol odor), and the effect is recognized to be significant, even in comparison to water itself (putrid odor).

The agent for removing odor or deodorizer of the present invention can be in any form that can be impregnated with a chlorous acid aqueous solution for use in odor removal or the like, including a medicine, quasi-drug, food additive, and medical device. A spray, liquid agent, gel agent and the like are examples thereof, but the form is not limited thereto. In a preferred embodiment, chlorous acid is used, for example, at a concentration of 400 ppm or higher, or 500 ppm or higher, but the concentration is not limited thereto. It is understood that sufficient sterilizing effects are observed even at less than 400 ppm for removing odor (deodorizing).

Odor that can be deodorized by the agent for removing odor or deodorizer of the present invention may include at least one, at least two or three odors selected from the group consisting of ammonium odor, amine odor (methyl mercaptan) and sulfur odor. In particular, the present invention, which can remove at least one of and preferably both of ammonium odor and amine odor that could not be completely removed with sodium hypochlorite, is recognized as achieving a non-conventional deodorizing effect.

The agent for removing odor or deodorizer of the present invention achieves a long-term odor removing effect. In one embodiment, the agent for removing odor or deodorizer of the present invention has an odor removing effect even when 3 hours or more has elapsed after odor removing treatment. Preferably, the agent for removing odor or deodorizer of the present invention has an odor removing effect even when 6 hours or more has elapsed after odor removing treatment. Still preferably, the agent for removing odor or deodorizer of the present invention has an odor removing effect even when 9 hours or more has elapsed after odor removing treatment. Still preferably, the agent for removing odor or deodorizer of the present invention has an odor removing effect even after 24 hours or more has elapsed after odor removing treatment. Removal of amine odor even when 24 hours has elapsed could not be achieved by conventional deodorizers, which is thus recognized as a significant effect of the present invention. Further, removal of ammonium odor even when 3 hours, 6 hours, 9 hours, or 24 hours has elapsed could not be achieved by conventional deodorizers, which is thus recognized as a significant effect of the present invention.

An article that can be used as the article for removing odor of the present invention is any article that can be impregnated with a chlorous acid aqueous solution for use in odor removal (deodorizing) or the like, including medical devices. A sheet, film, patch, brush, nonwoven fabric, paper, fabric, absorbent cotton, sponge and the like are examples thereof, but the article is not limited thereto. In a preferred embodiment, chlorous acid is impregnated, for example, at a concentration of 400 ppm or higher, or 500 ppm or higher, but the concentration is not limited thereto. For odor removal (deodorizing), it is understood that a sufficient sterilizing effect is observed even at less than 400 ppm. The material of an article is not limited. Any material may be used as long as the material is capable of absorbing and retaining a chlorous acid aqueous solution and is capable of being applied. In one embodiment, the sheet of the present invention is made of cotton.

In another aspect, the present invention provides an application of a chlorous acid aqueous solution for washing and removing microbes from the body (e.g., hand or finger). That is, in one aspect, the present invention provides a removing agent comprising a chlorous acid aqueous solution for removing microbes adhering to the body. In another

aspect, the present invention provides an article impregnated with a chlorous acid aqueous solution for removing microbes adhering to the body.

An article that can be used as the article for disinfecting viruses of the present invention is any article that can be impregnated with a chlorous acid aqueous solution for use in sterilization, microbe removal or the like, including medical devices. A sheet, film, patch, brush, nonwoven fabric, paper, fabric, absorbent cotton, sponge and the like are examples thereof, but the article is not limited thereto. Chlorous acid is impregnated at a concentration of 3000 ppm or higher, but the concentration is not limited thereto. This is because it has been demonstrated that microbes can be removed by the first wipe, for example, at a concentration of 1000 ppm depending on the microbial species, and other microbial species could be removed by the third wipe. The material of an article is not limited. Any material may be used as long as the material is capable of absorbing and retaining a chlorous acid aqueous solution and is capable of being applied. In one embodiment, the sheet of the present invention is made of cotton.

Any reference document cited herein, such as a scientific article, patent and patent application, is incorporated by reference in the present specification in the same manner as the entire contents are specifically described therein.

As described above, the present invention has been explained while presenting preferable embodiments to facilitate understanding. Hereinafter, the present invention is explained based on the Examples. However, the aforementioned explanation and the following Examples are provided solely for exemplification, not for limiting the present invention. Thus, the scope of the present invention is not limited to the Embodiments or Examples that are specifically described herein. The scope of the present invention is limited solely by the scope of the claims.

(Examples)

When necessary, animals used in the following Examples were handled in compliance to the Declaration of Helsinki. For reagents, the specific products described in the Examples were used. However, the reagents can be substituted with an equivalent product from another manufacturer (Sigma, Wako Pure Chemical Industries, Nacalai Tesque, or the like) . There are cases herein where an abbreviation "CAAS" is used for a chlorous acid aqueous solution. However, they have the same meaning.

(Example 1: Production of Chlorous Acid Aqueous Solution)

The chlorous acid aqueous solution used in the following Examples was produced as explained below.

(Example of Manufacturing Plant)

Figure 1 shows an example of a manufacturing plant that was used.

In Figure 1, each number is a member indicated in the following Tables.

[Table 1]

| Number | Name |
|---|---|
| 1 | Sulfuric acid input port |
| 2 | Hydrogen peroxide input port |
| 3 | Reaction bath |
| 4 | Collection bath |
| 5 | Gas adsorption bath |
| 6 | Gas washing bath |
| 7 | Air pump |
| 8 | Aeration value/Chloric acid input port |
| 9 | Agitation motor |

[Table 2]

| Number | Name |
|---|---|
| A | Valve for the sulfuric acid input, port |

(continued)

| Number | Name |
|---|---|
| B | Valve for hydrogen peroxide input port |
| C | Air pump faucet |
| D | Valve for discharging a reaction fluid |
| E | Trifurcated faucet |
| F | Not used |
| G | Aeration valve |

(Blending Examples for Each Solution)

Blending examples of each solution that can be used in the present manufacturing example are described below. Blend Table a is a blending example used in the following Example. Blending table b is a blending example of a chlorous acid aqueous solution with pH of 8.5. Blending table c is a blending example of a chlorous acid aqueous solution with pH of 6.5. Blending table disablending example of a chlorous acid aqueous solution with pH of 3.5. Blending table e is a blending example of a gas cleaning solution.

Blending Table a

[Table 3]

| | Name of Raw Material | Blended Amount |
|---|---|---|
| (1) | 25 % sodium chlorate aqueous solution | 200 g |
| (2) | 70 % (w/w) sulfuric acid | 300 g |
| (3) | 35 % hydrogen peroxide | 50g |

Blending Table b (Chlorous Acid Aqueous Solution pH 8.5)

[Table 4]

| | Name of Raw Material | Blended Amount | Concentration | Tolerance Range |
|---|---|---|---|---|
| (1) | Tap water | 738.02g | | |
| (2) | Sodium hydroxide | 32g | 3.2% (0.8mol/L) | 0.8%~4.0% (0.2mol/L~1.0mol/L) |
| (3) | Dipotassium hydrogen phosphate | 139.36g | 13.9%(0.8mol/L) | 8.7%~13.9% (0.5mol/L~0.8mol/L) |
| (4) | Sodium carbonate | 53g | 5.3% (0.5mol/L) | 1.06%~10.6% (0.1mol/L~1.0mol/L) |
| (5) | Sodium tetraborate (decahydrate) | 7.62 g | 0.76% (0.02mol/L) | 0.38%~5.7% (0.01mol/L~0.15mol/L) |
| (6) | 35%Hydrogen peroxide | 30g | 3.0% | |
| Total | | 1000g | | |

Blending Table c (Chlorous Acid Aqueous Solution pH 6.5)

[Table 5]

| | Name of Raw Material | Blended Amount | Concentration | Tolerance Range |
|---|---|---|---|---|
| (1) | Tap water | 738.64g | | |
| (2) | Potassium hydroxide | 44.8g | 4.5% (0.8mol/L) | 1.1%~5.6% (0.2mol/L~1.0mol/L) |

(continued)

| | Name of Raw Material | Blended Amount | Concentration | Tolerance Range |
|---|---|---|---|---|
| (3) | Dipotassium hydrogen phosphate | 139.36g | 13.9% (0.8 mol/L) | 8.7%~13.9% (0.5mol/L~0.8mol/L) |
| (4) | Succinic acid | 47.2g | 4.7% (0.4mol/L) | 2.3%~4.7% (0.2mol/L~0.4mol/L) |
| (5) | 35%Hydrogen peroxide | 30g | | |
| Total | | 1000g | | |

Blending Table d (Chlorous Acid Aqueous Solution pH 3.5)

[Table 6]

| | Name of Raw Material | Blended Amount | Concentration | Tolerance Range |
|---|---|---|---|---|
| (1) | Tap water | 938g | | |
| (2) | Sodium hydroxide | 32g | 3.2% (0.8mol/L) | 0.8%~4.0% (0.2mol/L~1.0mol/L) |
| (3) | 35%Hydrogen peroxide | 30g | 3.0% | |
| Total | | 1000g | | |

Blending Table e (Gas washing solution)

[Table 7]

| | Name of Raw Material | Blended Amount | Concentration | Tolerance Range |
|---|---|---|---|---|
| (1) | Tap water | 910g | | |
| (2) | Sodium hydroxide | 60g | 6.0% (1.5mol/L) | 6.0%≦ (1.5mol/L≦) |
| (3) | 35%Hydrogen peroxide | 30g | | |
| Total | | 1000g | | |

(Manufacturing Method of Chlorous Acid Aqueous Solution)

(1. Setting)

Setting was performed as follows.

1 Blending table e was fed into 6.
2 Blending table b, c, or d was fed into 5.
3 A, B, C, D, and G were confirmed to be sealed.
4 E was confirmed to be opened in the route 3→E→5.

(2. Reaction)

Reactions were conducted as follows.

5 G was opened.
6 (1) of Blending Table a was fed to 3 from 8.
7 G was closed.
8 (2) of Blending Table a was fed to 1.
9 A was opened.
10 A syringe was inserted into 1 to slowly feed 3.
11 Alter all of (2) of Blending Table a was completely fed, A was shut close.
12 9 was operated to agitate for one minute.
13 9 was stopped.

14 (3) of Blending Table a was fed into 2.
15 B was opened.
16 A syringe was inserted into 2 to slowly feed 3.
17 Alter all of (3) of Blending Table a was completely fed, B was shut close.
18 It was left standing for five minutes.
19 9 was operated to agitate for 15 minutes.
20 9 was stopped.
21 It was left standing for 10 minutes.
22 7 was operated.
23 C was opened to send in air.
24 It was left standing for one hour.
25 C was closed.
26 7 was stopped.
27 Faucet was moved so that E has the route 4→E→5.
28 D was opened.
29 After all of the reaction solution moved to 4, D was closed.
30 The route of E was changed to 3→E→5.
31 It was left standing for one hour.
32 The procedures of 5 to 31 were repeated 2-4 times as needed.
33 A solution was collected from 5, which was considered a chlorous acid aqueous solution.

(Component Analysis)

The component analysis table for the chlorous acid aqueous solution with pH of 8.5 of the present Example is shown below.

Component Analysis Table for Chlorous Acid Aqueous Solution with pH of 8.5

[Table 8]

| CAAS specification | Specification Value | Match/Not a Match |
|---|---|---|
| Content | 1.0 % or higher | 4.0% |
| Attribute | light yellowish green to yellowish red | light yellowish green |
| Confirmation Test (1) | When 0.1 ml of potassium permanganate aqueous solution (1→300) is added to 5 ml of an aqueous solution of the present product (1→20), the solution turns reddish purple. When 1 ml of sulfuric acid (1→20) is added thereto, the solution turns light yellow. | Match |
| Confirmation Test (2) | An aqueous solution of the present product (1→20) has portions of maximum absorbance at wavelengths 258-262 nm and 346-361 nm. | Match (Spectrograph is shown in Figure 2) |
| Confirmation Test (3) | If potassium iodide starch paper is dipped in the present product, the potassium iodide starch paper changes to a blue color and then the color fades. | Match |
| Purity Test (1) | 1.0 μg/g or lower for lead | Below detectable limit |
| Purity Test (2) | 1.0 μg/g or lower for $As_2O_3$ | Below detectable limit |

The component analysis table for the chlorous acid aqueous solution with pH of 6.5 of the manufacturing Example is shown below.

Component Analysis Table for ChlorousAcid Aqueous Solution with pH of 6.5

[Table 9]

| CAAS specification | Specification Value | Match/Not a Match |
|---|---|---|
| Content | 1.0 % or higher | 4.4% |

(continued)

| CAAS specification | Specification Value | Match/Not a Match |
|---|---|---|
| Attribute | light yellowish green to yellowish red | yellow |
| Confirmation Test (1) | When 0.1 ml of potassium permanganate aqueous solution (1→300) is added to 5 ml of an aqueous solution of the present product (1→20), the solution turns reddish purple. When 1 ml of sulfuric acid (1→20) is added thereto, the solution turns light yellow. | Match |
| Confirmation Test (2) | An aqueous solution of the present product (1→20) has portions of maximum absorbance at wavelengths 258-262 nm and 346-361 nm. | Match (Spectrogra ph is shown in Figure 3) |
| Confirmation Test (3) | If potassium iodide starch paper is dipped in the present product, the potassium iodide starch paper changes to a blue color and then the color fades. | Match |
| Purity Test (1) | 1.0 μg/g or lower for lead | Below detectable limit |
| Purity Test (2) | 1.0 μg/g or lower for $As_2O_3$ | Below detectable limit |

The component analysis table for the chlorous acid aqueous solution with pH of 3.5 of the manufacturing Example is shown below.

Component Analysis Table for Chlorous Acid Aqueous Solution with pH of 3.5

[Table 10]

| CAAS specification | Specification Value | Match/Not a Match |
|---|---|---|
| Content | 1.0 % or higher | 4.2% |
| Attribute | light yellowish green to yellowish red | yellowish red |
| Confirmation Test (1) | When 0.1 ml of potassium permanganate aqueous solution (1→300) is added to 5 ml of an aqueous solution of the present product (1→20), the solution turns reddish purple. When 1 ml of sulfuric acid (1→20) is added thereto, the solution turns light yellow. | Match |
| Confirmation Test (2) | An aqueous solution of the present product (1→20) has portions of maximum absorbance at wavelengths 258-262 nm and 346-361 nm. | Match (Spectrograph is shown in Figure 4) |
| Confirmation Test (3) | Ifpotassiumiodidestarch paper is dipped in the present product, the potassium iodide starch paper changes to a blue color and then the color fades. | Match |
| Purity Test (1) | 1.0 μg/g or lower for lead | Below detectable limit |
| Purity Test (2) | 1. 0 μg/g or lower for $As_2O_3$ | Below detectable limit |

UV spectrums immediately after manufacture of the chlorous acid aqueous solution (pH 3.0) and chlorous acid aqueous solution (pH 9.0) manufactured in the present Example are shown in Figures 5-8. Figure 5: UV spectrum immediately after manufacture at pH 3.0; Figure 6: UV spectrum 30 days after manufacture at pH 3.0; Figure 7: UV spectrum immediately after manufacture at pH 9.0; Figure 8: UV spectrum 30 days after manufacture at pH 9.0.

Next, sterilizing effects were examined by evaluating phenol coefficients. A phenol coefficient (PC) is a coefficient comparing antiseptic action of an antiseptic agent against microbes with respect to phenol. A phenol coefficient is expressed as a ratio of the maximum dilution factors of an antiseptic agent of interest and phenol at which microbes dies in 10 minutes, but not in 5 minutes, in diluents of the antiseptic agent and phenol inoculated with tested microbes Staphylococcus aureus, Salmonella typhi, Escherichia coli or the like.

Figure 9 shows sterilizing effect examination charts (phenol and chlorous acid concentrations) together with UV spectrums for pH of 3.5, 6.5, and 8.5 immediately after manufacture.

Figure 10 shows sterilizing effect examination charts (phenol and chlorous acid concentrations) together with UV spectrums for pH of 3.5, 6.5, and 8.5 after 10 days from manufacture.

Figure 11 shows sterilizing effect examination charts (phenol and chlorous acid concentrations) together with UV spectrums for pH of 3.5, 6.5, and 8.5 after 30 days from manufacture.

Figure 12 shows a phenol coefficient immediately after manufacture, a phenol coefficient 10 days after manufacture, and a phenol coefficient 30 days after manufacture at pH of 2.0 to 9.0 for every 0.5. It was revealed that pH is substantially maintained at pH 4.5-7.5 after 10 days and at pH 5.0-7.5 after 30 days.

Chlorous acid aqueous solutions were manufactured as a chlorous acid aqueous solution having each pH by the method of manufacturing described above. Herein, chlorous acid aqueous solutions from pH of 2.0 to 9.0 were manufactured. Storage property and retention of sterilizing effect were expressed in phenol coefficients. For the blend of a chlorous acid aqueous solution having each pH at the time of manufacture, those with pH of 3.5, 6.5 and 8.5 are described as representative examples. When a chlorous acid aqueous solution having other pH is manufactured, these blends can be combined and manufactured within the tolerance range.

A double-hump peak is found between pH 3.5 to pH 8.5 in a chlorous acid aqueous solution immediately after manufacture, and those within this range can be considered a chlorous acid aqueous solution. With regard to sterilizing effects, a sterilizing effect is recognized at every pH from 2.0 to 9.0. It can be seen that sterilizing effects of a chlorous acid aqueous solution between pH of 3.5 and 8.5 is especially high. Further, although a high sterilizing effect is recognized even at pH of 3.0 or lower, a double-humped peak is not maintained, having a special absorbent section only at the wavelength 350 nm. This is believed to be a sterilizing effect of chlorine dioxide ($ClO_2$). Further, at pH of 9.0, a special absorbent section is present only at wavelength 260 nm. It can be understood as being in a state of chlorous acid ions and having a low phenol coefficient and sterilizing power in comparison to other chlorous acid aqueous solutions.

When the phenol coefficient upon preservation of these chlorous acid aqueous solution for 30 days at 4°C were studied, UV spectrums as well as sterilizing effect disappeared at pH of 3.0 or lower. Further, at pH of 9.0, no change in UV spectrum or sterilizing effect was observed. Further, from pH of 3.5 to 8.5, a double-humped peak was recognized and, although the phenol coefficient was lower in comparison to that immediately after manufacture, the phenol coefficient was 50 or higher, and a sterilizing effect can thus be recognized. From the above, a chlorous acid aqueous solution is believed to be from pH of 3.5 to 8.5. Sterilizing effects were highly preserved at PH of 5.0 to 7.5 in particular, and the optimal pH was 5.5. However, it is believed as common sense to be present as chlorous acid ions, not chlorous acid aqueous solution, at pH of 7.0 or higher. From the above, the range of pH regions, preferably 3.5 to less than 7.0, is believed to be particularly useful as a chlorous acid aqueous solution. However, the range is not limited thereto.

In this regard, a chlorous acid aqueous solution (pH 3.5), which had a sterilizing effect immediately after manufacture but significantly decreased sterilizing effect after 30 days, was used and formulated according to the following blend so as to have pH from 6.0 to 7.0.

Chlorous Acid Aqueous Solution with pH 3.5

[Table 11]

| | Name of Raw Material | Blended Amount | Concentration | Tolerance Range |
|---|---|---|---|---|
| (1) | Tap water | 258.0g | | |
| (3) | Dipotassium hydrogen phosphate | 17.0g | 1.70% | 0.70%~13.90% |
| (4) | Succinic acid | 5.0g | 0.50% | 0.10%~5.60% |
| (5) | Chlorous acid aqueous solution (pH3.5) | 720.0g | 72.00% | 0.25%~75% |
| Total | | 1000g | 30000ppm | |

[Table 12]

| CAAS specification | Chlorous acid aqueous solution formulation manufactured using chlorous acid aqueous solution with pH of 3.5 |
|---|---|
| Content | 3.0 % |
| Attribute | yellow |
| Confirmation Test (1) | Match |

(continued)

| | |
|---|---|
| CAAS specification | Chlorous acid aqueous solution formulation manufactured using chlorous acid aqueous solution with pH of 3.5 |
| Confirmation Test (2) | Match <Spectrograph is shown in Figure 13> |
| Confirmation Test (3) | Match |
| Purity Test (1) | Below detectable limit |
| Purity Test (2) | Below detectable limit |

In this component specification, the result was the same as the case where a chlorous acid aqueous solution with a different pH was similarly made to have a pH region from pH 6.0 to pH 7.0. This chlorous acid aqueous solution formulation was used to carry out the following Example.

(Example 2 : Assessment Test on Sustainability of Sterilizing Power -Sustainability of Sterilizing Power against E. coli and Staphylococcus aureus-)

The present Example examined whether a sterilizing effect of a chlorous acid aqueous solution formulation on microbial strains (E. coli and Staphylococcus aureus), which are indicator strains for evaluating a microbial removing effect, has sustainability.

<Testing Method>

(Material)

(1) Reagents that were used
The chlorous acid aqueous solution formulation produced in Example 1, 0.1 M sodium thiosulfate
(2) Instrument that was used
Electronic balance, triangular flask with a stopper, beaker, pipette, stirrer, stirrer bar, test tube, and vortex mixer
(3) Tested Microbial species
E. coli (Escherichia coli IF03972)
Staphylococcus aureus (Staphylococcus aureus IF012732)

(Method)

1). Preparation Method of Concentrated Suspension of Test Microbes

After each tested bacterium was smeared on a common agar medium (Eiken Chemical Co., Ltd.) and cultured for 24 hours at 37°C, a colony that grew on the medium was extracted with a platinum loop to form a concentrated suspension with sterile saline. The solution was centrifuged and the supernatant was removed. The microbial cells were again homogeneously suspended in saline to produce a concentrated suspension of testedmicrobes ($\times 10^6$/ml). The microbial solution was prepared in accordance with turbidity so that the number of microbes would be at a certain amount.

2). Preparation Method of Sample Solution for Testing

An undiluted solution of a chlorous acid aqueous solution formulation was adjusted so that the chlorous acid ($HClO_2$) concentration would be 3600 ppm and 1000 ppm as of the starting point. At this time, dilution factors were recorded. Periodically, when the undiluted solution of chlorous acid aqueous solution was taken out, sterilized ion exchange water was used at a dilution factor recorded at the starting point to prepare each sample solution for testing. In addition, an accelerated test, which corresponds to six times the elapsed days of normal temperature storage, was carried out by storing at a temperature range of 40°C.

3). Method of Contacting Test Microbes and Method of Evaluating Effects on Microbes

1.0 ml of each concentrated suspension of test microbes ($\times 10^6$ microbes/ml) was added to 9.0 of each sample

solution for testing. The solution was homogeneously mixed, stored in a 25°C water bath, and homogeneously mixed again every 1, 5, and 10 minutes to collect 9.0 ml of each solution. After the collected solution was added to 1.0 ml of sterilized 0.1 mol/L sodium thiosulfate (prepared with various buffers) and mixed homogeneously, 1.0 ml of the solution was apportioned to each of two petri dishes after the solution was further left standing for 10 minutes. The number of surviving microbes was then measured according to a common method, i.e., pour-plate culture. The medium used at this time was a desoxycholate medium (Eiken Chemical Co., Ltd.) for E. coli and a mannitol salt agar medium with egg yolk (Eiken Chemical Co., Ltd.) for Staphylococcus aureus. After culturing for 24 hours at 37 °C, the number of typical colonies growing in the two plates were averaged and recorded as the number of surviving microbes.

The above method was carried out to determine the extent and presence of effect from the rate of decrease in the number of live microbes.

(Test Results)

Test for Examining Sustainability of Effect of Chlorous Acid Aqueous Solution Stored for 40 days at 40°C on Microbes

Even when stored under severe conditions such as storage in 40°C, loss or decrease in sterilizing effect against E. coli or Staphylococcus aureus was not observed for the chlorous acid aqueous solution of the present invention. A stable effect thereof was confirmed.

Test for Examining Sustainability of Effect of Chlorous Acid Aqueous Solution Stored at 40°C on E. coli, Unit: microbes/mL

[Table 13]

| Contact Concentration Chlorous Acid ($HClO_2$) Concentration | 0ppm | 3,600ppm | | | 1,000ppm | | |
|---|---|---|---|---|---|---|---|
| Time of Contact / Days Elapsed*1 | 0min | 1min | 5min | 10min | 1min | 5min | 10min |
| 0 | $1.9\times10^5$ | <100 | <100 | <100 | <100 | <100 | <100 |
| 42 | $7.4\times10^5$ | <100 | <100 | <100 | <100 | <100 | <100 |
| 60 | $1.9\times10^5$ | <100 | <100 | <100 | <100 | <100 | <100 |
| 84 | $2.8\times10^5$ | <100 | <100 | <100 | <100 | <100 | <100 |
| 120 | $4.0\times10^4$ | <100 | <100 | <100 | <100 | <100 | <100 |
| 150 | $4.0\times10^6$ | <100 | <100 | <100 | <100 | <100 | <100 |
| 180 | $8.3\times10^5$ | <100 | <100 | <100 | <100 | <100 | <100 |
| 240 | $1.4\times10^5$ | <100 | <100 | <100 | <100 | <100 | <100 |

*1: An accelerated test was carried out in 40°C storage, which corresponds to 6-fold acceleration in terms of days of storage at normal temperature.

Results of Tests for Examining Effect of Chlorous Acid Aqueous Solution Formulation stored at 40°C on Staphylococcus aureus,
Unit: microbes/mL

[Table 14]

| Contact Concentration Chlorous Acid ($HClO_2$) Concentration | 0ppm | 3,600ppm | | | 1,000ppm | | |
|---|---|---|---|---|---|---|---|
| Time of Contact / Days Elapsed*1 | 0min | 1min | 5min | 10min | 1min | 5min | 10min |
| 0 | $2.0\times10^4$ | <100 | <100 | <100 | <100 | <100 | <100 |
| 42 | $1.6\times10^5$ | <100 | <100 | <100 | <100 | <100 | <100 |
| 60 | $2.0\times10^4$ | <100 | <100 | <100 | <100 | <100 | <100 |
| 84 | $2.0\times10^5$ | <100 | <100 | <100 | <100 | <100 | <100 |
| 120 | $1.4\times10^5$ | <100 | <100 | <100 | <100 | <100 | <100 |
| 150 | $4.4\times10^4$ | <100 | <100 | <100 | <100 | <100 | <100 |
| 180 | $6.1\times10^5$ | <100 | <100 | <100 | <100 | <100 | <100 |
| 240 | $7.2\times10^4$ | <100 | <100 | <100 | <100 | <100 | <100 |

*1: An accelerated test was carried out in 40°C storage, which corresponds to 6-fold acceleration in terms of days of storage at normal temperature.

Tables 13 and 14 show sterilizing effects of the chlorous acid aqueous solution formulation manufactured in Example 1 when stored for 40 days at 40°C. As a result, it was found that a sterilizing effect significantly decreased in only 30 days for the chlorous acid aqueous solution (pH 3.5) of Example 1, but the sterilizing effect can be retained at least 240 days converted in terms of normal temperature by formulation said chlorous acid aqueous solution to pH of 6.0 to 7.5.

(Example 3 : Test for Examining Sustainability of Effect after Storage for 40 days at 40°C)

In the present Example, tests were carried out to examine sustainability of sterilizing effects against tested microbes of the chlorous acid aqueous solution stored for 40 days at 40°C (240 days converted in terms of normal temperature) used in Example 2, which was discretionarily diluted and further stored for 30 days at normal temperature. Even when a chlorous acid aqueous solution was stored under severe conditions such as storage in 40°C and diluted to a specified concentration and then the diluent was stored for 30 days at normal temperature, loss or decrease in sterilizing effects against E. coli or Staphylococcus aureus was not observed for the chlorous acid aqueous solution. A stable effect thereof was confirmed.

Results of Tests for Examining Effects on E. coli, Unit: microbes/mL

[Table 15]

| Contact Concentration Chlorous Acid ($HClO_2$) Concentration | 0ppm | 3,600ppm | | | 7,200ppm | | | 10,800ppm | | |
|---|---|---|---|---|---|---|---|---|---|---|
| Time of Contact / Days Elapsed | 0min | 1min | 5min | 10min | 1min | 5min | 10min | 1min | 5min | 10min |
| 0 | $1.4\times10^5$ | <100 | <100 | <100 | <100 | <100 | <100 | <100 | <100 | <100 |
| 30 | $5.5\times10^6$ | <100 | <100 | <100 | <100 | <100 | <100 | <100 | <100 | <100 |

Results of Tests for Examining Effects on Staphylococcus aureus,
Unit: microbes/mL

[Table 16]

| Contact Concentration Chlorous Acid ($HClO_2$) Concentration | 0ppm | 3,600ppm | | | 7,200ppm | | | 10,800ppm | | |
|---|---|---|---|---|---|---|---|---|---|---|
| Days Elapsed \ Time of Contact | 0min | 1min | 5min | 10min | 1min | 5min | 10min | 1min | 5min | 10min |
| 0 | $7.2x10^4$ | <100 | <100 | <100 | <100 | <100 | <100 | <100 | <100 | <100 |
| 30 | $4.0x10^5$ | <100 | <100 | <100 | <100 | <100 | <100 | <100 | <100 | <100 |

In this manner, it was revealed that a sterilizing effect can be retained for a long period (240 days converted in terms of normal temperature) even if a chlorous acid aqueous solution formulation is diluted or under a severe condition of 40°C.

(Example 4: Tests for Examining Stability of Sterilizing Power When Impregnated in Treatment Sheet)

In the present Example, the chlorous acid aqueous solution formulation of Example 1 was diluted to an available chlorine concentration of 1000 ppm or 500 ppm and the solution was used to impregnate a treatment sheet so that the ratio of solid to liquid is 1:3. Tests to examine the stability of sterilizing power were carried out at this time.

That is, the stability of sterilizing power when a diluent of a chlorous acid aqueous solution formulation was impregnated into a treatment sheet was examined.

<Testing Method>

(Materials)

1) Treatment sheet (material: 100 % cotton) : (about 27 × 40 cm) *Impregnated at a ratio of weight of treatment sheet (g) : amount of liquid (ml) = 1:3
2) Reagents that were used "Chlorous acidaqueous solution formulation (see Example 1: Table 11)", sodium hypochlorite (Sankurin12), 10w/w% potassiumiodide, 10% sulfuric acid, 0.1 M sodium thiosulfate
3) Instrument that was used Electronic balance, triangular flask with a stopper, beaker, pipette, stirrer, stirrer bar, treatment sheet (material: 100% cotton)

(Method)

<Testing Procedure>

(1) The available chlorine concentration of the "chlorous acid aqueous solution formulation (see Example 1: Table 11)" was measured, and the formulation was diluted to an available chlorine concentration of 1000 ppm [chlorous acid ($HClO_2$) concentration: 489 ppm] or 500 ppm [chlorous acid ($HClO_2$) concentration: 245 ppm].
(2) The available chlorine concentration of sodium hypochlorite was measured, and the sodium hypochlorite was diluted to an available chlorine concentration of 1000 ppm or 500 ppm.
(3) The available chlorine concentration of the diluted solution was measured to find the available chlorine concentration prior to contact with a treatment sheet.
(4) Treatment sheets were stacked, rolled up and inserted into a 200 ml-capacity PET bottle. A solution diluted so that the weight ratio of treatment sheet: agent solution would be 1:3 was poured into the 200 ml-capacity PET bottle containing the treatment sheets.
(5) After contact with treatment sheets, treatment sheets were pulled out from the 200 ml-capacity PET bottle immediately after contact, after 1 hour, after 2 hours, after 3 hours, after 12 hours, after 24 hours, after 48 hours

(after 2 days), after 72 hours (after 3 days), and after 168 hours (after 7 days). The sheets were used as specimens for examining available chlorine concentrations.
(6) Solutions from the treatment sheets, the specimens pulled out from the 200 ml-capacity PET bottle, were wrung out into a 100 mL beaker. The available chlorine concentration of the solution was measured.

(Measurement Method of Available Chlorine)

1. About 5 g of solution that was wrung out from the specimen was collected in a triangular flask with a stopper.
2. After adding 10 mL of 10 w/w % potassium iodide to the triangular flask with a stopper, 10 mL of 10% sulfuric acid was added.
3. After sealing, the mixture was left standing for 15 minutes in the dark.
4. 0.1 mol/L sodium thiosulfate solution was added until the color became light yellow. About 1 mL of 1 w/w % starch solution was added, and 0.1 mol/L sodium thiosulfate solution was added until the color of the solution became colorless.
5. Chlorine titer was found by the following equation.

$$R = (a \times f/w) \times 0.0035 \times 100$$

R; Chlorine titer value
a; Amount of 0.1 mol/L sodium thiosulfate solution added (g)
f; Factor of 0.1 mol/L sodium thiosulfate solution
w; Weight of measured specimen solution (g)
0.0035; Amount of chlorine corresponding to 1g of 0.1 mol/L sodium thiosulfate
100; Percentage

<Test Results>

The results are shown in Table 17 and Figure 14.
Chronological Change in Available Chlorine Concentration after Impregnating Each Diluent in Treatment Sheet

[Table 17]

| Agent Solution | | Available Chlorine Concentration (ppm) | | | |
|---|---|---|---|---|---|
| | | Chlorous Acid Aqueous Solution Formulation | | Sodium Hypochlorite Solution | |
| Set Concentration | | 1000 | 500 | 1000 | 500 |
| Time of Contact (Hours) | Before Contact | 1091 | 543 | 1045 | 549 |
| | Immediately After Contact | 1077 | 544 | 690 | 352 |
| | 1 | 1077 | 544 | 631 | 175 |
| | 2 | 1077 | 538 | 595 | 168 |
| | 3 | 1074 | 536 | 515 | 144 |
| | 12 | 1072 | 529 | 184 | 91 |
| | 24 | 1071 | 531 | 89 | 68 |
| | 48 | 1070 | 527 | 76 | 39 |
| | 72 | 1079 | 528 | 54 | 39 |
| | 168 | 1063 | 525 | 40 | 37 |
| | 504 | 1059 | 522 | 34 | 32 |
| | 720 | 1055 | 519 | 24 | 20 |

In this manner, it was found that an available chlorine concentration can be retained for a long period of time even if a chlorous acid aqueous solution formulation is diluted and impregnated into a treatment sheet.

(Example 5: Test for Examining Effects of Preventing Secondary Contamination upon Treating Vomit)

In the Present Example, each test was conducted to examine whether it is possible to prevent secondary contamination, such as microbes in vomit infecting the operator, when handling vomit by using a wet wipe in which the chlorous acid aqueous solution formulation obtained in Example 1 is impregnated into a treatment sheet.

<Testing Method>

(Materials)

1) Chlorous acid aqueous solution formulation (see Example 1: Table 11)
Treatment sheet (material: 100% cotton): (about 27 × 40 cm) * Impregnated at a ratio of weight of treatment sheet (g) : amount of liquid (ml) = 1:3
2) Imitation vomit (hereinafter, referred to as vomit) Miso solution: adjusted to pH 2 with hydrochloric acid
3) Test Microbes that were used
E. coli: Escherichia coli IF03927
Staphylococcus aureus: Staphylococcus aureus IF0 12732 Thermoduric Microbes (Bacillus cereus): Bacillus cereus NBRC15305 Microbes of the genus Salmonella : Salmonella Enteritidis IF03313 Enterohemorrhagic Escherichia coli 0157: Escherichia coli 0157 Enterohemorrhagic Escherichia coli 0111: Escherichia coli 0111 Enterohemorrhagic Escherichia coli 026: Escherichia coli 026

(Method)

<Testing Procedure>

Permeability of pathogenic microbes into a treatment sheet when treating vomit was examined to compare effects of preventing secondary contamination.

(1) Imitation vomit is spread in an area about the size of an A4 sheet.
(2) 30 agent solution-containing treatment sheets were laid thereon and left standing for 10 minutes.
(3) Treatment sheets were removed one at a time from the top. After diluting with saline, the sample was spread in a petri dish and cultured.

<Test Results>

TestsforComparingPermeabilityofPathogenicMicrobes (E. coli) when Treatment Sheets were Laid on Vomit

[Table 18]

| Stored at 25°C | Agent Solution | Chlorous Acid ($HClO_2$) Concentration | Number of Treatment Sheets Laid on Imitation Vomit | | | | | | | | | | | | |
|---|---|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | | 20 sheets | | 30 sheets |
| Day 0 | Tap Water | 0.4 ppm※ | $2.4\times10^7$ | $>10^6$ | $>10^6$ | $>10^6$ | $>10^6$ | $>10^6$ | $>10^6$ | $>10^6$ | $>10^6$ | | $>10^6$ | | $>10^6$ |
| | Alcohol (75%) | 0 ppm | $2.4\times10^7$ | $5.8\times10^6$ | $7.6x10^5$ | $2.3\times10^2$ | <10 | <10 | <10 | <10 | <10 | | <10 | | <10 |
| | Sodium Hypochlorite | 1000 ppm※ | $8.8\times10^6$ | <10 | <10 | <10 | <10 | <10 | <10 | <10 | <10 | | <10 | | <10 |
| | Chlorous Acid Aqueous Solution Formulation | 30,000 ppm | $2.4\times10^7$ | <10 | <10 | <10 | <10 | <10 | <10 | <10 | <10 | | <10 | | <10 |
| | | 10,000 ppm | $2.4\times10^7$ | <10 | <10 | <10 | <10 | <10 | <10 | <10 | <10 | | <10 | | <10 |
| | | 5,000 ppm | $2.4\times10^7$ | <10 | <10 | <10 | <10 | <10 | <10 | <10 | <10 | | <10 | | <10 |
| | | 3,000 ppm | $2.4\times10^7$ | <10 | <10 | <10 | <10 | <10 | <10 | <10 | <10 | | <10 | | <10 |
| | | 1,000 ppm | $2.4\times10^7$ | <10 | <10 | <10 | <10 | <10 | <10 | <10 | <10 | | <10 | | <10 |
| Day 7 | Tap Water | 0.4 ppm※ | $1.2\times10^7$ | $>10^6$ | $>10^6$ | $>10^6$ | $>10^6$ | $>10^6$ | $>10^6$ | $>10^6$ | $>10^6$ | | $>10^6$ | | $>10^6$ |
| | Alcohol (75%) | 0 ppm | $2.4\times10^7$ | $5.8\times10^6$ | $7.6\times10^5$ | $2.3\times10^2$ | <10 | <10 | <10 | <10 | <10 | | <10 | | <10 |
| | Sodium Hypochlorite | 1000 ppm※ | $1.2\times10^7$ | $>10^6$ | $>10^6$ | $>10^6$ | $>10^6$ | $>10^6$ | $>10^6$ | $>10^6$ | $>10^6$ | | $>10^6$ | | $>10^6$ |
| | Chlorous Acid Aqueous Solution Formulation | 30,000 ppm | $1.2\times10^7$ | <10 | <10 | <10 | <10 | <10 | <10 | <10 | <10 | | <10 | | <10 |
| | | 10,000 ppm | $12\times10^7$ | <10 | <10 | <10 | <10 | <10 | <10 | <10 | <10 | | <10 | | <10 |
| | | 5,000 ppm | $1.2\times10^7$ | <10 | <10 | <10 | <10 | <10 | <10 | <10 | <10 | | <10 | | <10 |
| | | 3,000 ppm | $1.2\times10^7$ | <10 | <10 | <10 | <10 | <10 | <10 | <10 | <10 | | <10 | | <10 |
| | | 1,000 ppm | $1.2\times10^7$ | <10 | <10 | <10 | <10 | <10 | <10 | <10 | <10 | | <10 | | <10 |

1

*Measured Value at Available Chlorine Concentration

Tests for Comparing Permeability of Pathogenic Microbes (St. aureus) when Treatment Sheets were Laid on Vomit

[Table 19]

| Stored at 25°C | Agent Solution | Chlorous Acid ($HClO_2$) Concentration | Number of Treatment Sheets Laid on Imitation Vomit | | | | | | | | | | | | |
|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | | 20 sheets | | 30 sheets |
| Day 0 | Tap Water | 0.4ppm※ | $1.5x10^{7}$ | $6.2x10^{4}$ | $1.1x10^{4}$ | $7.4x10^{3}$ | $3.1x10^{3}$ | $2.6x10^{2}$ | $8.1x10^{2}$ | $4.3x10^{2}$ | $1.2x10^{2}$ | | <10 | | <10 |
| | Alcohol (75%) | 0 ppm | $1.5x10^{7}$ | $6.9x10^{6}$ | $7.8x10^{5}$ | $1.3x10^{3}$ | $3.3x10^{2}$ | <10 | <10 | <10 | <10 | | <10 | | <10 |
| | Sodium Hypochlorite | 1000 ppm* | $9.1x10^{6}$ | <10 | <10 | <10 | <10 | <10 | <10 | <10 | <10 | | <10 | | <10 |
| | ChlorousAcid Aqueous Solution Formulation | 30,000 ppm | $1.5x10^{7}$ | <10 | <10 | <10 | <10 | <10 | <10 | <10 | <10 | | <10 | | <10 |
| | | 10,000 ppm | $1.5x10^{7}$ | <10 | <10 | <10 | <10 | <10 | <10 | <10 | <10 | | <10 | | <10 |
| | | 5,000 ppm | $1.5x10^{7}$ | <10 | <10 | <10 | <10 | <10 | <10 | <10 | <10 | | <10 | | <10 |
| | | 3,000 ppm | $1.5x10^{7}$ | <10 | <10 | <10 | <10 | <10 | <10 | <10 | <10 | | <10 | | <10 |
| | | 1,000 ppm | $1.5x10^{7}$ | $2.5x10^{2}$ | $2.6x10^{2}$ | <10 | <10 | <10 | <10 | <10 | <10 | | <10 | | <10 |
| Day 7 | Tap Water | 0.4 ppm* | $7.3x10^{6}$ | $>10^{6}$ | $>10^{6}$ | $>10^{6}$ | $>10^{6}$ | $>10^{6}$ | $>10^{6}$ | $>10^{6}$ | $>10^{6}$ | | $2.3x10^{2}$ | | <10 |
| | Alcohol (75%) | 0 ppm | $7.3x10^{6}$ | $5.1x10^{4}$ | $3.8x10^{4}$ | $2.6x10^{3}$ | $2.2x10^{2}$ | $1.8x10^{2}$ | $1.8x10^{2}$ | <10 | <10 | | <10 | | <10 |
| | Sodium Hypochlorite | 1000 ppm* | $7.3x10^{6}$ | $>10^{6}$ | $>10^{6}$ | $>10^{6}$ | $>10^{6}$ | $>10^{6}$ | $>10^{6}$ | $1.1x10^{4}$ | $7.1x10^{2}$ | | <10 | | <10 |
| | Chlorous Acid Aqueous Solution Formulation | 30,000 ppm | $7.3x10^{6}$ | <10 | <10 | <10 | <10 | <10 | <10 | <10 | <10 | | <10 | | <10 |
| | | 10,000 ppm | $7.3x10^{6}$ | <10 | <10 | <10 | <10 | <10 | <10 | <10 | <10 | | | | <10 |
| | | 5,000 ppm | $7.3x10^{6}$ | <10 | <10 | <10 | <10 | <10 | <10 | <10 | <10 | | <10 | | <10 |
| | | 3,000 ppm | $7.3x10^{6}$ | <10 | <10 | <10 | <10 | <10 | <10 | <10 | <10 | | <10 | | <10 |
| | | 1,000 ppm | $7.3x10^{6}$ | $1.2x10^{2}$ | $1,0x10^{2}$ | <10 | <10 | <10 | <10 | <10 | <10 | | <10 | | <10 |

*Measured Value at Available Chlorine Concentration

Tests for Comparing Permeability of Pathogenic Microbes (Thermoduric Microbes (Bacillus cereus)) when Treatment Sheets were Laid on Vomit

[Table 20]

| Stored at 25°C | Agent Solution | Chlorous Acid ($HClO_2$) Concentration | Number of Treatment Sheets Laid on Imitation Vomit | | | | | | | | | | | | |
|---|---|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | | 20 sheets | | 30 sheets |
| Day 0 | Tap Water | 0.4 ppm* | $1.2x10^7$ | $5.8x10^4$ | $8.0x10^3$ | $7.9x 10^3$ | $4,8x10^3$ | $8.9x10^2$ | $4.3x10^2$ | $1.3x10^2$ | 1.1x10 | | <10 | | <10 |
| | Alcohol (75%)) | 0 ppm | $1.2x10^7$ | $5,3x10^6$ | $4.9x10^5$ | $3.8x10^3$ | $1,6x10^2$ | <10 | <10 | <10 | <10 | | <10 | | <10 |
| | Sodium Hypochlorite | 1000 ppm* | $9.1x10^6$ | <10 | <10 | <10 | <10 | <10 | <10 | <10 | <10 | | <10 | | <10 |
| | ChlorousAcid Aqueous Solution Formulation | 30,000 ppm | $1.2x10^7$ | <10 | <10 | <10 | <10 | <10 | <10 | <10 | <10 | | <10 | | <10 |
| | | 10,000 ppm | $1.2x10^7$ | <10 | <10 | <10 | <10 | <10 | <10 | <10 | <10 | | <10 | | <10 |
| | | 5,000 ppm | $1.2x10^7$ | <10 | <10 | <10 | <10 | <10 | <10 | <10 | <10 | | <10 | | <10 |
| | | 3,000 ppm | $1.2x10^7$ | <10 | <10 | <10 | <10 | <10 | <10 | <10 | | | <10 | | |
| | | 1,000 ppm | $1.2x10^7$ | $1.2x10^2$ | $8.0x10^2$ | <10 | <10 | <10 | <10 | <10 | <10 | | <10 | | <10 |
| Day 7 | Tap Water | 0.4 ppm* | $7.3x10^6$ | $>10^6$ | $>10^6$ | $>10^6$ | $>10^6$ | $>10^6$ | $>10^6$ | $>10^6$ | $>10^6$ | | $2,3x10^2$ | | <10 |
| | Alcohol (75%) | 0 ppm | $7.3x10^6$ | $5.1x10^4$ | $3.8x10^4$ | $2.6x10^3$ | $2.2x10^2$ | $1.8x10^2$ | $1.8x10^2$ | <10 | <10 | | <10 | | <10 |
| | Sodium Hypochlorite | 1000 ppm* | $7.3x10^6$ | $>10^6$ | $>10^6$ | $>10^6$ | $>10^6$ | $>10^6$ | $>10^6$ | $1.1x10^4$ | $7.1x10^2$ | | <10 | | <10 |
| | Chlorous Acid Aqueous Solution Formulation | 30,000 ppm | $1.5x10^7$ | <10 | <10 | <10 | <10 | <10 | <10 | <10 | <10 | | <10 | | <10 |
| | | 10,000 ppm | $1.5x10^7$ | <10 | <10 | <10 | <10 | <10 | <10 | <10 | <10 | | <10 | | <10 |
| | | 5,000 ppm | $1.5x10^7$ | <10 | <10 | <10 | <10 | <10 | <10 | <10 | <10 | | <10 | | <10 |
| | | 3,000 ppm | $1.5x10^7$ | <10 | <10 | <10 | <10 | <10 | <10 | <10 | <10 | | <10 | | <10 |
| | | 1,000 ppm | $1.5x10^7$ | $2.5x10^2$ | $2.6x10^2$ | <10 | <10 | <10 | <10 | <10 | <10 | | <10 | | <10 |

*Measured Value at Available Chlorine Concentration

Tests for Comparing Permeability of Pathogenic Microbes (Microbes of Genus Salmonella: Salmonella Enteritidis IF03313) when Treatment Sheets were Laid on Vomit

[Table 21]

| Stored at 25°C | Agent Solution | Chlorous Acid ($HClO_2$) Concentration | Number of Treatment Sheets Laid on Imitation Vomit | | | | | | | | | | | | |
|---|---|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | | 20 sheets | | 30 sheets |
| Day 0 | Tap Water | 0.4 ppm* | $1.0x10^7$ | $>10^6$ | $>10^6$ | $>10^6$ | $>10^6$ | $>10^6$ | $8.8x10^4$ | $5.5x10^3$ | $1.0x10^3$ | | <10 | | <10 |
| | Alcohol (75%)) | 0 ppm | $1.2x10^7$ | $5.3x10^6$ | $4.9x10^5$ | $3.8x10^3$ | $1.6x10^2$ | <10 | <10 | <10 | <10 | | <10 | | <10 |
| | Sodium Hypochlorite | 1000 ppm* | $1.0x10^7$ | $2.0x10^2$ | <10 | <10 | <10 | <10 | <10 | <10 | <10 | | <10 | | <10 |
| | Chlorous Acid Aqueous Solution Formulation | 30,000 ppm | $1.0x10^7$ | <10 | <10 | <10 | <10 | <10 | <10 | <10 | <10 | | <10 | | <10 |
| | | 10,000 ppm | $1.0x10^7$ | <10 | <10 | <10 | <10 | <10 | <10 | <10 | <10 | | <10 | | <10 |
| | | 5,000 ppm | $1.0x10^7$ | <10 | <10 | <10 | <10 | <10 | <10 | <10 | <10 | | <10 | | <10 |
| | | 3,000 ppm | $1.0x10^7$ | <10 | <10 | <10 | <10 | <10 | <10 | <10 | <10 | | <10 | | <10 |
| | | 1,000 ppm | $1.0x10^7$ | $8.0x10^2$ | $2.6x10^2$ | <10 | <10 | <10 | <10 | <10 | <10 | | <10 | | <10 |
| Day 7 | Tap Water | 0.4 ppm※ | $8.7x10^6$ | $>10^6$ | $>10^6$ | $>10^6$ | $>10^6$ | $>10^6$ | $>10^6$ | $2.6x 10^4$ | $1.7x10^4$ | | $2.9x10^2$ | | <10 |
| | Alcohol (75%) | 0 ppm | $7.3x10^6$ | $5.1x10^4$ | $3.8x10^4$ | $2.6x10^3$ | $2.2x10^2$ | $1.8x10^2$ | $1.8x10^2$ | <10 | <10 | | <10 | | <10 |
| | Sodium Hypochlorite | 1000 ppm※ | $7.3x10^6$ | $>10^6$ | $>10^6$ | $>10^6$ | $>10^6$ | $>10^6$ | $>10^6$ | $1.1x10^4$ | $7.1x10^2$ | | <10 | | <10 |
| | Chlorous Acid Aqueous Solution Formulation | 30,000 ppm | $8.7x10^6$ | <10 | <10 | <10 | <10 | <10 | <10 | <10 | <10 | | <10 | | <10 |
| | | 10,000 ppm | $8.7x10^6$ | <10 | <10 | <10 | <10 | <10 | <10 | <10 | <10 | | <10 | | <10 |
| | | 5,000 ppm | $8.7x10^6$ | <10 | <10 | <10 | <10 | <10 | <10 | <10 | <10 | | <10 | | <10 |
| | | 3,000 ppm | $8.7x10^6$ | <10 | <10 | <10 | <10 | <10 | <10 | <10 | <10 | | <10 | | <10 |
| | | 1,000 ppm | $8.7x10^6$ | $9.1x10^2$ | $4.0x10^2$ | <10 | <10 | <10 | <10 | <10 | <10 | | <10 | | <10 |

*Measured Value at Available Chlorine Concentration

Tests for Comparing Permeability of Pathogenic Microbes (Enterohemorrhagic Escherichia coli 0157: Escherichia coli 0157) when Treatment Sheets were Laid on Vomit

[Table 22]

| Stored at 25°C | Agent Solution | Chlorous Acid ($HClO_2$) Concentration | Number of Treatment Sheets Laid on Imitation Vomit | | | | | | | | | | | |
|---|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | | 20 sheets | | 30 sheets |
| Day 0 | Tap Water | 0.4 ppm※ | $1.5x10^7$ | $>10^6$ | $>10^6$ | $>10^6$ | $>10^6$ | $>10^6$ | $>10^6$ | $>10^6$ | $>10^6$ | | $>10^6$ | | $>10^6$ |
| | Alcohol (75%) | 0 ppm | $1.5x10^7$ | $4.4x10^4$ | $9.2x10^5$ | $4.7x10^3$ | $2.6x 10^2$ | 5.0x10 | 4.0x10 | <10 | <10 | | <10 | | <10 |
| | Sodium Hypochlorite | 1000 ppm※ | $1.5x10^7$ | <10 | <10 | <10 | <10 | <10 | <10 | <10 | <10 | | <10 | | <10 |
| | Chlorous Acid Aqueous Solution Formulation | 30,000 ppm | $1.5x10^7$ | <10 | <10 | <10 | <10 | <10 | <10 | <10 | <10 | | <10 | | <10 |
| | | 10,000 ppm | $1.5x10^7$ | <10 | <10 | <10 | <10 | <10 | <10 | <10 | <10 | | <10 | | <10 |
| | | 5,000 ppm | $1.5x10^7$ | <10 | <10 | <10 | <10 | <10 | <10 | <10 | <10 | | <10 | | <10 |
| | | 3,000 ppm | $1.5x10^7$ | <10 | <10 | <10 | <10 | <10 | <10 | <10 | <10 | | <10 | | <10 |
| | | 1,000 ppm | $1.5x10^7$ | <10 | <10 | <10 | <10 | <10 | <10 | <10 | <10 | | <10 | | <10 |
| Day 7 | Tap Water | 0.4 ppm※ | $1.2x10^7$ | $>10^6$ | $>10^6$ | $>10^6$ | $>10^6$ | $>10^6$ | $>10^6$ | $>10^6$ | $>10^6$ | | $>10^6$ | | $>10^6$ |
| | Alcohol (75%) | 0 ppm | $1.2x10^7$ | $>10^6$ | $>10^6$ | $>10^6$ | $>10^6$ | $>10^6$ | $2.9x10^4$ | $9.0x10^2$ | $5.5x10^2$ | | <10 | | <10 |
| | Sodium Hypochlorite | 1000 ppm※ | $1.2x10^7$ | $>10^6$ | $>10^6$ | $>10^6$ | $>10^6$ | $>10^6$ | $>10^6$ | $1.1x10^4$ | $7.1x 10^2$ | | <10 | | <10 |
| | Chlorous Acid Aqueous Solution Formulation | 30,000 ppm | $1.2x10^7$ | <10 | <10 | <10 | <10 | <10 | <10 | <10 | <10 | | <10 | | <10 |
| | | 10,000 ppm | $1.2x10^7$ | <10 | <10 | <10 | <10 | <10 | <10 | <10 | <10 | | <10 | | <10 |
| | | 5,000 ppm | $1.2x10^7$ | <10 | <10 | <10 | <10 | <10 | <10 | <10 | <10 | | <10 | | <10 |
| | | 3,000 ppm | $1.2x10^7$ | <10 | <10 | <10 | <10 | <10 | <10 | <10 | <10 | | <10 | | <10 |
| | | 1,000 ppm | $1.2x10^7$ | <10 | <10 | <10 | <10 | <10 | <10 | <10 | <10 | | <10 | | <10 |

*Measured Value at Available Chlorine Concentration

Tests for Comparing Permeability of Pathogenic Microbes (Enterohemorrhagic Enterohemorrhagic Escherichia coli 0111: Escherichia coli 0111) when Treatment Sheets were Laid on Vomit

[Table 23]

| Stored at 25°C | Agent Solution | Chlorous Acid ($HClO_2$) Concentration | Number of Treatment Sheets Laid on Imitation Vomit | | | | | | | | | | |
|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | 20 sheets | 30 sheets |
| Day 0 | Tap Water | 0.4 ppm※ | $8.1x10^6$ | $>10^6$ | $>10^6$ | $>10^6$ | $>10^6$ | $>10^6$ | $>10^6$ | $>10^6$ | $>10^6$ | $>10^6$ | $>10^6$ |
| | Alcohol (75%) | 0 ppm | $8.7x10^6$ | $7.9x10^3$ | $6.7x 10^5$ | $4.4x10^3$ | $2.1x10^2$ | <10 | <10 | <10 | <10 | <10 | <10 |
| | Sodium Hypochlorite | 1000 ppm※ | $8.7x10^6$ | <10 | <10 | <10 | <10 | <10 | <10 | <10 | <10 | <10 | <10 |
| | ChlorousAcid Aqueous Solution Formulation | 30,000 ppm | $8.7x10^6$ | <10 | <10 | <10 | <10 | <10 | <10 | <10 | <10 | <10 | <10 |
| | | 10,000 ppm | $8.7x10^6$ | <10 | <10 | <10 | <10 | <10 | <10 | <10 | <10 | <10 | <10 |
| | | 5,000 ppm | $8.7x10^6$ | <10 | <10 | <10 | <10 | <10 | <10 | <10 | <10 | <10 | <10 |
| | | 3,000 ppm | $8.7x10^6$ | <10 | <10 | <10 | <10 | <10 | <10 | <10 | <10 | <10 | <10 |
| | | 1,000 ppm | $8.7x10^6$ | <10 | <10 | <10 | <10 | <10 | <10 | <10 | <10 | <10 | <10 |
| Day 7 | Tap Water | 0.4 ppm※ | $9.8x10^6$ | $>10^6$ | $>10^6$ | $>10^6$ | $>10^6$ | $>10^6$ | $>10^6$ | $>10^6$ | $>10^6$ | $>10^6$ | $>10^6$ |
| | Alcohol (75%) | 0 ppm | $9.8x10^6$ | $>10^6$ | $>10^6$ | $>10^6$ | $>10^6$ | $>10^6$ | $3.4x10^4$ | $1.1x10^2$ | $1.0x10^2$ | <10 | <10 |
| | Sodium Hypochlorite | 1000 ppm※ | $9.8x10^6$ | $>10^6$ | $>10^6$ | $>10^6$ | $>10^6$ | $>10^6$ | $>10^6$ | $>10^6$ | $>10^6$ | $>10^6$ | $>10^6$ |
| | Chlorous Acid Aqueous Solution Formulation | 30,000 ppm | $9.8x10^6$ | <10 | <10 | <10 | <10 | <10 | <10 | <10 | <10 | <10 | <10 |
| | | 10,000 ppm | $9.8x10^6$ | <10 | <10 | <10 | <10 | <10 | <10 | <10 | <10 | <10 | <10 |
| | | 5,000 ppm | $9.8x10^6$ | <10 | <10 | <10 | <10 | <10 | <10 | <10 | <10 | <10 | <10 |
| | | 3,000 ppm | $9.8x10^6$ | <10 | <10 | <10 | <10 | <10 | <10 | <10 | <10 | <10 | <10 |
| | | 1,000 ppm | $9.8x10^6$ | <10 | <10 | <10 | <10 | <10 | <10 | <10 | <10 | <10 | <10 |

*Measured Value at Available Chlorine Concentration

Tests for Comparing Permeability of Pathogenic Microbes (Enterohemorrhagic Escherichia coli 026: Escherichia coli 026) when Treatment Sheets were Laid on Vomit

[Table 24]

| Stored at 25°C | Agent Solution | Chlorous Acid ($HClO_2$) Concentration | Number of Treatment Sheets Laid on Imitation Vomit | | | | | | | | | | |
|---|---|---|---|---|---|---|---|---|---|---|---|---|---|
| | | | 0 sheet | 1 sheet | 2 sheets | 3 sheets | 4 sheets | 5 sheets | 6 sheets | 8 sheets | 10 sheets | 20 .. sheets | 30 sheets |
| Day 0 | Tap Water | 0.4 ppm※ | $8.7x10^6$ | $>10^6$ | $>10^6$ | $>10^6$ | $>10^6$ | $>10^6$ | $>10^6$ | $>10^6$ | $>10^6$ | $>10^6$ | $>10^6$ |
| | Alcohol (75%) | 0 ppm | $8.7x10^6$ | $7.9x10^3$ | $6.7x10^5$ | $4.4x10^3$ | $2.1 x10^2$ | <10 | <10 | <10 | <10 | <10 | <10 |
| | Sodium Hypochlorite | 1000 ppm※ | $8.7x10^6$ | <10 | <10 | <10 | <10 | <10 | <10 | <10 | <10 | <10 | <10 |
| | Chlorous Acid Aqueous Solution Formulation | 30,000 ppm | $8.7x10^6$ | <10 | <10 | <10 | <10 | <10 | <10 | <10 | <10 | <10 | <10 |
| | | 10,000 ppm | $8.7x10^6$ | <10 | <10 | <10 | <10 | <10 | <10 | <10 | <10 | <10 | <10 |
| | | 5,000 ppm | $8.1x10^6$ | <10 | <10 | <10 | <10 | <10 | <10 | <10 | <10 | <10 | <10 |
| | | 3,000 ppm | $8.7x10^6$ | <10 | <10 | <10 | <10 | <10 | <10 | <10 | <10 | <10 | <10 |
| | | 1,000 ppm | $8,1x10^6$ | <10 | <10 | <10 | <10 | <10 | <10 | <10 | <10 | <10 | <10 |
| Day 7 | Tap Water | 0.4 ppm※ | $9.8x10^6$ | $>10^6$ | $>10^6$ | $>10^6$ | $>10^6$ | $>10^6$ | $>10^6$ | $>10^6$ | $>10^6$ | $>10^6$ | $>10^6$ |
| | Alcohol (75%) | 0 ppm | $9.8x10^6$ | $>10^6$ | $>10^6$ | $>10^6$ | $>10^6$ | $>10^6$ | $3.4x10^4$ | $1.1x10^2$ | $1.0x10^2$ | <10 | <10 |
| | Sodium Hypochlorite | 1000 ppm※ | $9.8x10^6$ | $>10^6$ | $>10^6$ | $>10^6$ | $>10^6$ | $>10^6$ | $>10^6$ | $>10^6$ | $>10^6$ | $>10^6$ | $>10^6$ |
| | Chlorous Acid Aqueous Solution Formulation | 30,000 ppm | $9.8x10^6$ | <10 | <10 | <10 | <10 | <10 | <10 | <10 | <10 | <10 | <10 |
| | | 10,000 ppm | $9.8x10^6$ | <10 | <10 | <10 | <10 | <10 | <10 | <10 | <10 | <10 | <10 |
| | | 5,000 ppm | $9.8x10^6$ | <10 | <10 | <10 | <10 | <10 | <10 | <10 | <10 | <10 | <10 |
| | | 3,000 ppm | $9.8x10^6$ | <10 | <10 | <10 | <10 | <10 | <10 | <10 | <10 | <10 | <10 |
| | | 1,000 ppm | $9.8x10^6$ | <10 | <10 | <10 | <10 | <10 | <10 | <10 | <10 | <10 | <10 |

*Measured Value at Available Chlorine Concentration

Treatment sheets impregnated with tap water were contaminated by pathogenic microbes even when 30 sheets were stacked. With treatment sheets impregnated with 75% alcohol, imitation vomit could not be treated without contamination unless treated by stacking 5 or more sheets. Further, sodium hypochlorite was able to sterilize pathogenic microbes from the first sheet. However, odor of imitation vomit and sodium hypochlorite was severe such that the indoor environment rapidly deteriorated. Thus, it was difficult to treat the imitation vomit using sodium hypochlorite. For the chlorous acid aqueous solution formulation, pathogenic microbes were able to be removed from the first sheet at any concentration. Even after preservation for 7 days, the sterilizing effect thereof did not deteriorate. Further, when imitation vomit was covered with a treatment sheet impregnated with a chlorous acid aqueous solution formulation, barely any odor was detected and the imitation vomit could readily be wiped off.

(Example 6: Test for Examining Sterilizing Effect on Floor Surface When Treating Vomit)

In the present Example, tests for examining the sterilizing effects on a floor surface when treating vomit were conducted.

Each experiment was conducted to examine sterilizing effects upon treating vomit on a floor surface by using a wet wipe in which a chlorous acid aqueous solution diluent was impregnated in a treatment sheet.

<Testing Method>

(Materials)

1) Chlorous acid aqueous solution formulation (see Example 1: Table 11)
Treatment sheet (material: 100% cotton): (about 27 × 40 cm) *Impregnated at a ratio of weight of treatment sheet (g) : amount of liquid (ml) = 1:3
2) Imitation vomit Miso solution: adjusted to pH of 2 with hydrochloric acid
3) Test Microbes that were Used
E. coli: Escherichia coli IF03927
Staphylococcus aureus: Staphylococcus aureus IFO 12732 Thermoduric Microbes (Bacillus cereus): Bacillus cereus NBRC15305 Microbes of the genus Salmonella : Salmonella Enteritidis IF03313 Enterohemorrhagic Escherichia coli 0157: Escherichia coli 0157 Enterohemorrhagic Escherichia coli 0111: Escherichia coli 0111 Enterohemorrhagic Escherichia coli 026: Escherichia coli 026

(Method)

<Testing Procedure>

<Rubber Mattress>

(1) Imitation vomit was spread in an area about the size of an A4 sheet.
(2) 5 agent solution-containing treatment sheets were laid thereon.
(3) The imitation vomit was wiped off.
(4) A cotton swab was soaked with sterile saline. A portion where imitation vomit was spread was scrubbed with the cotton swab to collect microbes remaining on the floor. After suspending in saline, the solution was spread on a petri dish and cultured. The number of each of the microbes was measured.
(5) Test operations 3 and 4 were repeated a total of 7 times (repeated until the imitation vomit could be removed completely) .

<Tatami Mattress>

(1) Imitation vomit was spread in an area about the size of an A4 sheet.

(2) 5 dry sheets were laid thereon.
(3) An agent solution was poured on the dry sheets to wipe off the imitation vomit.
(4) A cotton swab was soaked with sterile saline. A portion where imitation vomit was spread was scrubbed with the cotton swab to collect microbes remaining on the floor. After suspending in saline, the solution was spread on a petri dish and cultured. The number of each of the microbes was measured.
(5) Test operations 3 and 4 were repeated a total of 7 times (repeated until the imitation vomit could be removed completely) .

<Carpet>

(1) Imitation vomit was spread in an area about the size of an A4 sheet.
(2) 5 dry sheets were laid thereon.
(3) An agent solution was poured on the dry sheets to wipe off the imitation vomit.
(4) A cotton swab was soaked with sterile saline. A portion where imitation vomit was spread was scrubbed with the cotton swab to collect microbes remaining on the floor. After suspending in saline, the solution was spread on a petri dish and cultured. The number of each of the microbes was measured.
(5) Test operations 3 and 4 were repeated a total of 7 times (repeated until the imitation vomit could be removed completely) .

Pictures of Examples of each procedure for rubber mattress, tatami mattress, and carpet are shown in Figure 15.

<Test Results>

Tables summarizing individual test results are shown below.

(Results for Rubber Mattress)

Results of Tests for Examining Sterilizing Effects on "E. Coli" Remaining on Floor Surface after Wiping off Imitation Vomit

[Table 25]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $1.9x10^7$ | $2.3x10^4$ | $4.8x10^3$ | $9.2x10^2$ | | - |
| | Alcohol (75%) | 0 ppm | $1.9x10^7$ | $2.3x10^4$ | $4.8x10^3$ | $9.2x10^2$ | | <10 |
| | Sodium Hypochlorite | 1000 ppm※ | $1.9x10^7$ | <10 | <10 | <10 | | - |
| | Chlorous Acid Aqueous Solution Formulation | 30,000 ppm | $1.9x10^7$ | <10 | <10 | <10 | | - |
| | | 10,000 ppm | $1.9x10^7$ | <10 | <10 | <10 | | - |
| | | 5,000 ppm | $1.9x10^7$ | <10 | <10 | <10 | | - |
| | | 3,000 ppm | $1.9x10^7$ | <10 | <10 | <10 | | - |
| | | 1,000 ppm | $1.9x10^7$ | <10 | <10 | <10 | | - |
| | | 500 ppm | $1.9x10^7$ | <10 | <10 | <10 | | - |
| Day 7 | Tap Water | 0.4 ppm※ | $1.2x10^7$ | $>10^6$ | $>10^6$ | $5.6x10^4$ | | - |
| | Alcohol (75%) | 0 ppm | $1.2x10^7$ | $4.3x10^4$ | $7.2x10^3$ | $1.2x10^3$ | | <10 |
| | Sodium Hypochlorite | 1000 ppm※ | $9.7x10^7$ | $2.3x10^4$ | $7.8x10^3$ | $2.1x10^3$ | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $9.7x10^7$ | <10 | <10 | <10 | | - |
| | | 10,000 ppm | $9.7x10^7$ | <10 | <10 | <10 | | - |
| | | 5,000 ppm | $9.7x10^7$ | <10 | <10 | <10 | | - |
| | | 3,000 ppm | $9.7x10^7$ | <10 | <10 | <10 | | - |
| | | 1,000 ppm | $9.7x10^7$ | <10 | <10 | <10 | | - |
| | | 500 ppm | $9.7x10^7$ | <10 | <10 | <10 | | - |

*Measured Value at Available Chlorine Concentration

Results of Tests for Examining Sterilizing Effects on "Staphylococcus aureus" Remaining on Floor Surface after Wiping off Imitation Vomit

[Table 26]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $1.1x10^7$ | $5.6x10^3$ | $3.8x10^2$ | $1.4x10^2$ | | - |
| | Alcohol (75%) | 0 ppm | $1.1x10^7$ | $8.3x10^5$ | $2.7x10^3$ | $1.5x10^2$ | | <10 |
| | Sodium Hypochlorite | 1000 ppm※ | $1.0x10^7$ | <10 | <10 | <10 | | - |
| | Chlorous Acid Aqueous Solution Formulation | 30,000 ppm | $1.1x10^7$ | <10 | <10 | <10 | | - |
| | | 10,000 ppm | $1.1x10^7$ | <10 | <10 | <10 | | - |
| | | 5,000 ppm | $1.1x10^7$ | <10 | <10 | <10 | | - |
| | | 3,000 ppm | $1.1x10^7$ | <10 | <10 | <10 | | - |
| | | 1,000 ppm | $1.1x10^7$ | <10 | <10 | <10 | | - |
| | | 500 ppm | $1.1x10^7$ | $1.1x10^2$ | <10 | <10 | | - |
| Day 7 | Tap Water | 0.4 ppm※ | $7.3x10^6$ | $5.1x10^4$ | $2.2x10^4$ | $6.4x10^3$ | | - |
| | Alcohol (75%) | 0 ppm | $7.3x10^6$ | $6.2x10^3$ | $1.6x10^3$ | $1.0x10^3$ | | <10 |
| | Sodium Hypochlorite | 1000 ppm※ | $8.7x10^6$ | $8.7x10^6$ | $7.3x10^3$ | $3.3x10^3$ | | - |
| | Chlorous Acid Aqueous Solution Formulation | 30,000 ppm | $8.7x10^6$ | <10 | <10 | <10 | | - |
| | | 10,000 ppm | $8.7x10^6$ | <10 | <10 | <10 | | - |
| | | 5,000 ppm | $8.7x10^6$ | <10 | <10 | <10 | | - |
| | | 3,000 ppm | $8.7x10^6$ | <10 | <10 | <10 | | - |
| | | 1,000 ppm | $8.7x10^6$ | <10 | <10 | <10 | | - |
| | | 500 ppm | $8.7x10^6$ | <10 | <10 | <10 | | - |

*Measured Value at Available Chlorine Concentration

Results of Tests for Examining Sterilizing Effects on "Bacillus cereus". Remaining on Floor Surface after Wiping off Imitation Vomit

[Table 27]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $1.4x10^7$ | $2.7x10^3$ | $1.6x10^3$ | $5.2x10^2$ | | - |
| | Alcohol (75%) | 0 ppm | $1.0x10^7$ | $4.1x10^3$ | $2.9x10^3$ | $1.1x10^3$ | | $3.9x10^3$ |
| | Sodium Hypochlorite | 1000 ppm※ | $7.2x10^6$ | <10 | <10 | <10 | | - |
| | Chlorous Acid Aqueous Solution Formulation | 30,000 ppm | $1.4x10^7$ | <10 | <10 | <10 | | - |
| | | 10,000 ppm | $1.4x10^7$ | <10 | <10 | <10 | | - |
| | | 5,000 ppm | $1.4x10^7$ | <10 | <10 | <10 | | - |
| | | 3,000 ppm | $1.4x10^7$ | <10 | <10 | <10 | | - |
| | | 1,000 ppm | $1.4x10^7$ | <10 | <10 | <10 | | - |
| | | 500 ppm | $1.4x10^7$ | $1.5x10^2$ | <10 | <10 | | - |
| Day 7 | Tap Water | 0.4 ppm※ | $1.0x10^7$ | $9.6x10^5$ | $1.3x10^3$ | $1.1x10^2$ | | - |
| | Alcohol (75%) | 0 ppm | $1.0x10^7$ | $4.9x10^3$ | $3.6x10^3$ | $2.1x10^3$ | | $3.3x10^3$ |
| | Sodium Hypochlorite | 1000 ppm※ | $8.9x10^5$ | $2.1x10^3$ | $6.5x10^2$ | $1.8x10^2$ | | - |
| | Chlorous Acid Aqueous Solution Formulation | 30,000 ppm | $8.9x10^6$ | <10 | <10 | <10 | | - |
| | | 10,000 ppm | $8.9x10^6$ | <10 | <10 | <10 | | - |
| | | 5,000 ppm | $8.9x10^6$ | <10 | <10 | <10 | | - |
| | | 3,000 ppm | $8.9x10^6$ | <10 | <10 | <10 | | - |
| | | 1,000 ppm | $8.9x10^6$ | <10 | <10 | <10 | | - |
| | | 500 ppm | $8.9x10^6$ | $1.0x10^2$ | <10 | <10 | | - |

*Measured Value at Available Chlorine Concentration

Results of Tests for Examining Sterilizing Effects on "Microbes of the genus Salmonella" Remaining on Floor Surface after Wiping off Imitation Vomit

[Table 28]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $9.2x10^6$ | $5.5x10^3$ | $2.1x10^2$ | <10 | | - |
| | Alcohol (75%) | 0 ppm | $1.4x10^7$ | $9.6x10^5$ | $1.3x10^3$ | $1.1x10^2$ | | <10 |
| | Sodium Hypochlorite | 1000 ppm※ | $6.7x10^6$ | <10 | <10 | <10 | | - |
| | Chlorous Acid Aqueous Solution Formulation | 30,000 ppm | $1.4x10^7$ | <10 | <10 | <10 | | - |
| | | 10,000 ppm | $1.4x10^7$ | <10 | <10 | <10 | | - |
| | | 5,000 ppm | $1.4x10^7$ | <10 | <10 | <10 | | - |
| | | 3,000 ppm | $1.4x10^7$ | <10 | <10 | <10 | | - |
| | | 1,000 ppm | $1.4x10^7$ | <10 | <10 | <10 | | - |
| | | 500 ppm | $1.4x10^7$ | $1.5x10^2$ | <10 | <10 | | - |
| Day 7 | Tap Water | 0.4 ppm※ | $7.3x10^6$ | $5.1x10^4$ | $2.2x10^4$ | $6.4x10^3$ | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Alcohol (75%) | 0 ppm | $7.3 \times 10^6$ | $6.2 \times 10^3$ | $1.6 \times 10^3$ | $1.0 \times 10^3$ | | <10 |
| | Sodium Hypochlorite | 1000 ppm※ | $7.1 \times 10^6$ | $1.1 \times 10^3$ | $3.6 \times 10^2$ | 7.0x10 | | - |
| | Chlorous Acid Aqueous Solution Formulation | 30,000 ppm | $9.2 \times 10^6$ | <10 | <10 | <10 | | - |
| | | 10,000 ppm | $9.2 \times 10^6$ | <10 | <10 | <10 | | - |
| | | 5,000 ppm | $9.2 \times 10^6$ | <10 | <10 | <10 | | - |
| | | 3,000 ppm | $9.2 \times 10^6$ | <10 | <10 | <10 | | - |
| | | 1,000 ppm | $9.2 \times 10^6$ | <10 | <10 | <10 | | - |
| | | 500 ppm | $9.2 \times 10^6$ | 4.0x10 | <10 | <10 | | - |

*Measured Value at Available Chlorine Concentration

Results of Tests for Examining Sterilizing Effects on "Enterohemorrhagic Escherichia coli 0157" Remaining on Floor Surface after Wiping off Imitation Vomit

[Table 29]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $1.2 \times 10^7$ | $6.1 \times 10^5$ | $3.3 \times 10^3$ | $1.0 \times 10^3$ | | - |
| | Alcohol (75%) | 0 ppm | $1.4 \times 10^7$ | $9.6 \times 10^3$ | $1.3 \times 10^3$ | $1.1 \times 10^2$ | | <10 |
| | Sodium Hypochlorite | 1000 ppm※ | $4.1 \times 10^6$ | <10 | <10 | <10 | | - |
| | Chlorous Acid Aqueous Solution Formulation | 30,000 ppm | $1.4 \times 10^7$ | <10 | <10 | <10 | | - |
| | | 10,000 ppm | $1.4 \times 10^7$ | <10 | <10 | <10 | | - |
| | | 5,000 ppm | $1.4 \times 10^7$ | <10 | <10 | <10 | | - |
| | | 3,000 ppm | $1.4 \times 10^7$ | <10 | <10 | <10 | | - |
| | | 1,000 ppm | $1.4 \times 10^7$ | <10 | <10 | <10 | | - |
| | | 500 ppm | $1.4 \times 10^7$ | $1.5 \times 10^2$ | <10 | <10 | | - |
| Day 7 | Tap Water | 0.4 ppm※ | $1.2 \times 10^7$ | $6.1 \times 10^5$ | $7.3 \times 10^3$ | $2.9 \times 10^3$ | | - |
| | Alcohol (75%) | 0 ppm | $4.1 \times 10^6$ | $1.0 \times 10^4$ | $6.5 \times 10^3$ | $1.6 \times 10^3$ | | <10 |
| | Sodium Hypochlorite | 1000 ppm※ | $3.7 \times 10^6$ | $4.2 \times 10^3$ | $1.2 \times 10^3$ | $1.1 \times 10^2$ | | - |
| | Chlorous Acid Aqueous Solution Formulation | 30,000 ppm | $3.7 \times 10^6$ | <10 | <10 | <10 | | - |
| | | 10,000 ppm | $3.7 \times 10^6$ | <10 | <10 | <10 | | - |
| | | 5,000 ppm | $3.7 \times 10^6$ | <10 | <10 | <10 | | - |
| | | 3,000 ppm | $3.7 \times 10^6$ | <10 | <10 | <10 | | - |
| | | 1,000 ppm | $3.7 \times 10^6$ | <10 | <10 | <10 | | - |
| | | 500 ppm | $3.7 \times 10^6$ | <10 | <10 | <10 | | - |

*Measured Value at Available Chlorine Concentration

Results of Tests for Examining Sterilizing Effects on "Enterohemorrhagic Escherichia coli 0111" Remaining on Floor Surface after Wiping off Imitation Vomit

[Table 30]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | 1.4x10$^{7}$ | 2.7x10$^{3}$ | <10 | <10 | | - |
| | Alcohol (75%) | 0 ppm | 1.4x10$^{7}$ | 9.6x10$^{3}$ | 1.3x10$^{3}$ | 1.1x10$^{2}$ | | <10 |
| | Sodium Hypochlorite | 1000 ppm※ | 1.4x10$^{7}$ | <10 | <10 | <10 | | - |
| | Chlorous Acid Aqueous Solution Formulation | 30,000 ppm | 1.4x10$^{7}$ | <10 | <10 | <10 | | - |
| | | 10,000 ppm | 1.4x10’ | <10 | <10 | <10 | | - |
| | | 5,000 ppm | 1.4x10$^{7}$ | <10 | <10 | <10 | | - |
| | | 3,000 ppm | 1.4x10$^{7}$ | <10 | <10 | <10 | | - |
| | | 1,000 ppm | 1.4x10’ | <10 | <10 | <10 | | - |
| | | 500 ppm | 1.4x10$^{7}$ | 1.5x10$^{2}$ | <10 | <10 | | - |
| Day 7 | Tap Water | 0.4 ppm※ | 9.8x10$^{6}$ | 4.2x10$^{3}$ | 1.5x10$^{3}$ | 1.1x10$^{3}$ | | - |
| | Alcohol (75%) | 0 ppm | 9.8x10$^{6}$ | 9.3x10$^{3}$ | 6.1x10$^{3}$ | 2.7x10$^{3}$ | | - |
| | Sodium Hypochlorite | 1000 ppm | 6.0x10$^{6}$ | 1.5x10$^{3}$ | 1.0x10$^{3}$ | 2.6x10$^{2}$ | | <10 |
| | Chlorous Acid Aqueous Solution Formulation | 30,000 ppm | 60x10$^{6}$ | <10 | <10 | <10 | | - |
| | | 10,000 ppm | 6.0x10$^{6}$ | <10 | <10 | <10 | | - |
| | | 5,000 ppm | 6.0x10$^{6}$ | <10 | <10 | <10 | | - |
| | | 3,000 ppm | 6.0x10$^{6}$ | <10 | <10 | <10 | | - |
| | | 1,000 ppm | 6.0x10$^{6}$ | <10 | <10 | <10 | | - |
| | | 500 ppm | 6.0x10$^{6}$ | 1.5x10$^{2}$ | <10 | <10 | | - |

*Measured Value at Available Chlorine Concentration

Results of Tests for Examining Sterilizing Effects on "Enterohemorrhagic Escherichia coli 026" Remaining on Floor Surface after Wiping off Imitation Vomit

[Table 31]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | 1.4x10$^{7}$ | 2.7x10$^{3}$ | 1.7x10$^{3}$ | 4.1x10$^{2}$ | | - |
| | Alcohol (75%) | ppm※ | 1.4x10$^{7}$ | 9.6x10$^{5}$ | 1.3x10$^{3}$ | 1.1x10$^{2}$ | | <10 |
| | Sodium Hypochlorite | 1000 ppm※ | 1.4x10$^{7}$ | <10 | <10 | <10 | | |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $1.4x10^7$ | <10 | <10 | <10 | | - |
| | | 10,000 ppm | $1.4x10^7$ | <10 | <10 | <10 | | - |
| | | 5,000 ppm | $1.4x10^7$ | <10 | <10 | <10 | | - |
| | | 3,000 ppm | $1.4x10^7$ | <10 | <10 | <10 | | - |
| | | 1,000 ppm | $1.4x10^7$ | <10 | <10 | <10 | | - |
| | | 500 ppm | $1.4x10^7$ | $1.5x10^2$ | <10 | <10 | | - |
| Day 7 | Tap Water | 0.4 ppm※ | $1.3x10^7$ | $9.5x10^3$ | $6.2x10^3$ | $3.1x10^3$ | | - |
| | Alcohol (75%) | 0 ppm | $1.3x10^7$ | $8.8x10^3$ | $4.9x10^3$ | $3.0x10^3$ | | <10 |
| | Sodium Hypochlorite | 1000 ppm※ | $3.8x10^6$ | $3.2x10^3$ | $1.0x10^3$ | $3.0x10^2$ | | - |
| | Chlorous Acid Aqueous Solution Formulation | 30,000 ppm | $3.8x10^6$ | <10 | <10 | <10 | | - |
| | | 10,000 ppm | $3.8x10^6$ | <10 | <10 | <10 | | - |
| | | 5,000 ppm | $3.8x10^6$ | <10 | <10 | <10 | | - |
| | | 3,000 ppm | $3.8x10^6$ | <10 | <10 | <10 | | - |
| | | 1,000 ppm | $3.8x10^6$ | <10 | <10 | <10 | | - |
| | | 500 ppm | $3.8x10^6$ | <10 | <10 | <10 | | - |

*Measured Value at Available Chlorine Concentration

(Results for Tatami Mattress)

Results of Tests for Examining Sterilizing Effects on "E. Coli" Remaining on Floor Surface after Wiping off Imitation Vomit

[Table 32]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $6.4x10^6$ | $8.6x10^4$ | $5.1x10^3$ | $4.4x10^3$ | | - |
| | Alcohol (75%) | 0 ppm | $6.4x10^6$ | $9.6x10^3$ | $1.3x10^3$ | $1.1x10^2$ | | <10 |
| | Sodium Hypochlorite | 1000 ppm※ | $6.4x10^6$ | <10 | <10 | <10 | | - |
| | Chlorous Acid Aqueous Solution Formulation | 30,000 ppm | $6.4x10^6$ | <10 | <10 | <10 | | - |
| | | 10,000 ppm | $6.4x10^6$ | <10 | <10 | <10 | | - |
| | | 5,000 ppm | $6.4x10^6$ | <10 | <10 | <10 | | - |
| | | 3,000 ppm | $6.4x10^6$ | <10 | <10 | <10 | | - |
| | | 1,000 ppm | $6.4x10^6$ | <10 | <10 | <10 | | - |
| | | 500 ppm | $6.4x10^6$ | $2.0x10^2$ | <10 | <10 | | - |
| Day 7 | Tap Water | 0.4 ppm※ | $6.0x10^6$ | $4.8x10^4$ | $8.1x10^3$ | $5.6x10^3$ | | - |
| | Alcohol (75%) | 0 ppm | $6.0x10^6$ | $6.2x10^4$ | $7.8x10^3$ | $4.6x10^3$ | | <10 |
| | Sodium Hypochlorite | 1000 ppm※ | $6.0x10^6$ | <10 | <10 | <10 | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $6.0x10^6$ | <10 | <10 | <10 | | - |
| | | 10,000 ppm | $6.0x10^6$ | <10 | <10 | <10 | | - |
| | | 5,000 ppm | $6.0x10^6$ | <10 | <10 | <10 | | - |
| | | 3,000 ppm | $6.0x10^6$ | <10 | <10 | <10 | | - |
| | | 1,000 ppm | $6.0x10^6$ | <10 | <10 | <10 | | - |
| | | 500 ppm | $6.0x10^6$ | $1.7x10^2$ | <10 | <10 | | - |

*Measured Value at Available Chlorine Concentration

Results of Tests for Examining Sterilizing Effects on "Staphylococcus aureus" Remaining on Floor Surface after Wiping off Imitation Vomit

[Table 33]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $5.3x10^6$ | $2.1x10^4$ | $1.0x10^3$ | $2.1x10^3$ | | - |
| | Alcohol (75%) | 0 ppm | $5.3x10^6$ | $4.9x10^3$ | $2.8x10^3$ | $1.4x10^3$ | | <10 |
| | Sodium Hypochlorite | 1000 ppm※ | $5.3x10^6$ | <10 | <10 | <10 | | - |
| | Chlorous Acid Aqueous Solution Formulation | 30,000 ppm | $5.3x10^6$ | <10 | <10 | <10 | | - |
| | | 10,000 ppm | $5.3x10^6$ | <10 | <10 | <10 | | - |
| | | 5,000 ppm | $5.3x10^6$ | <10 | <10 | <10 | | - |
| | | 3,000 ppm | $5.3x10^6$ | <10 | <10 | <10 | | - |
| | | 1,000 ppm | $5.3x10^6$ | <10 | <10 | <10 | | - |
| | | 500 ppm | $5.3x10^6$ | $2.0x10^2$ | $1.2x10^2$ | <10 | | - |
| Day 7 | Tap Water | 0.4 ppm※ | $7.3x10^6$ | $4.0x10^4$ | $3.8 x10^3$ | $3.1x10^3$ | | - |
| | Alcohol (75%) | 0 ppm | $7.3x10^6$ | $7.4x10^3$ | $3.6x10^3$ | $3.3x10^3$ | | <10 |
| | Sodium Hypochlorite | 1000 ppm※ | $7.3x10^6$ | <10 | <10 | <10 | | - |
| | Chlorous Acid Aqueous Solution Formulation | 30,000 ppm | $7.3x10^6$ | <10 | <10 | <10 | | - |
| | | 10,000 ppm | $7.3x10^6$ | <10 | <10 | <10 | | - |
| | | 5,000 ppm | $7.3x10^6$ | <10 | <10 | <10 | | - |
| | | 3,000 ppm | $7.3x10^6$ | <10 | <10 | <10 | | - |
| | | 1,000 ppm | $7.3x10^6$ | <10 | <10 | <10 | | - |
| | | 500 ppm | $7.3x10^6$ | <10 | <10 | <10 | | - |

*Measured Value at Available Chlorine Concentration

Results of Tests for Examining Sterilizing Effects on "Bacillus cereus" Remaining on Floor Surface after Wiping off Imitation Vomit

[Table 34]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $2.8x10^6$ | $3.1x10^4$ | $1.8x10^3$ | $2.6x10^3$ | | - |
| | Alcohol (75%) | 0 ppm | $2.8x10^6$ | $4.9x10^3$ | $2.8x10^3$ | $1.4x10^3$ | | $1.1x10^3$ |
| | Sodium Hypochlorite | 1000 ppm※ | $2.8x10^6$ | <10 | <10 | <10 | | - |
| | Chlorous Acid Aqueous Solution Formulation | 30,000 ppm | $2.8x10^6$ | <10 | <10 | <10 | | - |
| | | 10,000 ppm | $2.8x10^6$ | <10 | <10 | <10 | | - |
| | | 5,000 ppm | $2.8x10^6$ | <10 | <10 | <10 | | - |
| | | 3,000 ppm | $2.8x10^6$ | <10 | <10 | <10 | | - |
| | | 1,000 ppm | $2.8x10^6$ | <10 | <10 | <10 | | - |
| | | 500 ppm | $2.8x10^6$ | $3.5x10^2$ | 7.0x10 | <10 | | - |
| Day 7 | Tap Water | 0.4 ppm※ | $8.9x10^6$ | $9.6x10^5$ | $1.3x10^3$ | $1.1x10^2$ | | - |
| | Alcohol (75%) | 0 ppm | $8.9x10^6$ | $4.8x10^3$ | $4.2x10^2$ | $3.9x10^2$ | | $2.1x10^3$ |
| | Sodium Hypochlorite | 1000 ppm※ | $8.9x10^6$ | <10 | <10 | <10 | | - |
| | Chlorous Acid Aqueous Solution Formulation | 30,000 ppm | $8.9x10^6$ | <10 | <10 | <10 | | - |
| | | 10,000 ppm | $8.9x10^6$ | <10 | <10 | <10 | | - |
| | | 5,000 ppm | $8.9x10^6$ | <10 | <10 | <10 | | - |
| | | 3,000 ppm | $8.9x10^6$ | <10 | <10 | <10 | | - |
| | | 1,000 ppm | $8.9x10^6$ | <10 | <10 | <10 | | - |
| | | 500 ppm | $8.9x10^6$ | $1.0x10^2$ | <10 | <10 | | - |

*Measured Value at Available Chlorine Concentration

Results of Tests for Examining Sterilizing Effects on "Microbes of Genus Salmonella" Remaining on Floor Surface after Wiping off Imitation Vomit

(Floor)

[Table 35]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm* | $1.2x10^7$ | $5.5x10^3$ | $2.1x10^2$ | <10 | | - |
| | Alcohol (75%) | 0 ppm | $1.2x10^7$ | $2.6x10^3$ | $2.0x10^3$ | $2.3x10^3$ | | <10 |
| | Sodium Hypochlorite | 1000 ppm* | $1,2x10^7$ | <10 | <10 | <10 | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $1.2x10^7$ | <10 | <10 | <10 | | - |
| | | 10,000 ppm | $1.2x10^7$ | <10 | <10 | <10 | | - |
| | | 5,000 ppm | $1.2x10^7$ | <10 | <10 | <10 | | - |
| | | 3,000 ppm | $1.2x10^7$ | <10 | <10 | <10 | | - |
| | | 1,000 ppm | $1.2x10^7$ | <10 | <10 | <10 | | - |
| | | 500 ppm | $1.2x10^7$ | $1.0x10^2$ | <10 | <10 | | - |
| Day 7 | Tap Water | 0.4 ppm* | $9.2x10^6$ | $7.3x10^3$ | 5.9x103 | $3.5x10^3$ | | - |
| | Alcohol (75%) | 0 ppm | $9.2x10^6$ | **$3.9x10^3$** | **$3.3x10^3$** | **$2.3x10^3$** | | <10 |
| | Sodium Hypochlorite | 1000 ppm※ | $9.2x10^6$ | **$1.5x10^3$** | **$7.7x10^2$** | **$1.8x10^2$** | | - |
| | Chlorous Acid Aqueous Solution Formulation | 30,000 ppm | $9.2x10^6$ | <10 | <10 | <10 | | - |
| | | 10,000 ppm | $9.2x10^6$ | <10 | <10 | <10 | | - |
| | | 5,000 ppm | $9.2x10^6$ | <10 | <10 | <10 | | - |
| | | 3,000 ppm | $9.2x10^6$ | <10 | <10 | <10 | | - |
| | | 1,000 ppm | $9.2x10^6$ | <10 | <10 | <10 | | - |
| | | 500 ppm | $9.2x10^6$ | $1.6x10^2$ | <10 | <10 | | - |

*Measured Value at Available Chlorine Concentration

Results of Tests for Examining Sterilizing Effects on "Enterohemorrhagic Escherichia coli 0157" Remaining on Floor Surface after Wiping off Imitation Vomit

[Table 36]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $8.1\times10^6$ | $1.2\times10^4$ | $3.1\times10^3$ | $2.9\times10^3$ | | - |
| | Alcohol (75%) | 0 ppm | $8.1\times10^6$ | $4.2\times10^3$ | $4.1\times10^3$ | $4.0\times10^3$ | | <10 |
| | Sodium Hypochlorite | 1000 ppm※ | $8.1\times10^6$ | <10 | <10 | <10 | | - |
| | Chlorous Acid Aqueous Solution Formulation | 30,000 ppm | $8.1\times10^6$ | <10 | <10 | <10 | | - |
| | | 10,000 ppm | $8.1\times10^6$ | <10 | <10 | <10 | | - |
| | | 5,000 ppm | $8.1\times10^6$ | <10 | <10 | <10 | | - |
| | | 3,000 ppm | $8.1\times10^6$ | <10 | <10 | <10 | | - |
| | | 1,000 ppm | $8.1\times10^6$ | <10 | <10 | <10 | | - |
| | | 500 ppm | $8.1\times10^6$ | $1.5\times10^2$ | <10 | <10 | | - |
| Day 7 | Tap Water | 0.4 ppm※ | $3.7\times10^6$ | $6.1\times10^5$ | $7.3\times10^3$ | $2.9\times10^3$ | | - |
| | Alcohol (75%) | 0 ppm | $3.7\times10^6$ | $3.9\times10^3$ | $3.6\times10^3$ | $2.9\times10^3$ | | <10 |
| | Sodium Hypochlorite | 1000 ppm※ | $3.7\times10^6$ | <10 | <10 | <10 | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $3.7\times10^6$ | <10 | <10 | <10 | | - |
| | | 10,000 ppm | $3.7\times10^6$ | <10 | <10 | <10 | | - |
| | | 5,000 ppm | $3.7\times10^6$ | <10 | <10 | <10 | | - |
| | | 3,000 ppm | $3.7\times10^6$ | <10 | <10 | <10 | | - |
| | | 1,000 ppm | $3.7\times10^6$ | <10 | <10 | <10 | | - |
| | | 500 ppm | $3.7\times10^6$ | <10 | <10 | <10 | | - |

*Measured Value at Available Chlorine Concentration

Results of Tests for Examining Sterilizing Effects on "Enterohemorrhagic Escherichia coli 0111" Remaining on Floor Surface after Wiping off Imitation Vomit

[Table 37]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $5.2\times10^6$ | $7.4\times10^3$ | $1.2\times10^3$ | $9.7\times10^2$ | | - |
| | Alcohol (75%) | 0 ppm | $5.2\times10^6$ | $2.6\times10^3$ | $1.5\times10^3$ | $1.3\times10^3$ | | <10 |
| | Sodium Hypochlorite | 1000 ppm | $5.2\times10^6$ | <10 | <10 | <10 | | - |
| | Chlorous Acid Aqueous Solution Formulation | 30,000 ppm | $5.2\times10^6$ | <10 | <10 | <10 | | - |
| | | 10,000 ppm | $5.2\times10^6$ | <10 | <10 | <10 | | - |
| | | 5,000 ppm | $5.2\times10^6$ | <10 | <10 | <10 | | - |
| | | 3,000 ppm | $5.2\times10^6$ | <10 | <10 | <10 | | - |
| | | 1,000 ppm | $5.2\times10^6$ | <10 | <10 | <10 | | - |
| | | 500 ppm | $5.2\times10^6$ | $1.0\times10^2$ | <10 | <10 | | - |
| Day 7 | Tap Water | 0.4 ppm※ | $6.0\times10^6$ | $3.2\times10^3$ | $2.5\times10^3$ | $2.1\times10^3$ | | - |
| | Alcohol (75%) | 0 ppm | $6.0\times10^6$ | $3.5\times10^3$ | $2.0\times10^3$ | $1.6\times10^2$ | | <10 |
| | Sodium Hypochlorite | 1000 ppm | $6.0\times10^6$ | <10 | <10 | <10 | | - |
| | Chlorous Acid Aqueous Solution Formulation | 30,000 ppm | $6.0\times10^6$ | <10 | <10 | <10 | | - |
| | | 10,000 ppm | $6.0\times10^6$ | <10 | <10 | <10 | | - |
| | | 5,000 ppm | $6.0\times10^6$ | <10 | <10 | <10 | | - |
| | | 3,000 ppm | $6.0\times10^6$ | <10 | <10 | <10 | | - |
| | | 1,000 ppm | $6.0\times10^6$ | <10 | <10 | <10 | | - |
| | | 500 ppm | $6.0\times10^6$ | $1.1\times10^2$ | <10 | <10 | | - |

*Measured Value at Available Chlorine Concentration

Results of Tests for Examining Sterilizing Effects on "Enterohemorrhagic Escherichia coli 026" Remaining on Floor Surface after Wiping off Imitation Vomit

[Table 38]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $4.7\times10^6$ | $2.7\times10^3$ | <10 | <10 | | - |
| | Alcohol (75%) | 0 ppm | $4.7\times10^6$ | $1.6\times10^3$ | $1.5\times10^3$ | $1.0\times10^2$ | | <10 |
| | Sodium Hypochlorite | 1000 ppm※ | $4.7\times10^6$ | <10 | <10 | <10 | | - |
| | Chlorous Acid Aqueous Solution Formulation | 30,000 ppm | $4.7\times10^6$ | <10 | <10 | <10 | | - |
| | | 10,000 ppm | $4.7\times10^6$ | <10 | <10 | <10 | | - |
| | | 5,000 ppm | $4.7\times10^6$ | <10 | <10 | <10 | | - |
| | | 3,000 ppm | $4.7\times10^6$ | <10 | <10 | <10 | | - |
| | | 1,000 ppm | $4.7\times10^6$ | <10 | <10 | <10 | | - |
| | | 500 ppm | $4.7\times10^6$ | $1.3\times10^2$ | <10 | <10 | | - |
| Day 7 | Tap Water | 0.4 ppm※ | $3.8\times10^6$ | $8.5\times10^3$ | $7.0\times10^3$ | $4.9\times10^3$ | | - |
| | Alcohol (75%) | 0 ppm | $3.8\times10^6$ | $2.2\times10^3$ | $1.8\times10^3$ | $9.4\times10^2$ | | <10 |
| | Sodium Hypochlorite | 1000 ppm※ | $3.8\times10^6$ | $8.8\times10^3$ | $4.9\times10^3$ | $3.0\times10^3$ | | - |
| | Chlorous Acid Aqueous Solution Formulation | 30,000 ppm | $3.8\times10^6$ | <10 | <10 | <10 | | - |
| | | 10,000 ppm | $3.8\times10^6$ | <10 | <10 | <10 | | - |
| | | 5,000 ppm | $3.8\times10^6$ | <10 | <10 | <10 | | - |
| | | 3,000 ppm | $3.8\times10^6$ | <10 | <10 | <10 | | - |
| | | 1,000 ppm | $3.8\times10^6$ | <10 | <10 | <10 | | - |
| | | 500 ppm | $3.8\times10^6$ | <10 | <10 | <10 | | - |

*Measured-Value at Available Chlorine Concentration

(Carpet)

Results of Tests for Examining Sterilizing Effects on "E. Coli" Remaining on Floor Surface after Wiping off Imitation Vomit

[Table 39]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $6.4\times10^6$ | $8.6\times10^4$ | $5.1\times10^3$ | $4.4\times10^3$ | | - |
| | Alcohol (75%) | 0 ppm | $6.4\times10^6$ | $3.9\times10^3$ | $3.3\times10^3$ | $2.0\times10^3$ | | <10 |
| | Sodium Hypochlorite | 1000 ppm※ | $6.4\times10^6$ | <10 | <10 | <10 | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $6.4\times10^6$ | <10 | <10 | <10 | | - |
| | | 10,000 ppm | $6.4\times10^6$ | <10 | <10 | <10 | | - |
| | | 5,000 ppm | $6.4\times10^6$ | <10 | <10 | <10 | | - |
| | | 3,000 ppm | $6.4\times10^6$ | <10 | <10 | <10 | | - |
| | | 1,000 ppm | $6.4\times10^6$ | <10 | <10 | <10 | | - |
| | | 500 ppm | $6.4\times10^6$ | $8.0\times10$ | <10 | <10 | | - |
| Day 7 | Tap Water | 0.4 ppm※ | $6.0\times10^6$ | $9,1\times10^4$ | $5.9\times10^3$ | $3.6\times10^3$ | | - |
| | Alcohol (75%) | 0 ppm | $6.0\times10^6$ | $4.2\times10^4$ | $7.5\times10^3$ | $4.3\times10^3$ | | <10 |
| | Sodium Hypochlorite | 1000 ppm※ | $6.0\times10^6$ | <10 | <10 | <10 | | - |
| | Chlorous Acid Aqueous Solution Formulation | 30,000 ppm | $6.0\times10^6$ | <10 | <10 | <10 | | - |
| | | 10,000 ppm | $6.0\times10^6$ | <10 | <10 | <10 | | - |
| | | 5,000 ppm | $6.0\times10^6$ | <10 | <10 | <10 | | - |
| | | 3,000 ppm | $6.0\times10^6$ | <10 | <10 | <10 | | - |
| | | 1,000 ppm | $6.0\times10^6$ | <10 | <10 | <10 | | - |
| | | 500 ppm | $6.0\times10^6$ | $1.3\times10^2$ | <10 | <10 | | - |

*Measured Value at Available Chlorine Concentration

Results of Tests for Examining Sterilizing Effects on "Staphylococcus aureus" Remaining on Floor Surface after Wiping off Imitation Vomit

[Table 40]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $5.3\times10^6$ | $5.3\times10^3$ | $4.4\times10^3$ | $3.8\times10^3$ | | - |
| | Alcohol (75%) | 0 ppm | $5.3\times10^6$ | $4.3\times10^3$ | $5.7\times10^3$ | $2.2\times10^3$ | | <10 |
| | Sodium Hypochlorite | 1000 ppm※ | $5.3\times10^6$ | <10 | <10 | <10 | | - |
| | Chlorous Acid Aqueous Solution Formulation | 30,000 ppm | $5.3\times10^6$ | <10 | <10 | <10 | | - |
| | | 10,000 ppm | $5.3\times10^6$ | <10 | <10 | <10 | | - |
| | | 5,000 ppm | $5.3\times10^6$ | <10 | <10 | <10 | | - |
| | | 3,000 ppm | $5.3\times10^6$ | <10 | <10 | <10 | | - |
| | | 1,000 ppm | $5.3\times10^6$ | <10 | <10 | <10 | | - |
| | | 500 ppm | $5.3\times10^6$ | 60x10 | $1.2\times10^2$ | <10 | | - |
| Day 7 | Tap Water | 0.4 ppm※ | $7.3\times10^6$ | $5.6\times10^4$ | $3.2\times10^3$ | $1.8\times10^3$ | | - |
| | Alcohol (75%) | 0 ppm | $7.3\times10^6$ | $3.4\times10^3$ | $1.9\times10^3$ | $1.3\times10^3$ | | <10 |
| | Sodium Hypochlorite | 1000 ppm※ | $7.3\times10^6$ | <10 | <10 | <10 | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| | Chlorous Acid Aqueous Solution Formulation | 30,000 ppm | $7.3\times10^6$ | <10 | <10 | <10 | | - |
| | | 10,000 ppm | $7.3\times10^6$ | <10 | <10 | <10 | | - |
| | | 5,000 ppm | $7.3\times10^6$ | <10 | <10 | <10 | | - |
| | | 3,000 ppm | $7.3\times10^6$ | <10 | <10 | <10 | | - |
| | | 1,000 ppm | $7.3\times10^6$ | <10 | <10 | <10 | | - |
| | | 500 ppm | $7.3\times10^6$ | <10 | <10 | <10 | | - |

*Measured Value at Available Chlorine Concentration

Results of Tests for Examining Sterilizing Effects on "Bacillus cereus" Remaining on Floor Surface after Wiping off Imitation Vomit

[Table 41]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $2.8\times10^6$ | $4.1\times10^3$ | $1.9\times10^3$ | $2.0\times10^3$ | | - |
| | Alcohol (75%) | 0 ppm | $2.8\times10^6$ | $4.9\times10^3$ | $3.3\times10^3$ | $2.6\times10^3$ | | $1.8\times10^3$ |
| | Sodium Hypochlorite | 1000 ppm※ | $2.8\times10^6$ | <10 | <10 | <10 | | - |
| | Chlorous Acid Aqueous Solution Formulation | 30,000 ppm | $2.8\times10^6$ | <10 | <10 | <10 | | - |
| | | 10,000 ppm | $2.8\times10^6$ | <10 | <10 | <10 | | - |
| | | 5,000 ppm | $2.8\times10^6$ | <10 | <10 | <10 | | - |
| | | 3,000 ppm | $2.8\times10^6$ | <10 | <10 | <10 | | - |
| | | 1,000 | $2.8\times10^6$ | <10 | <10 | <10 | | - |
| | | 500 ppm | $2.8\times10^6$ | $1.0\times10^2$ | <10 | <10 | | - |
| Day 7 | Tap Water | 0.4 ppm※ | $8.9\times10^6$ | $7.5\times10^5$ | $5.3\times10^3$ | $2.6\times10^2$ | | - |
| | Alcohol (75%) | 1000 ppm※ | $8.9\times10^6$ | $4.9\times10^3$ | $3.7\times10^2$ | $6.2\times10^2$ | | - |
| | Sodium Hypochlorite | 0 ppm | $8.9\times10^6$ | $6.7\times10^3$ | $5.2\times10^3$ | $4.9\times10^3$ | | $2.0\times10^3$ |
| | Chlorous Acid Aqueous Solution Formulation | 30,000 ppm | $8.9\times10^6$ | <10 | <10 | <10 | | - |
| | | 10,000 ppm | $8.9\times10^6$ | <10 | <10 | <10 | | - |
| | | 5,000 ppm | $8.9\times10^6$ | <10 | <10 | <10 | | - |
| | | 3,000 ppm | $8.9\times10^6$ | <10 | <10 | <10 | | - |
| | | 1,000 ppm | $8.9\times10^6$ | <10 | <10 | <10 | | - |
| | | 500 ppm | $8.9\times10^6$ | $1.0\times10^2$ | <10 | <10 | | - |

*Measured Value at Available Chlorine Concentration

Results of Tests for Examining Sterilizing Effects on "Microbes of the Genus Salmonella" Remaining on Floor

Surface after Wiping off Imitation Vomit

[Table 42]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $1.2\times10^7$ | $7.2\times10^3$ | $5.1\times10^3$ | $8.0\times10^2$ | | - |
| | Alcohol (75%) | 0 ppm | $1.2\times10^7$ | $8.0\times10^3$ | $5.1\times10^3$ | $3.3\times10^3$ | | <10 |
| | Sodium Hypochlorite | 1000 ppm※ | $1.2\times10^7$ | <10 | <10 | <10 | | - |
| | Chlorous Acid Aqueous Solution Formulation | 30,000 ppm | $1.2\times10^7$ | <10 | <10 | <10 | | - |
| | | 10,000 ppm | $1.2\times10^7$ | <10 | <10 | <10 | | - |
| | | 5,000 ppm | $1.2\times10^7$ | <10 | <10 | <10 | | - |
| | | 3, 000 ppm | $1.2\times10^7$ | <10 | <10 | <10 | | - |
| | | 1,000 ppm | $1.2\times10^7$ | <10 | <10 | <10 | | - |
| | | 500 ppm | $1.2\times10^7$ | $1.1\times10^2$ | <10 | <10 | | - |
| Day 7 | Tap Water | 0.4 ppm※ | $9.2\times10^6$ | $4.9\times10^3$ | $3.2\times10^3$ | $1.4\times10^3$ | | - |
| | Alcohol (75%) | 0 ppm | $9.2\times10^6$ | $2.4\times10^3$ | $1.7\times10^3$ | $1.2\times10^3$ | | <10 |
| | Sodium | 1000 ppm※ | $9.2\times10^6$ | <10 | <10 | <10 | | - |
| | Chlorous Acid Aqueous Solution Formulation | 30,000 ppm | $9.2\times10^6$ | <10 | <10 | <10 | | - |
| | | 10,000 ppm | $9.2\times10^6$ | <10 | <10 | <10 | | - |
| | | 5,000 ppm | $9.2\times10^6$ | <10 | <10 | <10 | | - |
| | | 3,000 ppm | $9.2\times10^6$ | <10 | <10 | <10 | | - |
| | | 1,000 ppm | $9.2\times10^6$ | <10 | <10 | <10 | | - |
| | | 500 ppm | $9.2\times10^6$ | $1.5\times10^2$ | <10 | <10 | | - |

*Measured Value at Available Chlorine Concentration

Results of Tests for Examining Sterilizing Effects on "Enterohemorrhagic Escherichia coli 0157" Remaining on Floor Surface after Wiping off Imitation Vomit

[Table 43]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $8.1\times10^6$ | $6.1\times10^3$ | $3.7\times10^3$ | $3.1\times10^3$ | | - |
| | Alcohol (75%) | 0 ppm | $8.1\times10^6$ | $4.2\times10^3$ | $4.1\times10^3$ | $4.0\times10^3$ | | <10 |
| | Sodium Hypochlorite | 1000 ppm※ | $8.1\times10^6$ | <10 | <10 | <10 | | - |
| | Chlorous Acid Aqueous Solution Formulation | 30,000 ppm | $8.1\times10^6$ | <10 | <10 | <10 | | - |
| | | 10,000 ppm | $8.1\times10^6$ | <10 | <10 | <10 | | - |
| | | 5,000 ppm | $8.1\times10^6$ | <10 | <10 | <10 | | - |
| | | 3,000 ppm | $8.1\times10^6$ | <10 | <10 | <10 | | - |
| | | 1,000 ppm | $8.1\times10^6$ | <10 | <10 | <10 | | - |
| | | 500 ppm | $8.1\times10^6$ | $1.0\times10^2$ | <10 | <10 | | - |

(continued)

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 7 | Tap Water | 0.4 ppm※ | $3.7\times10^6$ | $6.3\times10^3$ | $4.2\times10^3$ | $2.8\times10^3$ | | - |
| | Alcohol (75%) | 0 ppm | $3.7\times10^6$ | $3.9\times10^3$ | $2.4\times10^3$ | $1.3\times10^3$ | | <10 |
| | Sodium Hypochlorite | 1000 ppm※ | $3.7\times10^6$ | <10 | <10 | <10 | | - |
| | Chlorous Acid Aqueous Solution Formulation | 30,000 ppm | $3.7\times10^6$ | <10 | <10 | <10 | | - |
| | | 10,000 ppm | $3.7\times10^6$ | <10 | <10 | <10 | | - |
| | | 5,000 ppm | $3.7\times10^6$ | <10 | <10 | <10 | | - |
| | | 3,000 ppm | $3.7\times10^6$ | <10 | <10 | <10 | | - |
| | | 1,000 ppm | $3.7\times10^6$ | <10 | <10 | <10 | | - |
| | | 500 ppm | $3.7\times10^6$ | $1.0\times10^2$ | <10 | <10 | | - |

*Measured Value at Available Chlorine Concentration

Results of Tests for Examining Sterilizing Effects on "Enterohemorrhagic Escherichia coli 0111" Remaining on Floor Surface after Wiping off Imitation Vomit

[Table 44]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $5.2\times10^6$ | $9.1\times10^3$ | $6.8\times10^3$ | $3.2\times10^3$ | | - |
| | Alcohol (75%) | 0 ppm | $5.2\times10^6$ | $2.5\times10^3$ | $1.5\times10^3$ | $1.3\times10^3$ | | <10 |
| | Sodium Hypochlorite | 1000 ppm | $5.2\times10^6$ | <10 | <10 | <10 | | - |
| | Chlorous Acid Aqueous Solution Formulation | 30,000 ppm | $5.2\times10^6$ | <10 | <10 | <10 | | - |
| | | 10,000 ppm | $5.2\times10^6$ | <10 | <10 | <10 | | - |
| | | 5,000 ppm | $5.2\times10^6$ | <10 | <10 | <10 | | - |
| | | 3,000 ppm | $5.2\times10^6$ | <10 | <10 | <10 | | - |
| | | 1,000 ppm | $5.2\times10^6$ | <10 | <10 | <10 | | - |
| | | 500 ppm | $5.2\times10^6$ | $9.0\times10$ | <10 | <10 | | - |
| Day 7 | Tap Water | 0.4 ppm※ | $6.0\times10^6$ | $2.6\times10^3$ | $1.4\times10^3$ | $1.0\times10^3$ | | - |
| | Alcohol (75%) | 1000 ppm | $6.0\times10^6$ | $3.3\times10^3$ | $2.7\times10^3$ | $2.0\times10^2$ | | <10 |
| | Sodium Hypochlorite | 0 ppm | $6.0\times10^6$ | $2.3\times10^3$ | $1.9\times10^3$ | $1.1\times10^3$ | | - |
| | Chlorous Acid Aqueous Solution Formulation | 30,000 ppm | $6.0\times10^6$ | <10 | <10 | <10 | | - |
| | | 10,000 ppm | $6.0\times10^6$ | <10 | <10 | <10 | | - |
| | | 5,000 ppm | $6.0\times10^6$ | <10 | <10 | <10 | | - |
| | | 3,000 ppm | $6.0\times10^6$ | <10 | <10 | <10 | | - |
| | | 1,000 ppm | $6.0\times10^6$ | <10 | <10 | <10 | | - |
| | | 500 ppm | $6.0\times10^6$ | $1.0\times10^2$ | <10 | <10 | | - |

*Measured Value at Available Chlorine Concentration

Results of Tests for Examining Sterilizing Effects on "Enterohemorrhagic Escherichia coli 026" Remaining on Floor Surface after Wiping off Imitation Vomit

[Table 45]

| Stored at 25°C | Agent Solution | Chlorous Acid ($HClO_2$) Concentration | Number of Times Wiped | | | | | |
|---|---|---|---|---|---|---|---|---|
| | | | 0 Time | 1 Time | 2 Times | 3 Times | | 7 Times |
| Day 0 | Tap Water | 0.4 ppm※ | $4.7\times10^6$ | $2.7\times10^3$ | $1.9\times10^3$ | $5.8\times10^2$ | | - |
| | Alcohol (75%) | 0 ppm | $4.7\times10^6$ | $1.6\times10^3$ | $1.5\times10^3$ | $1.0\times10^2$ | | <10 |
| | Sodium Hypochlorite | 1000 ppm※ | $4.7\times10^6$ | <10 | <10 | <10 | | - |
| | Chlorous Acid Aqueous Solution Formulation | 30,000 ppm | $4.7\times10^6$ | <10 | <10 | <10 | | - |
| | | 10,000 ppm | $4.7\times10^6$ | <10 | <10 | <10 | | - |
| | | 5,000 ppm | $4.7\times10^6$ | <10 | <10 | <10 | | - |
| | | 3,000 ppm | $4.7\times10^6$ | <10 | <10 | <10 | | - |
| | | 1,000 ppm | $4.7\times10^6$ | <10 | <10 | <10 | | - |
| | | 500 ppm | $4.7\times10^6$ | $1.3\times10^2$ | <10 | <10 | | - |
| Day 7 | Tap Water | 0.4 ppm※ | $3.8\times110^6$ | $8.5\times10^3$ | $7.0\times10^3$ | $4.9\times10^3$ | | - |
| | Alcohol (75%) | 0 ppm | $3.8\times10^6$ | $2.2\times10^3$ | $1.8\times10^3$ | $9.4\times10^2$ | | <10 |
| | Sodium Hypochlorite | 1000 ppm※ | $3.8\times10^6$ | $8.8\times10^3$ | $4.9\times10^3$ | $3.0\times10^3$ | | - |
| | Chlorous Acid Aqueous Solution Formulation | 30,000 ppm | $3.8\times10^6$ | <10 | <10 | <10 | | - |
| | | 10,000 ppm | $3.8\times10^6$ | <10 | <10 | <10 | | - |
| | | 5,000 ppm | $3.8\times10^6$ | <10 | <10 | <10 | | - |
| | | 3,000 ppm | $3.8\times10^6$ | <10 | <10 | <10 | | - |
| | | 1,000 ppm | 3.8x106 | <10 | <10 | <10 | | - |
| | | 500 ppm | $3.8\times10^6$ | <10 | <10 | <10 | | - |

*Measured Value at Available Chlorine Concentration

With a treatment sheet impregnated with tap water, pathogenic microbes could not be sterilized after one wipe. Similarly, a treatment sheet impregnated with 75 % alcohol could not sterilize pathogenic microbes. Further, in a case of sodium hypochlorite, pathogenic microbes could be sterilized on day 0. However, when preserved for 7 days, there were cases in which pathogenic microbes could not be sterilized. For a chlorous acid aqueous solution formulation, pathogenic microbes could be removed with the first sheet at any concentration. Even after 7 days of preservation, the sterilizing effect thereof did not deteriorate.

Further, sensual impressions of the experimenter during the experiment in the present Example were summarized.

[Table 46]

| Agent Used | Chlorous Acid Concentration(ppm) or Available Chlorine Concentration (ppm) | | Impression upon Wiping | Environmental Change | Remarks |
|---|---|---|---|---|---|
| Tap Water | (Immediately after impregnation) | 0 | Every wipe of vomit only spread the contaminating microbes | none | none in particular |
| | (Storage for one week at room temperature) | 0 | Every wipe of vomit only spread the contaminating microbes | Water had a putrid odor | mold grew on the sheet |
| Chlorous Acid Aqueous Solution Formulation | (Immediately after impregnation) | 30000 | It was possible to wipe off the vomit cleanly | none | Removal of vomit and microbes could be carried out together |
| | (Immediately after impregnation) | 10000 | It was possible to wipe off the vomit cleanly | none | Removal of vomit and microbes could be carried out together |
| | (Immediately after impregnation) | 5000 | It was possible to wipe off the vomit cleanly | none | Removal of vomit and microbes could be carried out together |
| | (Immediately after impregnation) | 3000 | It was possible to wipe off the vomit cleanly | none | Removal of vomit and microbes could be carried out together |
| | (Immediately after impregnation) | 1000 | It was possible to wipe off the vomit cleanly | none | Removal of vomit and microbes could be carried out together |
| | (Immediately after impregnation) | 500 | It was possible to wipe off the vomit cleanly | none | Removal of vomit and microbes could be carried out together |
| | (Storage for one week at room temperature) | 30000 | It was possible to wipe off the vomit cleanly | none | There was no change from Day 1 even after storage |
| | (Storage for one week at room temperature) | 10000 | It was possible wipe off the vomit cleanly | none | There was no change from Day 1 even after storage |

(continued)

| Agent Used | Chlorous Acid Concentration(ppm) or Available Chlorine Concentration (ppm) | | Impression upon Wiping | Environmental Change | Remarks |
|---|---|---|---|---|---|
| | (Storage for one week at room temperature) | 5000 | It was possible to wipe off the vomit cleanly | none | There was no change from Day 1 even after storage |
| | (Storage for one week at room temperature) | 3000 | It was possible to wipe off the vomit cleanly | none | There was no change from Day 1 even after storage |
| | (Storage for one week at room temperature) | 1000 | It was possible to wipe off the vomit cleanly | none | There was no change from Day 1 even after storage |
| | (Storage for one week at room temperature) | 500 | It was possible to wipe off the vomit cleanly | none | There was no change from Day 1 even after storage |
| Sodium Hypochlorite | (Immediately after impregnation | 1000 ppm | It was possible to wipe off the vomit | There was a strong chlorine odor | Odor spread throughout the room. The odor remained on hands and fingers. |
| | (Storage for one week at room temperature) | 1000 ppm | Lint or the like was spread over the floor due to deterioration of the sheet | There was a strong chlorine odor | It was not possible to wipe, as the sheet became worn and tattered |
| 75 (w/w)% Alcohol | (Immediately after impregnation) | 0 | The vomit was difficult to wipe off, as it solidified. Residuals remained, which could not be wiped off. | Alcoholic odor | Vomit and contaminating microbes could not be readily removed |
| | (Storage for one week at room temperature) | 0 | The vomit was difficult to wipe off, as it solidified. Residuals remained, which could not be wiped off. | Alcoholic odor could removed | Vomit and contaminating microbes could not be readily removed |

(Example 7: Tests for Examining the Presence of Sterilizing Effects When Diluted to Each Dilution Factor and Impregnated into Treatment Sheet)

The present Example was intended to examine the presence of sterilizing effects of a chlorous acid aqueous solution diluted to each chlorous acid concentration.

<Testing Method>

(Materials)

1) Chlorous acid aqueous solution formulation (see Example 1: Table 11)

Treatment sheet (material: 100% cotton): (about 27 × 40 cm) *Impregnated at a ratio of weight of treatment sheet (g) : amount of liquid (ml) = 1:3

2) Test Microbes that were Used

E. coli: Escherichia coli IF03927

Staphylococcus aureus: Staphylococcus aureus IF0 12732 Microbes of the genus Salmonella: Salmonella Enteritidis IF03313

(Method)

(Testing Procedure)

(1) Treatment sheets were impregnated with a diluted solution of a "chlorous acid aqueous solution formulation" at each dilution factor.

(2) Each treatment sheet impregnated with a test solution was wrung to collect the impregnating solution as a sample solution for testing.

(3) 1.0 ml of concentrated suspension of each tested microbe was added to 9.0 ml of each sample solution for testing and the mixture was mixed homogeneously. The mixture was then stored in a 25°C water bath and mixed homogenously again every 1, 5, and 10 minutes to collect 9.0 ml of each solution.

(4) The collected solution was added to 1.0 ml of sterilized 0.1 mol/L sodium thiosulfate solution (prepared with various buffer) and mixed homogeneously. After the mixture was further left standing for 10 minutes, 1.0 ml thereof was apportioned to each of the two petri dishes. The number of surviving microbes was then measured according to a common method by pour-plate culture.

(Results)

The results thereof are shown below.

Table of Ranges of Concentrations for Using Chlorous AcidAqueous Solution

[Table 47]

| Dilution Factor of Impregnation | Chlorous Acid ($HClO_2$) Concentration ※ | Sterilizing Effect on Each Pathogenic Microbe | | |
|---|---|---|---|---|
| | | E. coli | Staphylococcus aureus | Bacillus cereus (Nutritional Cell) |
| 60 times | 500ppm | ○ | × | × |
| 30 times | 1000ppm | ○ | ○ | ○ |
| 29 times | 1034ppm | ○ | ○ | ○ |
| 27 times | 1111ppm | ○ | ○ | ○ |
| 26 times | 1154ppm | ○ | ○ | ○ |
| 25 times | 1200ppm | ○ | ○ | ○ |
| 24 times | 1250ppm | ○ | ○ | ○ |
| 23 times | 1304ppm | ○ | ○ | ○ |
| 22 times | 1364ppm | ○ | ○ | ○ |
| 21 times | 1429ppm | ○ | ○ | ○ |
| 20 times | 1500ppm | ○ | ○ | ○ |
| 19 times | 1579ppm | ○ | ○ | ○ |
| 18 times | 1667ppm | ○ | ○ | ○ |
| 17 times | 1765ppm | ○ | ○ | ○ |
| 16 times | 1875ppm | ○ | ○ | ○ |
| 15 times | 2000ppm | ○ | ○ | ○ |

(continued)

| Dilution Factor of Impregnation | Chlorous Acid ($HClO_2$) Concentration ※ | Sterilizing Effect on Each Pathogenic Microbe | | |
|---|---|---|---|---|
| | | E. coli | Staphylococcus aureus | Bacillus cereus (Nutritional Cell) |
| 14 times | 2143ppm | ○ | ○ | ○ |
| 13 times | 2308ppm | ○ | ○ | ○ |
| 12 times | 2500ppm | ○ | ○ | ○ |
| 11 times | 2727ppm | ○ | ○ | ○ |
| 10 times | 3000ppm | ○ | ○ | ○ |
| 9 times | 3333ppm | ○ | ○ | ○ |
| 8 times | 3750ppm | ○ | ○ | ○ |
| 7 times | 4286ppm | ○ | ○ | ○ |
| 6 times | 5000ppm | ○ | ○ | ○ |
| 5 times | 6000ppm | ○ | ○ | ○ |
| 4 times | 7500ppm | ○ | ○ | ○ |
| ○ There is a sterilizing effect capable of complete sterilization<br>× No sterilization effect capable of complete sterilization | | | | |

(Example 8: Tests for Evaluating Sterilization Effects and Rate of Impregnation When Impregnated in Treatment Sheet)

In the present Example, each experiment was conducted to examine the sterilizing power of an impregnation solution when an AUTOLOC super diluent was impregnated in a treatment sheet.

(Materials)

1) Treatment sheet (material: 100 % cotton) : (about 27 × 40 cm)
2) Reagents that were used
Chlorous acid aqueous solution formulation (see Example 1: Table 11)", 0.1 M sodium thiosulfate
3) Instrument that was used
Electronic balance, triangular flask with a stopper, beaker, pipette, stirrer, stirrer bar, test tube, vortex mixer
4) Tested microbe species
E. coli (Escherichia coli IF03972)
Staphylococcus aureus (Staphylococcus aureus IF012732)

(Method)

1). Preparation Method of Concentrated Suspension of Tested Microbes

After each tested microbe was smeared on a common agar medium (Eiken Chemical Co., Ltd.) and cultured for 24 hours at 37°C, a colony that grew on the medium was extracted with a platinum loop to form a concentrated suspension with sterile saline. The solution was centrifuged and the supernatant was removed. The microbial cells were again homogeneously suspended in saline to produce a concentrated suspension of testedmicrobes (× $10^7$/ml). The microbial solution was prepared in accordance with turbidity so that the number of microbes would be at a certain amount.

2). Preparation Method of Sample Solution for Testing

When solutions of a chlorous acid aqueous solution formulation (see Example 1: Table 11) (chlorous acid concentration: 43200ppm) diluted at each dilution factor (4-fold, 6-fold, 12-fold, 30-fold, 40-fold, 50-fold, 100-fold, 150-fold, 200-fold, 300-fold, 400-fold, 600-fold, and 800-fold) were impregnated in a treatment sheet, the diluents were

impregnated at a ratio at which the amount of impregnated solution has an impregnation ratio of 300 % to 2000 % with respect to 1 kg of treatment sheets. Wet sheets were then wrung to collect a solution as a specimen. The specimen solution was further diluted for use as a sample solution for testing.

3). Method of Contacting Test Microbes and Method of Evaluating Microbes

1.0 ml of concentrated suspension of each tested microbes (× $10^6$/ml) was added to 9.0 ml of each sample solution for testing. The mixture solution was homogeneously mixed, stored in a 25°C water bath, and homogeneously mixed again every 1, 5, and 10 minutes to collect 9.0 ml of each solution. After the collected solution was added to 1.0 ml of sterilized 0.1 mol/L sodium thiosulfate solution (prepared with various buffer) and mixed homogeneously, 1.0 ml of the solution was apportioned to each of two petri dishes after the solution was further left standing for 10 minutes. The number of surviving microbes was then measured according to a common method, by pour-plate culture. The medium'used at this time was a desoxycholate medium (Eiken Chemical Co., Ltd.) for E. coli and mannitol salt agar medium with egg yolk (Eiken Chemical Co., Ltd.) for Staphylococcus aureus. After culturing for 24 hours at 37°C, the numbers of typical colonies growing in the two plates were averaged and recorded as the number of surviving microbes.

The above method was carried out. In addition, dilution factors at which the number of surviving microbes can be verified with 5 minutes of contact time but not with 10 minutes of contact time were judged as having an effect (detectable limit 100 microbes/ mL).

(Results)

A table describing the effect of a diluent of the chlorous acid aqueous solution formulation "AUTOLOC super" impregnated into a treatment sheet on "E. Coli" is shown below.

Table for Examining Effects of Diluent of Chlorous Acid Aqueous Solution Formulation "AUTOLOC super" Impregnated into Treatment Sheet on "E. Coli"

[Table 48]

| Dilution Factor (-fold) | Diluent | ←------ Impregnation Rate(%) ------→ | | | | | | | | | | | | | | | | | |
|---|---|---|---|---|---|---|---|---|---|---|---|---|---|---|---|---|---|---|---|
| | | 2000 | 1900 | 1800 | 1700 | 1600 | 1500 | 1400 | 1300 | 1200 | 1100 | 1000 | 900 | 800 | 700 | 600 | 500 | 400 | 300 |
| 4 | - | - | - | - | - | - | - | - | - | - | - | - | - | - | - | - | - | - | - |
| 6 | - | - | - | - | - | - | - | - | - | - | - | - | - | - | - | - | - | - | - |
| 12 | - | - | - | - | - | - | - | - | - | - | - | - | - | - | - | - | - | - | - |
| 30 | - | - | - | - | - | - | - | - | - | - | - | - | - | - | - | - | - | - | - |
| 40 | - | - | - | - | - | - | - | - | - | - | - | - | - | - | - | - | - | - | - |
| 50 | - | - | - | - | - | - | - | - | - | - | - | - | - | - | - | - | - | - | - |
| 59 | - | - | - | - | - | - | - | - | - | - | - | - | - | - | - | - | - | - | - |
| 73 | - | - | - | - | - | - | - | - | - | - | - | - | - | - | - | - | - | - | + |
| 86 | - | - | - | - | - | - | - | - | - | - | - | - | - | - | - | - | - | + | + |
| 100 | - | - | - | - | - | - | - | - | - | - | - | - | - | - | - | - | + | + | + |
| 114 | - | - | - | - | - | - | - | - | - | - | - | - | - | - | - | + | + | + | + |
| 127 | - | - | - | - | - | - | - | - | - | - | - | - | - | - | + | + | + | + | + |
| 141 | - | - | - | - | - | - | - | - | - | - | - | - | - | + | + | + | + | + | + |
| 154 | - | - | - | - | - | - | - | - | - | - | - | - | + | + | + | + | + | + | + |
| 168 | - | - | - | - | - | - | - | - | - | - | - | + | + | + | + | + | + | + | + |
| 181 | - | - | - | - | - | - | - | - | - | - | + | + | + | + | + | + | + | + | + |
| 195 | - | - | - | - | - | - | - | - | - | + | + | + | + | + | + | + | + | + | + |
| 208 | - | - | - | - | - | - | - | - | + | + | + | + | + | + | + | + | + | + | + |
| 222 | - | - | - | - | - | - | - | + | + | + | + | + | + | + | + | + | + | + | + |
| 235 | - | - | - | - | - | - | + | + | + | + | + | + | + | + | + | + | + | + | + |
| 249 | - | - | - | - | - | + | + | + | + | + | + | + | + | + | + | + | + | + | + |
| 263 | - | - | - | - | + | + | + | + | + | + | + | + | + | + | + | + | + | + | + |
| 276 | - | - | - | + | + | + | + | + | + | + | + | + | + | + | + | + | + | + | + |
| 290 | - | - | + | + | + | + | + | + | + | + | + | + | + | + | + | + | + | + | + |
| 300 | - | + | + | + | + | + | + | + | + | + | + | + | + | + | + | + | + | + | + |
| 400 | + | + | + | + | + | + | + | + | + | + | + | + | + | + | + | + | + | + | + |
| 600 | + | + | + | + | + | + | + | + | + | + | + | + | + | + | + | + | + | + | + |
| 800 | + | + | + | + | + | + | + | + | + | + | + | + | + | + | + | + | + | + | + |

+: Could not be completely sterilized

-: Completely sterilized

Next, a table describing the effect of a diluent of the chlorous acid aqueous solution formulation "AUTOLOC super" impregnated into a treatment sheet on "Staphylococcus aureus" is shown below.

Table for Examining Effects of Diluent of Chlorous Acid Aqueous Solution Formulation "AUTOLOC super" impregnated into Treatment Sheet on "Staphylococcus aureus"

[Table 49]

| Dilution Factor (-fold) | Diluent | ←---------- Impregnation Rate (%) ---------→ | | | | | | | | | | | | | | | | | |
|---|---|---|---|---|---|---|---|---|---|---|---|---|---|---|---|---|---|---|---|
| | | 2000 | 1900 | 1800 | 1700 | 1600 | 1500 | 1400 | 1300 | 1200 | 1100 | 1000 | 900 | 800 | 700 | 600 | 500 | 400 | 300 |
| 4 | - | - | - | - | - | - | - | - | - | - | - | - | - | - | - | - | - | - | - |
| 6 | - | - | - | - | - | - | - | - | - | - | - | - | - | - | - | - | - | - | - |
| 12 | - | - | - | - | - | - | - | - | - | - | - | - | - | - | - | - | - | - | - |
| 24 | - | - | - | - | - | - | - | - | - | - | - | - | - | - | - | - | - | - | - |
| 29 | - | - | - | - | - | - | - | - | - | - | - | - | - | - | - | - | - | - | + |
| 35 | - | - | - | - | - | - | - | - | - | - | - | - | - | - | - | - | - | + | + |
| 40 | - | - | - | - | - | - | - | - | - | - | - | - | - | - | - | - | + | + | + |
| 45 | - | - | - | - | - | - | - | - | - | - | - | - | - | - | - | + | + | + | + |
| 51 | - | - | - | - | - | - | - | - | - | - | - | - | - | - | + | + | + | + | + |
| 56 | - | - | - | - | - | - | - | - | - | - | - | - | - | + | + | + | + | + | + |
| 62 | - | - | - | - | - | - | - | - | - | - | - | - | + | + | + | + | + | + | + |
| 67 | - | - | - | - | - | - | - | - | - | - | - | + | + | + | + | + | + | + | + |
| 73 | - | - | - | - | - | - | - | - | - | - | + | + | + | + | + | + | + | + | + |
| 78 | - | - | - | - | - | - | - | - | - | + | + | + | + | + | + | + | + | + | + |
| 83 | - | - | - | - | - | - | - | - | + | + | + | + | + | + | + | + | + | + | + |
| 89 | - | - | - | - | - | - | - | + | + | + | + | + | + | + | + | + | + | + | + |
| 94 | - | - | - | - | - | - | + | + | + | + | + | + | + | + | + | + | + | + | + |
| 100 | - | - | - | - | - | + | + | + | + | + | + | + | + | + | + | + | + | + | + |
| 105 | - | - | - | - | + | + | + | + | + | + | + | + | + | + | + | + | + | + | + |
| 110 | - | - | - | + | + | + | + | + | + | + | + | + | + | + | + | + | + | + | + |
| 116 | - | - | + | + | + | + | + | + | + | + | + | + | + | + | + | + | + | + | + |
| 150 | + | + | + | + | + | + | + | + | + | + | + | + | + | + | + | + | + | + | + |
| 200 | + | + | + | + | + | + | + | + | + | + | + | + | + | + | + | + | + | + | + |
| 300 | + | + | + | + | + | + | + | + | + | + | + | + | + | + | + | + | + | + | + |
| 400 | + | + | + | + | + | + | + | + | + | + | + | + | + | + | + | + | + | + | + |
| 600 | + | + | + | + | + | + | + | + | + | + | + | + | + | + | + | + | + | + | + |
| 800 | + | + | + | + | + | + | + | + | + | + | + | + | + | + | + | + | + | + | + |

+: Could not be completely sterilized

-: Completely sterilized

It can be seen from the above results that impregnation rates and dilution factors have an almost proportional relationship. In addition, it was demonstrated that a certain amount of sterilizing component is lost due to a sheet (nonwoven fabric), but sterilizing power is stably retained thereafter. It is believed that the reason there is no difference between 2000% impregnation and a diluent and results became equivalent because the maximum impregnation rate is 2000% and there is an amount of sterilizing component to the extent where there is no effect on the amount of lost sterilizing component. Further, it was demonstrated that use in various applications is made possible with a suitable concentration and impregnation rate.

(Example 9: Tests for Examining Sterilizing Effects on Infectious PathogenicMicrobes in the Presence of OrganicMatter, Assuming Use in Vomit Treatment)

In the present Example, each experiment was conducted for examining sterilizing power of an AUTOLOC super diluent in the presence of an organic matter (protein).

<Testing Method>

(Material)

1) Reagents that were used: Chlorous acid aqueous solution formulation (see Example 1: Table 11), 0.1 M sodium thiosulfate
2) Protein that was used
Polypeptone (Becton Dickinson) diluent
3) Instrument that was used
Electronic balance, triangular flask with a stopper, beaker, pipette, stirrer, stirrer bar, test tube, and vortex mixer (3) Tested Microbial species
Enterohemorrhagic Escherichia coli 0157 (Escherichia coli 0157 sakai strain)

(Method)

1). Preparation Method of Concentrated Suspension of Tested Microbes

A colony on a MacConkey medium was extracted with a platinum loop. An LB medium was used to culture for 24 hours at 37°C. The colony was centrifuged and washed twice with sterilized saline. The obtained microbial solution was considered to be a concentrated suspension of tested microbes (x $10^7$/ml). The microbial solution was prepared in accordance with the number of microbes that would be at a certain amount.

2). Preparation Method of Sample Solution for Testing

Solutions of chlorous acid aqueous solution formulation (see Example 1: Table 11) diluted at each dilution factor (5-fold, 10-fold, 20-fold, 30-fold, 50-fold, and 100-fold) were used as specimens. Each diluent was prepared so that the final dilution factor was 10-fold, 20-fold, 40-fold, 60-fold, 100-fold, and 200-fold.

3) . Method of Contacting Test Microbes and Method of Evaluating Microbes

1.0 ml of polypeptone solution at each concentration (10 %, 1 %, or 0.1 % as peptone concentration) and 1. 0 ml of concentrated suspension of tested microbes ($\times 10^7$/ml) were added to 3.0 ml of sterilized ion exchange water and homogeneously mixed. 5.0 ml of each sample solution for testing was further added and homogeneously mixed. The mixture was stored in a 25°C water bath.
The concentrations of proteins were adjusted so that the final concentrations were 1%, 0.1% or 0.01 % peptone.
The solution was homogeneously mixed again every 1, 5, and 10 minutes to collect 1.0 ml of each solution. After the collected solution was added to 9.0 ml of sterilized 0.1 mol/L sodium thiosulfate solution (prepared with various buffer) and mixed homogeneously, 1.0 ml of the mixture was apportioned to a petri dish after the mixture was further left standing for 10 minutes. The number of surviving microbes was then measured according to a common method, by pour-plate culture. The medium used at this time was a MacConkey medium (Nissui Chemical Industries Co., Ltd.). After culturing for 24 hours at 37°C, the numbers of typical colonies growing on the plate were averaged and recorded as the number of surviving microbes.

The above method was carried out. Dilution factors at which the number of surviving microbes can be verified with 5 minutes of contact time but not with 10 minutes of contact time were judged as having an effect (detectable limit 100 microbes/ mL).

(Results)

Effects of a diluent of the chlorous acid aqueous solution formulation "AUTOLOC super" on "Enterohemorrhagic Escherichia coli O157" is shown below.

Table for Examining Effects of Diluent of Chlorous Acid Aqueous Solution Formulation "AUTOLOC super" on "Enterohemorrhagic Escherichia coli 0157"
Unit: log CFU/mL

[Table 50]

| Polypeptone(%) | Dilution Factor (- Fold) | Time of Contact (min) | | | |
|---|---|---|---|---|---|
| | | 0 min※ 1 | 1 min | 5 min | 10 min |
| 1.00 | 10-fold | 7.0 | 5.6 | 5.3 | 5.3 |
| | 20-fold | 7.0 | 5.7 | 5.6 | 5.4 |
| | 40-fold | 7.0 | 5.7 | 5.5 | 5.6 |
| | 60-fold | 7.0 | 5.9 | 5.8 | 5.8 |
| | 100-fold | 7.0 | 5.7 | 5.5 | 5.4 |
| | 200-fold | 7.0 | 5.9 | 5.6 | 5.7 |
| | Sterilized Water (control) | 6.9 | 6.0 | 6.0 | 5.9 |
| 0.10 | 10-fold | 7.0 | -※ 2 | -※ 2 | -※ 2 |
| | 20-fold | 7.0 | 5.0 | 4.5 | 3.8 |
| | 40-fold | 7.0 | 5.0 | 4.9 | 4.8 |
| | 60-fold | 7.0 | 5.8 | 5.6 | 5.5 |
| | 100-fold | 7.0 | 5.3 | 5.4 | 5.3 |
| | 200-fold | 7.0 | 5.9 | 5.9 | 5.7 |
| | Sterilized Water (control) | 6.9 | 5.9 | 6.0 | 6.0 |
| 0.01 | 10-fold | 7.0 | -※ 2 | -※ 2 | -※ 2 |
| | 20-fold | 7.0 | -※ 2 | -※ 2 | -※ 2 |
| | 40-fold | 7.0 | -※ 2 | -※ 2 | -※ 2 |
| | 60-fold | 7.0 | -※ 2 | -※ 2 | -※ 2 |
| | 100-fold | 7.0 | 4.5 | -※ 2 | -※ 2 |
| | 200-fold | 7.0 | 5.3 | 4.9 | 4.6 |
| | Sterilized Water (control) | 6.9 | 6.0 | 6.0 | 6.0 |
| *1 0 min: Starting number of microbes upon contact with an agent solution is shown.<br>*2 -: Completely sterilized | | | | | |

The above results demonstrated that sterilization is possible in an organic matter at any concentration. In addition, it can be seen that about 90-99% sterilization was possible even in an organic matter with a high concentration of 1 % (sterilized from $10^7$ CFU/ml to $10^5$CFU/ml) . Further, from the result of 20-fold dilution of 0.1%, it can be seen that, although slowly along with passage of time, reduction in the number of microbes was possible. It was demonstrated from the above that sterilizing components can be stably retained even in the presence of an organic matter and the number of microbes can be reduced by extending the time of sterilization treatment.

(Example 10: Tests for Examining Effects of Removing and Washing Contaminating Microbes Adhering to Hands and Fingers)

Tests were conducted in the present Example to examine the effects of removing and washing contaminating microbes adhering to the hands and fingers. That is, each experiment was conducted to examine whether contaminating microbes adhering to the hands and fingers could be removed by wiping with a wet wipe impregnated with an AUTOLOC super diluent when vomit accidentally adhered to a hand of an operator when handling vomit.

(Materials)

1) Chlorous acid aqueous solution (see Example 1: Table 11) Treatment sheet (material: 100% cotton): (about 27 x 40 cm) *Impregnated at a ratio of weight of treatment sheet (g) : amount of liquid (ml) = 1:3
2) "Test Microbes that were used"
E. coli: Escherichia coli IF03927
Staphylococcus aureus: Staphylococcus aureus IFO 12732 Thermoduric Microbes (Bacillus cereus): Bacillus cereus NBRC15305

(Methods)

Testing Procedure:

1) Both hands were sprayed with a hand-held sprayer. The hands were rubbed together so that the spray adhered to the hands and fingers.
2) A treatment sheet impregnated with a solution was used to wipe off both hands and fingers of 1).
3) Both hands were carefully wiped off with a cotton swab, which was suspended in saline. After dilution, the sample was spread on a petri dish and cultured by using a selected medium. The number of each of the microbes was measured.

Results of Tests for Examining Effects of Removing and Washing E. coli (E. coli IF03927)

[Table 51]

| Agent Solution | Chlorous Acid ($HClO_2$) Concentration | Number of Times Wiped | | | | | |
|---|---|---|---|---|---|---|---|
| | | 0 Time | 1 Time | 2 Times | 3 Times | 4 Times | 5 Times |
| Tap Water | 0.4 ppm* | $4.1\times10^7$ | $2.3\times10^4$ | $9.0\times10^2$ | $7.6\times10^2$ | $7.0\times10$ | 4.0x10 |
| Alcohol (75%) | 0 ppm | $4.1x10^7$ | $2.7\times10^4$ | $6.7\times10^2$ | <10 | <10 | <10 |
| Chlorous Acid Aqueous Solution Formulation | 30,000 ppm | $4.1\times10^7$ | <10 | <10 | <10 | <10 | <10 |
| | 10,000 ppm | $4.1\times10^7$ | <10 | <10 | <10 | <10 | <10 |
| | 5,000 ppm | $4.1\times10^7$ | <10 | <10 | <10 | <10 | <10 |
| | 3,000 ppm | $4.1\times10^7$ | <10 | <10 | <10 | <10 | <10 |
| | 1,000 ppm | $4.1\times10^7$ | <10 | <10 | <10 | <10 | <10 |
| *Measured Value at Available Chlorine Concentration | | | | | | | |

Results of Tests for Examining Effects of Removing and Washing Staphylococcus aureus (St. aureus)

[Table 52]

| Agent Solution | Chlorous Acid ($HClO_2$) Concentration | Number of Times Wiped | | | | | |
|---|---|---|---|---|---|---|---|
| | | 0 Time | 1 Time | 2 Times | 3 Times | 4 Times | 5 Times |
| Tap Water | 0.4 ppm※ | $1.1\times10^7$ | $4.4\times10^3$ | $1.5\times10^2$ | $1.3\times10^2$ | <10 | <10 |
| Alcohol (75%) | 0 ppm | $1.1\times10^7$ | $1.5\times10^4$ | $8.1\times10^3$ | $2.6\times10^3$ | $8.6\times10^2$ | <10 |
| Chlorous Acid Aqueous So ution Formulation | 30,000 ppm | $1,1\times10^7$ | <10 | <10 | <10 | <10 | <10 |
| | 10,000 ppm | $1.1\times10^7$ | <10 | <10 | <10 | <10 | <10 |
| | 5,000 ppm | $1.1\times10^7$ | <10 | <10 | <10 | <10 | <10 |
| | 3,000 ppm | $1.1\times10^7$ | <10 | <10 | <10 | <10 | <10 |
| | 1,000 ppm | $1.1\times10^7$ | $2,5x10^2$ | $1.2\times10^2$ | <10 | <10 | <10 |
| *Measured Value at Available Chlorine Concentration | | | | | | | |

Results of Tests for Examinig Effects of Removing and Washing Thermoduric Microbes (Bacillus cereus)

[Table 53]

| Agent Solution | Chlorous Acid ($HClO_2$) Concentration | Number of Times Wiped | | | | | |
|---|---|---|---|---|---|---|---|
| | | 0 Time | 1 Time | 2 Times | 3 Times | 4 Times | 5 Times |
| Tap Water | 0.4 ppm | $9.2\times10^6$ | $2.9\times10^3$ | $7.3\times10^2$ | $2.9x10^2$ | 8.0x10 | <10 |
| Alcohol (75%) | 0 ppm | $9.2\times10^6$ | $2.1\times10^4$ | $4.8\times10^3$ | $2.0\times10^3$ | <10 | <10 |
| Chlorous Acid Aqueous Solution Formulation | 30,000 ppm | $9.2\times10^6$ | <10 | <10 | <10 | <10 | <10 |
| | 10,000 ppm | $9.2\times10^6$ | <10 | <10 | <10 | <10 | <10 |
| | 5,000 ppm | $9.2\times10^6$ | <10 | <10 | <10 | <10 | <10 |
| | 3,000 ppm | $9.2\times10^6$ | <10 | <10 | <10 | <10 | <10 |
| | 1,000 ppm | $9.2\times10^6$ | $1.1x10^2$ | $4.8\times10$ | <10 | <10 | <10 |

*Measured Value at Available Chlorine Concentration

(Example 11: Deodorizing Effect of Chlorous Acid Aqueous Solution)

In order to evaluate tests on deodorizing effects, the present Example examined deodorizing effects by using standards regarding air freshening and deodorization ([II]-2 Method of Testing Deodorizing Efficacy (Chemical Deodorization)) set forth by the Air Fresheners and Deodorizers Conference (http://www.houkou.gr.jp/) as the efficacy testing method, which is adopted by Japan Food Research Laboratories.

The details thereof are explained below.

(Agents Used)

Chlorous acid aqueous solution

Sodium hypochlorite: Reagent, Wako Pure Chemical Industries, chemical standard solution

(Target Odorous Substances)

Ammonium odor: Ammonium Standard Solution II (Wako Pure Chemical Industries, for testing malodorous substances)
Amine odor: methyl mercaptan standard solution (Wako Pure Chemical Industries, for testing malodorous substances)
Sulfur odor: hydrogen sulfide (self-made, used hydrogen sulfide that was generated by adding sulfuric acid to zinc sulfide)

(Detector)

Gas detector tube made by GASTEC Corporation

Ammonium odor: Detector tube ammonium 3L made by GASTEC Corporation
Amino odor: Detector tube methyl mercaptan 71 made by GASTEC Corporation
Sulfur odor: Detector tube hydrogen sulfide 4LL made by GASTEC Corporation

(Testing Method)

Tests were conducted in compliance with ([II]-2 Method of Testing Deodorizing Efficacy (Chemical Deodorization)) set forth by the Air Fresheners and Deodorizers Conference.

(1) Application Method

The tests were carried out by static culture method, using a commercially-available airbag with a spigot having a volume of about 10 L as test containers. The volume of airbags was appropriately changed in accordance with the size of the product. Materials that do not absorb or desorb malodor, fragrance or the like, such as fluoroplastics (polyvinyl fluoride, FRP or the like), polyester (PET or the like), aluminum laminate (thin film of aluminum laminated to a plastic film) or the like was used as the material of the airbags and odor bags.

(2) Testing Procedure

Airbags or equivalent thereof containing a product in a state of use and malodor and airbags or equivalent thereof containing only malodor (blank: in the present Example, said product is sold in a sealed state and it is not possible to put a fan or product therein as shown in Figure 16. Thus, since it is necessary to open the bags with a pair of scissors or a utility knife and to seal again, it was determined that such an operation requires proficiency. Thus, a glass container from Hyogo Prefectural Institute of Technology (Figure 17) was used) were prepared. The bags were filled with odorless air. Malodor concentrations were measured over time at room temperature (about 20°C). (3 points or higher, detection tube method (Figure 16), measured by an instrument analysis methodor olfactorymeasurementmethod. When decrease in the blank was observed, the decrease was suitably corrected. When measuring by olfactory measuring method, the airbag spigot was opened for direct sniffing, or gas was transferred to an odor bag while wearing a mask to perform olfactory measurement in an autonomic respiration form. Measured air was not returned to the source).

(Detailed Results)

(1) Results of Examining Deodorizing Effect on Ammonium Odor (Malodorous Substance: Ammonium)

For ammonium odor, the residual concentrations were detected and recorded for control (no specimen), 20 ml (cc) of distilled water, sodium hypochlorite (expressed as Na hypochlorite, 400 ppm), and chlorous acid aqueous solution (expressed as CAAS, 400 ppm) prior to spraying and 0 hour, 3 hours, 6 hours, 9 hours, and 24 hours after spraying.
It is known that an odorous substance adsorbs to an airbag and naturally decreases when the odorous substance is in the airbag. In this regard, the amount of loss due to such a natural decrease was measured to normalize the observed values in a control segment.
For the values, the values of the raw data were recorded. The concentration prior to spraying was adjusted

and the controls were normalized to be 100. The results are shown in the following Table (In the Table, Cont indicates controls and h indicates time).

Before correction (ppm)

[Table 54]

| | | Cont | Spray 20 cc | | |
|---|---|---|---|---|---|
| | | | Distilled water | Na hypochlorite | CAAS |
| Before spraying | | 200 | 200 | 320 | 350 |
| After spraying | 0h | 200 | 30 | 30 | 30 |
| | 3h | 180 | 35 | 50 | 10 |
| | 6h | 150 | 33 | 50 | 5 |
| | 9h | 110 | 20 | 40 | 2 |
| | 24h | 90 | 20 | 40 | 0 |
| After correction (Control = 100) | | | | | |
| | | Cont | Spray 20 cc | | |
| | | | Distilled water | Na hypochlorite | CAAS |
| Before spraying | | 100 | 100 | 100 | 100 |
| After spraying | 0h | 100 | 15 | 9 | 9 |
| | 3h | 100 | 19 | 17 | 3 |
| | 6h | 100 | 22 | 21 | 2 |
| | 9h | 100 | 18 | 23 | 1 |
| | 24h | 100 | 22 | 28 | 0 |

(Results)

Results are shown in Table 54 and Figure 18.

Against ammonium odor (malodorous substance: ammonium), there was certainly a deodorizing effect. First, in the results of each agent segment (distilled water segment, sodium hypochlorite (Na hypochlorite) solution 400 ppm segment, chlorous acid aqueous solution (CAAS) 400 ppm segment) when a segment only containing a malodorous substance is 100 as a control, the values in the test segments where sodium hypochlorite or CAAS was added decreased immediately after spraying to 10 or less, which can be judged as having a deodorizing effect. However, in the Na hypochlorite segment, it was found that ammonium odor arose once time has passed. Thus, only chlorous acid aqueous solution" could be judged as having a deodorizing effect for a long period of time. When confirming with Japan Food Research Laboratories regarding the implementation of the deodorizing tests on urine of pets (dogs/cats), ammonium was set as the standard material. Thus, the present invention is recognized as having a deodorizing effect on urine of pets (dogs/cats).

Although data is not shown, when the ammonium concentration was increased about 10-fold, the deodorizing effect decreased. However, a chlorous acid aqueous solution exhibited the most deodorizing effect. Since the amount of spraying was not changed, there was more of a reduced tendency in the deodorizing effect in comparison to the above-described experiment, thus failing to reach complete deodorization. Thus, when an ammonium concentration is increased, similar increase of a chlorous acid aqueous solution is considered preferable.

(2) Results of Examining Deodorizing Effect on Amine Odor (Malodorous Substance: Methyl Mercaptan)

Next, for amine odor (methyl mercaptan), the residual concentrations were detected and recorded for control (no specimen), 20 ml (cc) of distilled water, sodium hypochlorite (expressed as Na hypochlorite, 400 ppm), and chlorous acid aqueous solution (expressed as CAAS, 400 ppm) prior to spraying and 0 hour, 3 hours, 6 hours, 9 hours, and 24

hours after spraying. The experiment was conducted twice.

Before correction (ppm)

[Table 55]

| | | Cont | Spray 20 cc | | |
|---|---|---|---|---|---|
| | | | Distilled water | Na hypochlorite | CAAS |
| Before spraying | | 80 | 80 | 90 | 110 |
| After spraying | 0h | 75 | 50 | 20 | 50 |
| | 3h | 52 | 20 | 0 | 20 |
| | 6h | 40 | 10 | 0 | 7 |
| | 9h | 30 | 8 | 5 | 2.5 |
| | 24h | 20 | 6 | 6 | 0 |
| After correction (Control = 100) | | | | | |
| | | Cont | Spray 20 cc | | |
| | | | Distilled water | Na hypochlorite | CAAS |
| Before spraying | | 100 | 100 | 100 | 100 |
| After | 0h | 100 | 63 | 22 | 45 |
| | 3h | 100 | 36 | 0 | 26 |
| | 6h | 100 | 23 | 0 | 12 |
| | 9h | 100 | 25 | 14 | 6 |
| | 24h | 100 | 28 | 25 | 0 |

Before correction (ppm)

[Table 56]

| | | Cont | Spray 20 cc | | |
|---|---|---|---|---|---|
| | | | Distilled water | Na hypochlorite | CAAS |
| Before spraying | | 80 | 70 | 70 | 70 |
| After spraying | 0h | 73 | 40 | 8 | 45 |
| | 3h | 48 | 20 | 5 | 12 |
| | 6h | 42 | 10 | 5 | 10 |
| | 9h | 31 | 10 | 7 | 5 |
| | 24h | 15 | 7 | 5 | 0 |
| After correction (Control =100) | | | | | |
| | | Cont | Spray 20 cc | | |
| | | | Distilled water | Na hypochlorite | CAAS |
| Before Spraying | | 100 | 100 | 100 | 100 |

(continued)

| | | Cont | Spray 20 cc | | |
|---|---|---|---|---|---|
| | | | Distilled water | Na hypochlorite | CAAS |
| After Spraying | 0h | 100 | 57 | 11 | 64 |
| | 3h | 100 | 43 | 11 | 26 |
| | 6h | 100 | 25 | 12 | 25 |
| | 9h | 100 | 34 | 24 | 17 |
| | 24h | 100 | 49 | 35 | 0 |

(Results)

Results are shown in Tables 55-56 and Figures 19-20. In the first experiment, in the results of each agent segment (distilled water segment, sodium hypochlorite (Na hypochlorite) solution 400 ppm segment, chlorous acid aqueous solution (CAAS) 400 ppm segment) when a segment only containing a malodorous substance is 100 as a control, the value in the Na hypochlorite segment decreased in 3 hours after spraying to 10 or less, which can be judged as having a deodorizing effect. However, a tendency for amine odor to rise was found once time has passed. Further, a tendency of a decrease as time passed was observed. As a result, only "chlorous acid aqueous solution" could be judged as having a deodorizing effect after 9 hours.

In both experiments, a complete deodorizing effect was demonstrated only by a chlorous acid aqueous solution. In any case, although sodium hypochlorite exerts a strong deodorizing effect immediately after spraying, a phenomenon of rising odor can be seen thereafter as time passes. However, although a deodorizing effect that is as rapid as the deodorizing effect for sodium hypochlorite is not observed, the deodorizing agent of the present invention gradually exerts a deodorizing effect that ultimately becomes stronger than the deodorizing effect of sodiumhypochlorite as if they switchplaces. For this reason, the deodorizing agent of the present invention is recognized as a slow-acting deodorizing agent.

(3) Results of Examining Deodorizing Effects on Sulfide Odor (Malodorous Substance: Hydrogen Sulfide)

Before correction (ppm)

[Table 57]

| | | Cont | Spray 20 cc | | |
|---|---|---|---|---|---|
| | | | Distilled water | Na hypochlorite | CAAS |
| Before spraying | | 40 | 28 | 40 | 38 |
| After spraying | 0h | 38 | 2.5 | 0 | 0 |
| | 3h | 20 | 0 | 0 | 0 |
| | 6h | 13 | 0 | 0 | 0 |
| | 9h | 10 | 0 | 0 | 0 |
| | 24h | 5 | 0 | 0 | 0 |
| After correction (Control = 100) | | | | | |
| | | Cont | Spray 20 cc | | |
| | | | Distilled water | Na hypochlorite | CAAS |
| Before spraying | | 100 | 100 | 100 | 100 |

(continued)

| | | Cont | Spray 20 cc | | |
|---|---|---|---|---|---|
| | | | Distilled water | Na hypochlorite | CAAS |
| After spraying | 0h | 100 | 9 | 0 | 0 |
| | 3h | 100 | 0 | 0 | 0 |
| | 6h | 100 | 0 | 0 | 0 |
| | 9h | 100 | 0 | 0 | 0 |
| | 24h | 100 | 0 | 0 | 0 |

Before correction (ppm)

[Table 58]

| | | Cont | Spray 20 cc | | |
|---|---|---|---|---|---|
| | | | Distilled water | Na hypochlorite | CAAS |
| Before spraying | | 43 | 50 | 50 | 50 |
| After spraying | 0h | 38 | 6 | 0 | 2.5 |
| | 3h | 19 | 2.5 | 0 | 3.4 |
| | 6h | 12 | 2.5 | 0 | 0 |
| | 9h | 10 | 0 | 0 | 0 |
| | 24h | 4 | 0 | 0 | 0 |
| After correction (Control = 100) | | | | | |
| | | Cont | Spray 20 cc | | |
| | | | Distilled water | Na hypochlorite | CAAS |
| Before spraying | | 100 | 100 | 100 | 100 |
| After spraying | 0h | 100 | 12 | 0 | 5 |
| | 3h | 100 | 10 | 0 | 14 |
| | 6h | 100 | 16 | 0 | 0 |
| | 9h | 100 | 0 | 0 | 0 |
| | 24h | 100 | 0 | 0 | 0 |

(Results)

The results are shown in Tables 57-58 and Figures 21-22.

It was found that there is a deodorizing effect on sulfur odor (malodorous substance: hydrogen sulfide). However, in any segment where an agent was added, the value decreased immediately after spraying to 10 or less, which is judged as having a deodorizing effect. No rise in odor was confirmed up to 24 hours thereafter. At the tested concentrations, hydrogen sulfide was deodorized even with water. Similar results were obtained in the second tests as the first test, having a deodorizing effect.

(Summary)

From the above, the deodorizing agent using the chlorous acid aqueous solution of the present invention was revealed to have an excellent deodorizing effect in comparison to sodium hypochlorite of conventional art. Inparticular, it is understood that many superb points are observed with respect to effects against ammonium odor and amine odor,

such as the fact that there is no rise.

As described above, the present invention is exemplified by the use of its preferred Embodiments and Examples. However, the present invention is not limited thereto. Various embodiments can be practiced within the scope of the structures recited in the claims. It is understood that the scope of the present invention should be interpreted solely based on the claims. Furthermore, it is understood that any patent, any patent application, and any references cited in the present specification should be incorporated by reference in the present specification in the same manner as the contents are specifically described therein.

[Industrial Applicability]

An aqueous solution comprising a chlorous acid aqueous solution obtained by the present invention can be utilized in applications in a sterilizing agent as well as deodorant, bleach, blood draining agent and the like.

## Claims

1. A method of preserving a chlorous acid aqueous solution for a long period of time, comprising maintaining the chlorous acid aqueous solution at 10°C or lower.

2. The method of claim 1, wherein the chlorous acid aqueous solution is an aqueous solution comprising a chlorous acid aqueous solution, metal hydroxide, and metal phosphate.

3. The method of claim 2, wherein the metal comprises potassium.

4. The method of claim 2 or 3, wherein the metal hydroxide comprises sodium hydroxide and/or potassium hydroxide and the metal phosphate comprises sodium phosphate and/or potassium phosphate.

5. The method of any one of claims 1 to 4, wherein pH is 3.2 or higher and lower than 7.0.

6. The method of any one of claims 1 to 5, wherein pH is 5.0 to 7.0.

7. The method of any one of claims 1 to 6, wherein pH is about 5.5.

8. The method of any one of claims 2 to 7, wherein the chlorous acid aqueous solution is 0.25%-75%, potassium dihydrogen phosphate is 0.70% to 13.90%, and potassium hydroxide is 0.10% to 5.60%.

9. The method of any one of claims 2 to 8, wherein sodium hydroxide and potassium hydroxide are 0.1 N to 1.0 N and buffer pH of sodium phosphate and potassium phosphate is 3.2 or higher and lower than 7.0.

10. The method of claim 1, comprising maintaining the chlorous acid aqueous solution at 4°C or lower.

[Fig. 1]

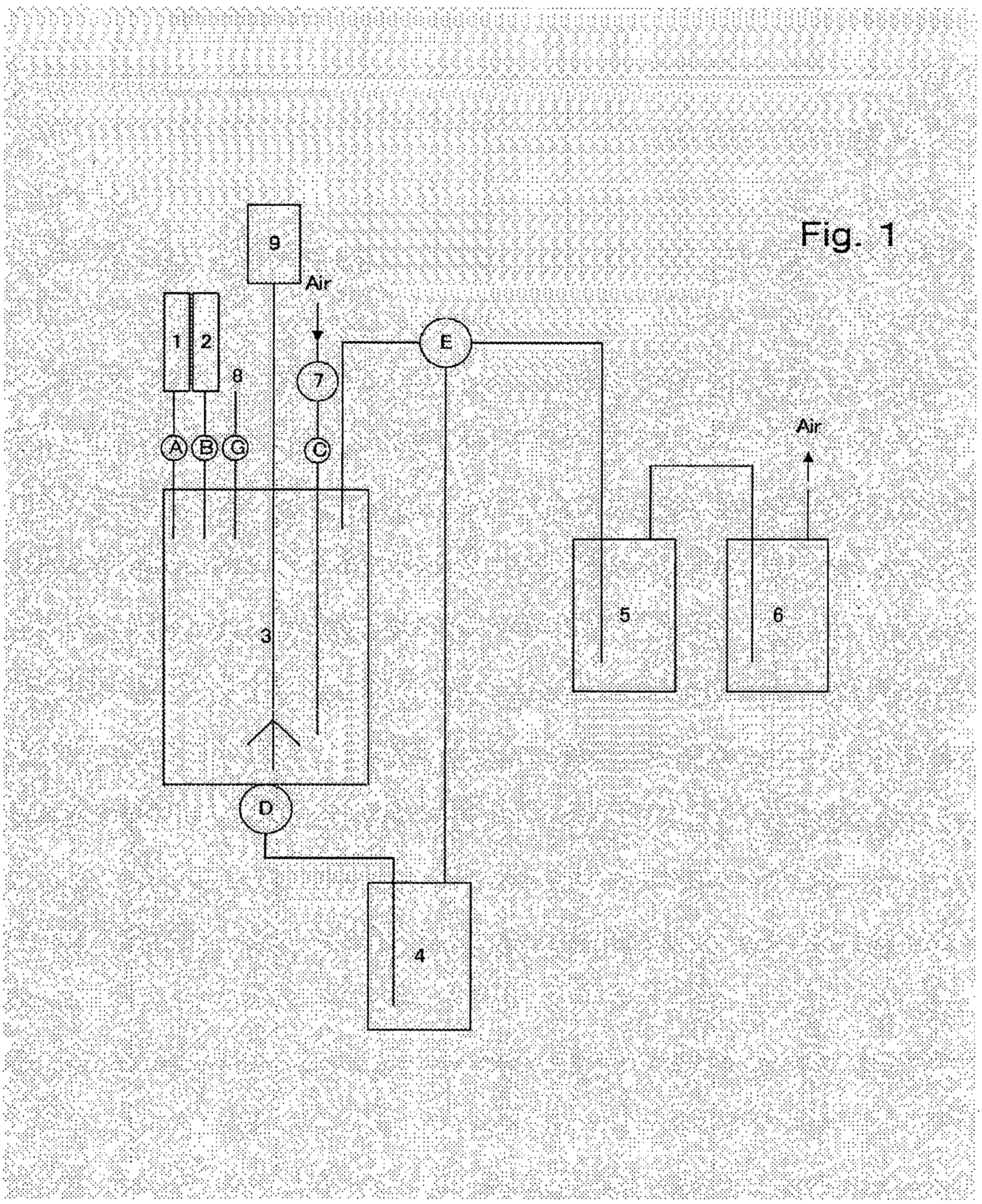

[Fig. 2]

Fig. 2

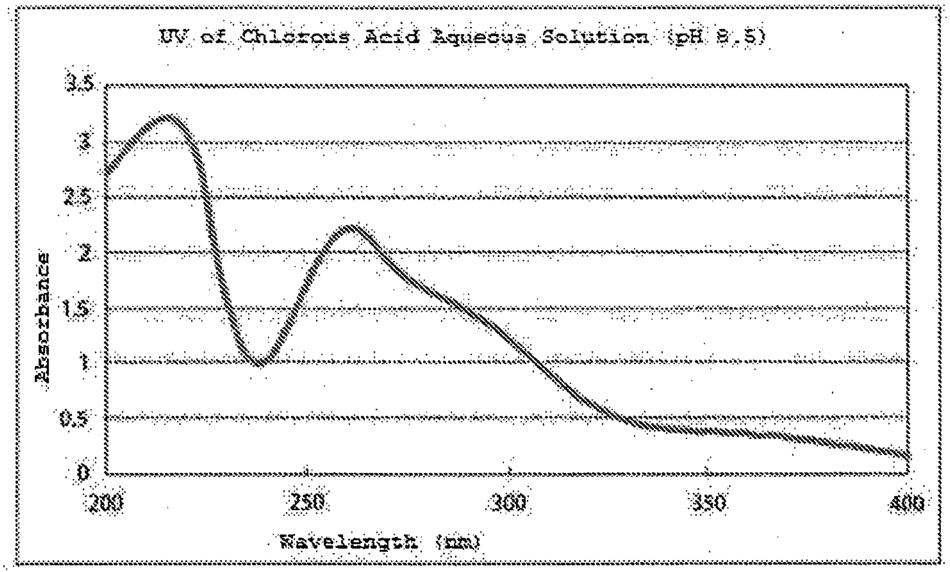


[Fig. 3]

Fig. 3

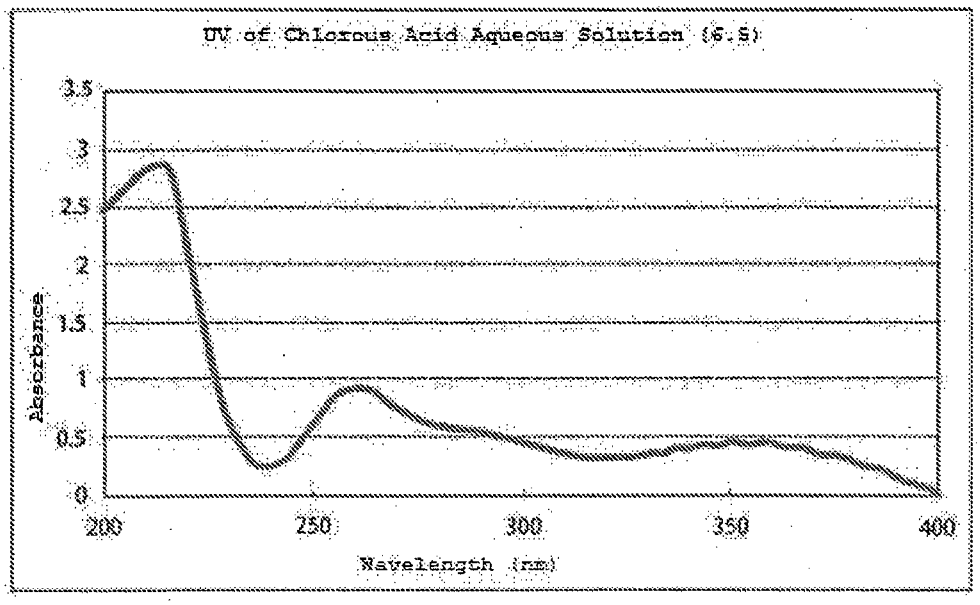

[Fig. 4]

Fig. 4

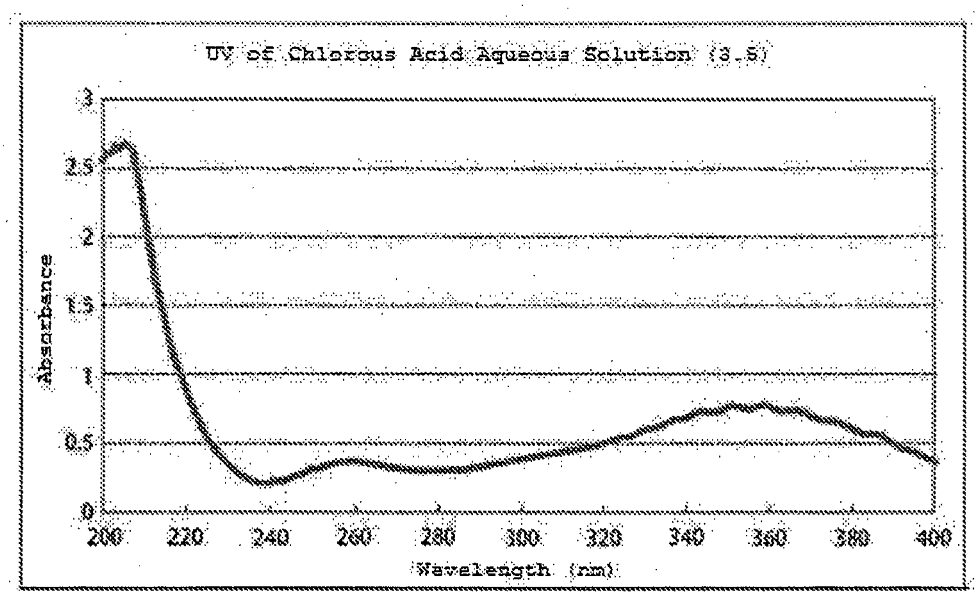


[Fig. 5]

Fig. 5

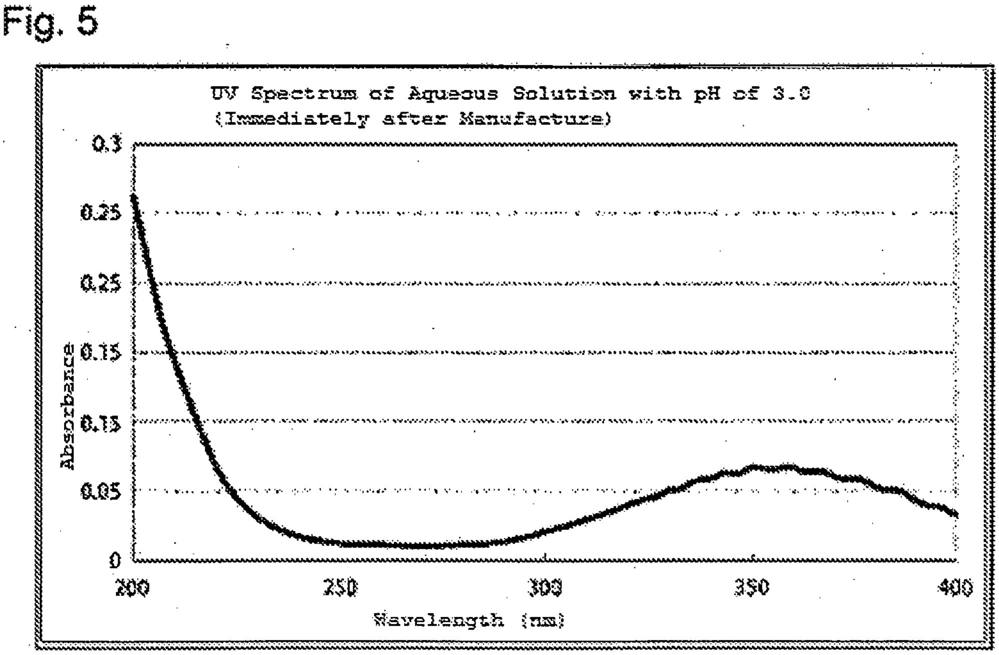

[Fig. 6]

Fig. 6

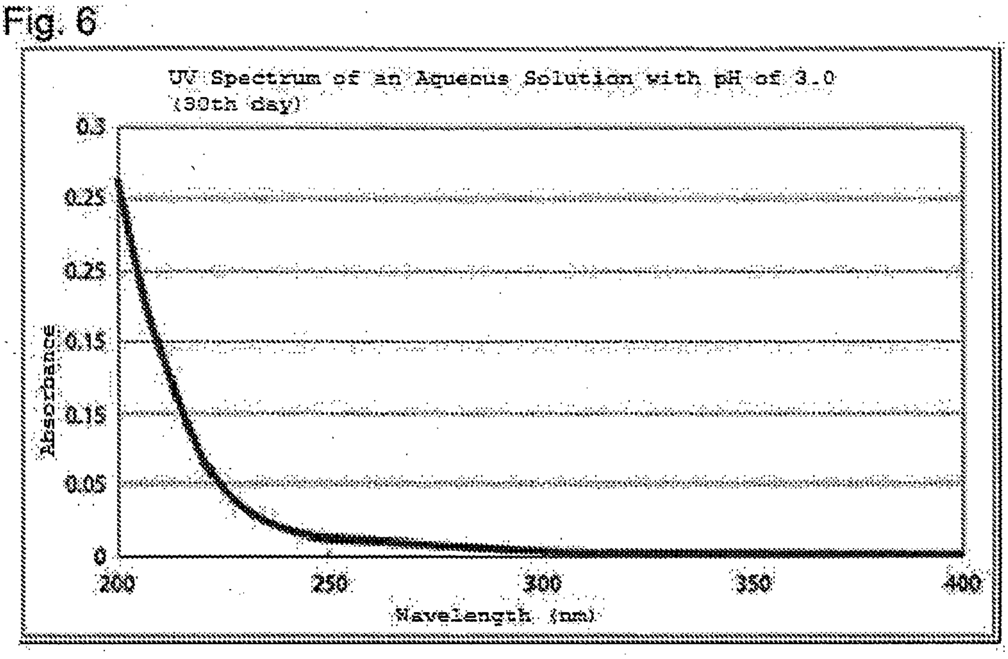


[Fig. 7]

Fig. 7

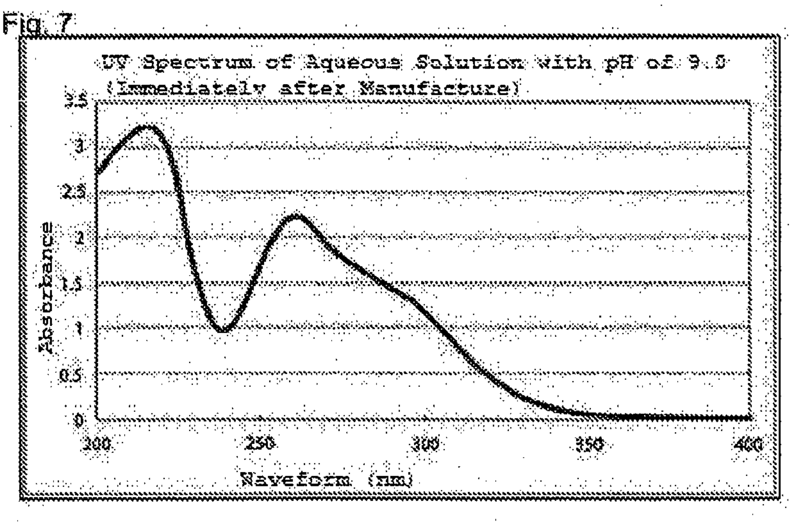

[Fig. 8]

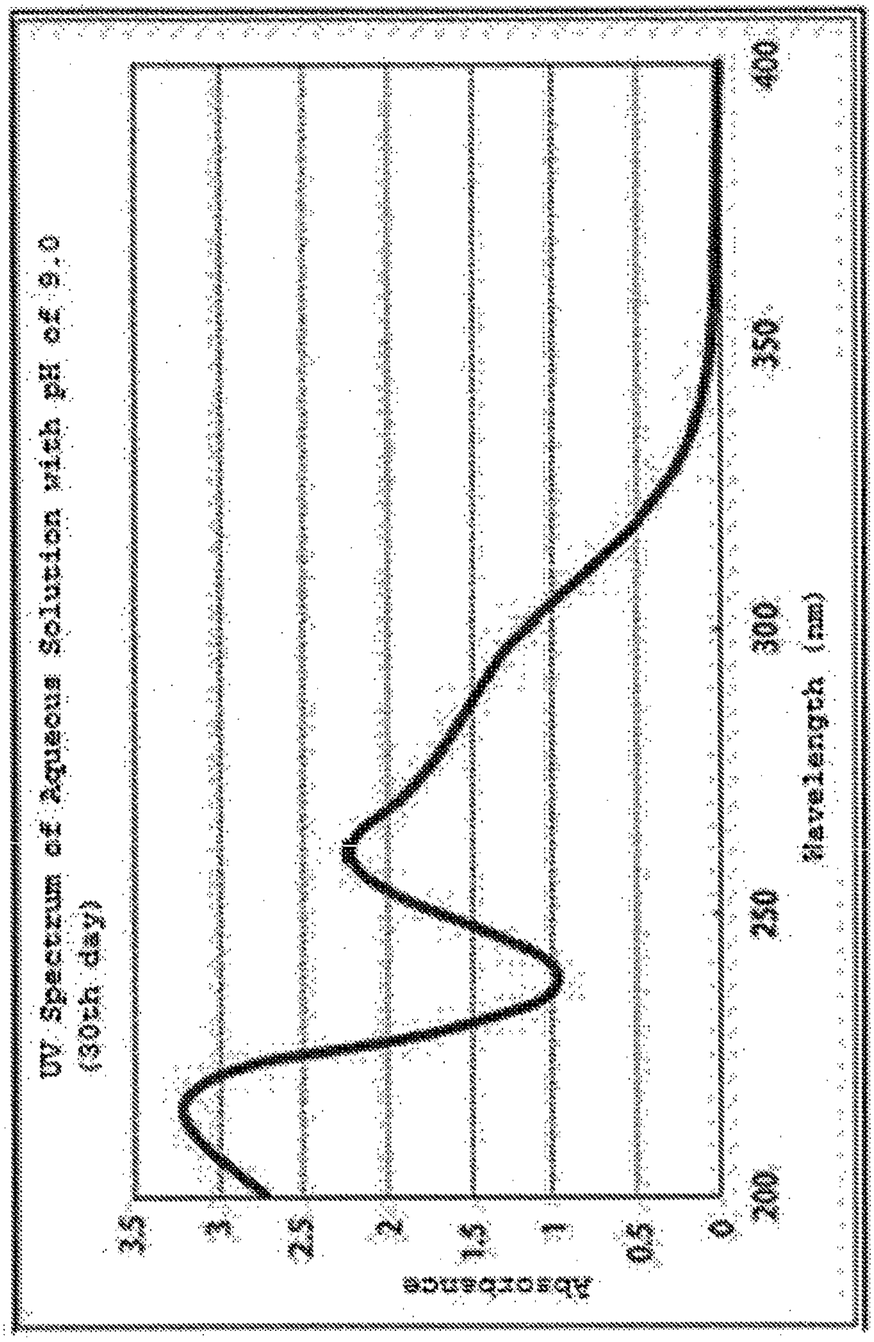
UV Spectrum of Aqueous Solution with pH of 9.0
(30th day)
Absorbance
3.5
3
2.5
2
1.5
1
0.5
0
200
250
300
350
400
Wavelength (nm)

Fig. 8

# Fig. 9

Immediately After Manufacture

Sterilizing Effect Examination Chart (pH3.5)

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | − | − | − | − |
| 0.008% | 80ppm | − | − | − | − |
| 0.007% | 70ppm | − | − | − | − |
| 0.006% | 60ppm | − | − | − | − |
| 0.005% | 50ppm | + | − | − | − |
| 0.004% | 40ppm | + | − | − | − |
| 0.003% | 30ppm | + | + | + | + |
| 0.002% | 20ppm | + | + | + | + |
| 0.001% | 10ppm | + | + | + | + |
| 0.000% | 0ppm | + | + | + | + |

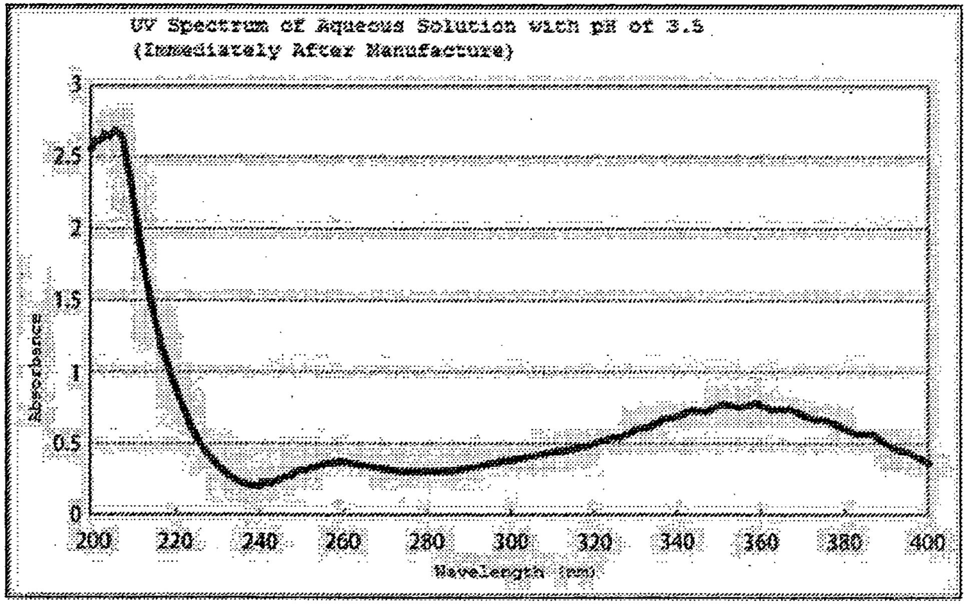

Sterilizing Effect Examination Chart (pH6.5)

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | − | − | − | − |
| 0.008% | 80ppm | − | − | − | − |
| 0.007% | 70ppm | − | − | − | − |
| 0.006% | 60ppm | − | − | − | − |
| 0.005% | 50ppm | + | − | − | − |
| 0.004% | 40ppm | + | − | − | − |
| 0.003% | 30ppm | + | + | + | − |
| 0.002% | 20ppm | + | + | + | + |
| 0.001% | 10ppm | + | + | + | + |
| 0.000% | 0ppm | + | + | + | + |

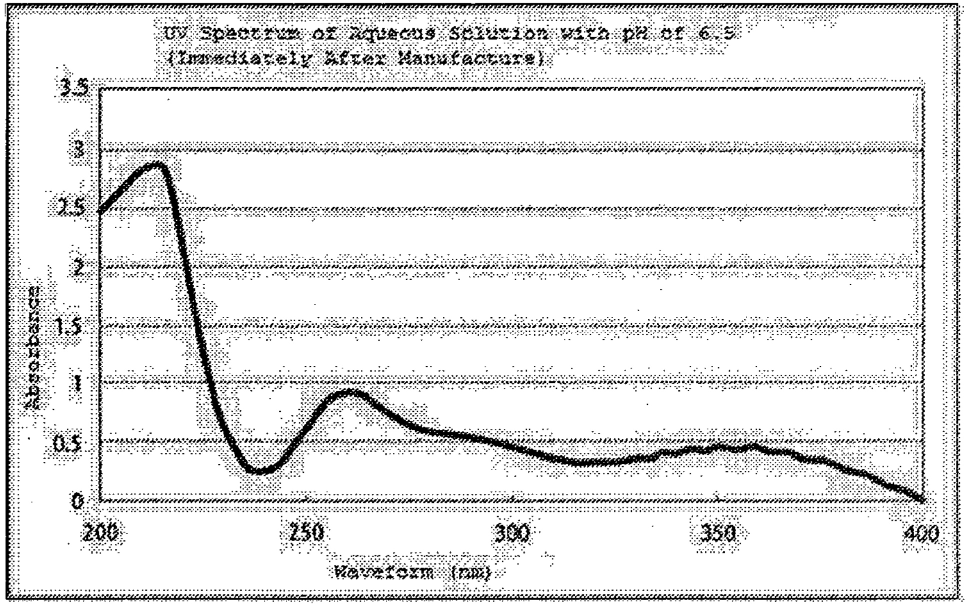

Sterilizing Effect Examination Chart (pH8.5)

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | − | − | − | − |
| 0.008% | 80ppm | + | − | − | − |
| 0.007% | 70ppm | + | + | − | − |
| 0.006% | 60ppm | + | + | + | + |
| 0.005% | 50ppm | + | + | + | + |
| 0.004% | 40ppm | + | + | + | + |
| 0.003% | 30ppm | + | + | + | + |
| 0.002% | 20ppm | + | + | + | + |
| 0.001% | 10ppm | + | + | + | + |
| 0.000% | 0ppm | + | + | + | + |

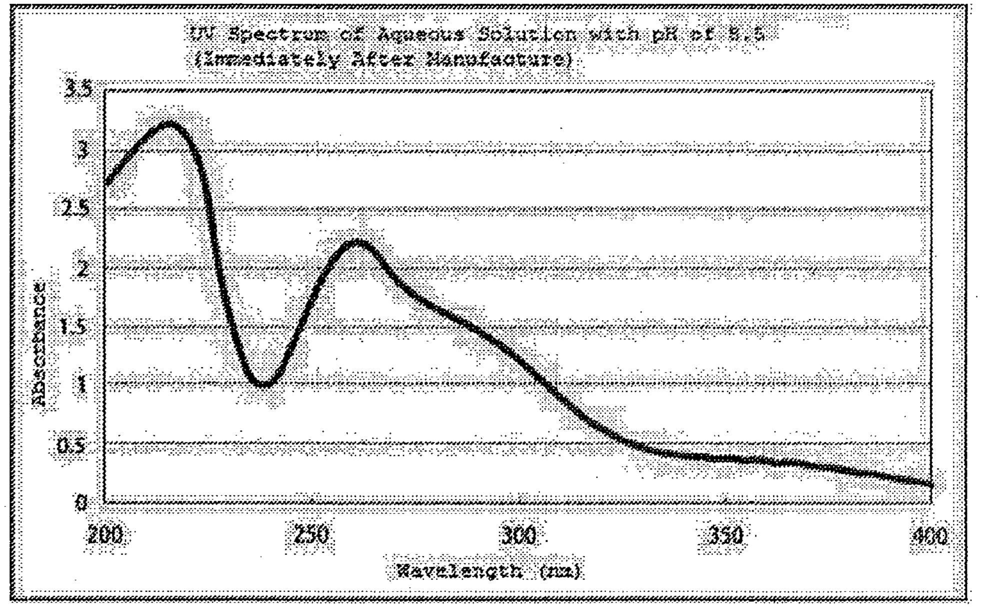

# Fig 10

10th Day

**Sterilizing Effect Examination Chart (pH3.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | + | − | − | − |
| 0.010% | 100ppm | + | + | − | − |
| 0.008% | 80ppm | + | + | + | + |
| 0.007% | 70ppm | + | + | + | + |
| 0.006% | 60ppm | + | + | + | + |
| 0.005% | 50ppm | + | + | + | + |
| 0.004% | 40ppm | + | + | + | + |
| 0.003% | 30ppm | + | + | + | + |
| 0.002% | 20ppm | + | + | + | + |
| 0.001% | 10ppm | + | + | + | + |
| 0.000% | 0ppm | + | + | + | + |

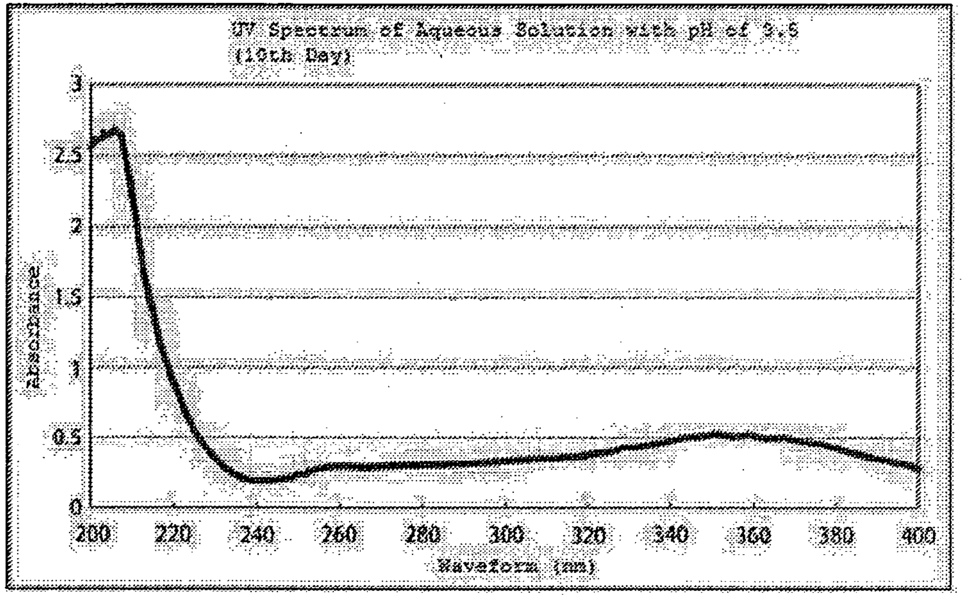

## Sterilizing Effect Examination Chart (pH6.5)

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | − | − | − | − |
| 0.008% | 80ppm | − | − | − | − |
| 0.007% | 70ppm | − | − | − | − |
| 0.006% | 60ppm | − | − | − | − |
| 0.005% | 50ppm | + | − | − | − |
| 0.004% | 40ppm | + | − | − | − |
| 0.003% | 30ppm | + | + | + | − |
| 0.002% | 20ppm | + | + | + | + |
| 0.001% | 10ppm | + | + | + | + |
| 0.000% | 0ppm | + | + | + | + |

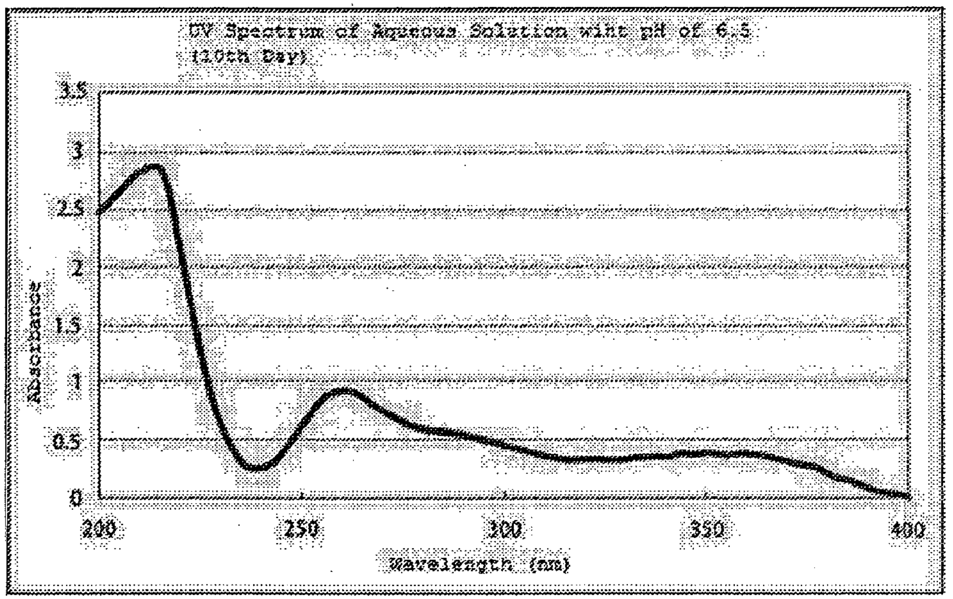

**Sterilizing Effect Examination Chart (pH8.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | + | − | − | − |
| 0.008% | 80ppm | + | + | − | − |
| 0.007% | 70ppm | + | + | + | + |
| 0.006% | 60ppm | + | + | + | + |
| 0.005% | 50ppm | + | + | + | + |
| 0.004% | 40ppm | + | + | + | + |
| 0.003% | 30ppm | + | + | + | + |
| 0.002% | 20ppm | + | + | + | + |
| 0.001% | 10ppm | + | + | + | + |
| 0.000% | 0ppm | + | + | + | + |

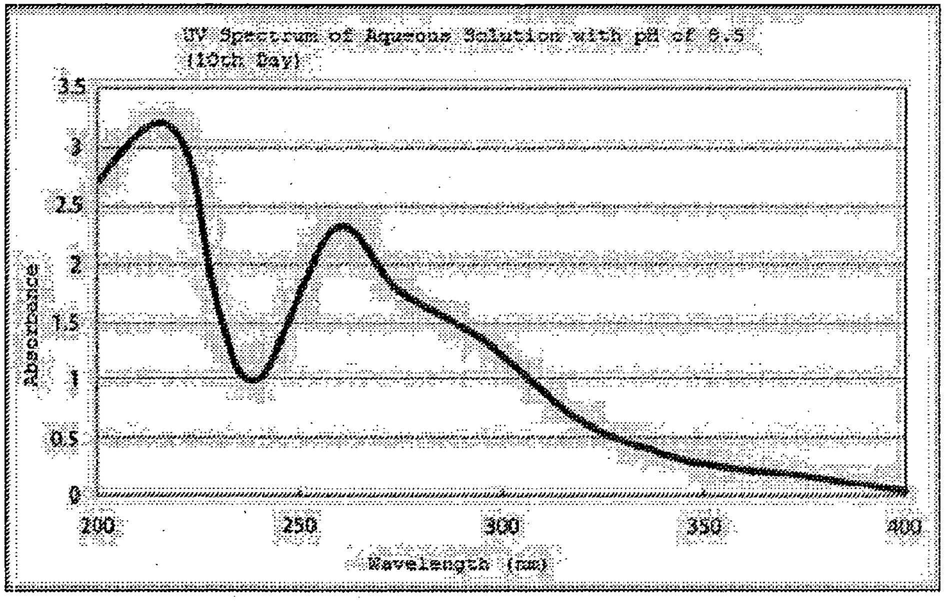

Fig. 11

30th Day

**Sterilizing Effect Examination Chart (pH3.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 500ppm | − | − | − | − |
| 0.010% | 400ppm | − | − | − | − |
| 0.008% | 350ppm | + | + | − | − |
| 0.007% | 300ppm | + | + | + | + |
| 0.006% | 200ppm | + | + | + | + |
| 0.005% | 250ppm | + | + | + | + |
| 0.004% | 200ppm | + | + | + | + |
| 0.003% | 150ppm | + | + | + | + |
| 0.002% | 100ppm | + | + | + | + |
| 0.001% | 50ppm | + | + | + | + |
| 0.000% | 0ppm | + | + | + | + |

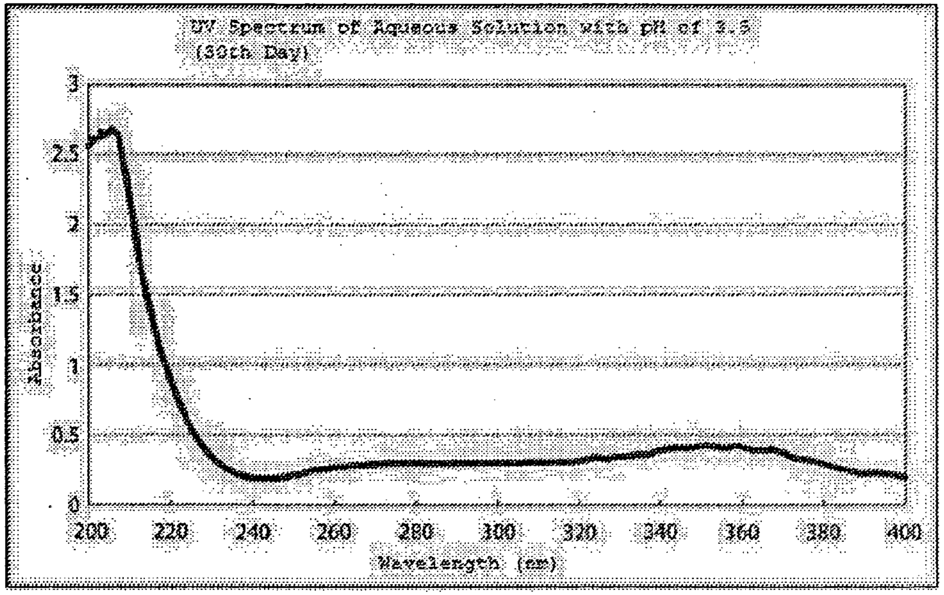

**Sterilizing Effect Examination Chart (pH6.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | − | − | − | − |
| 0.008% | 80ppm | − | − | − | − |
| 0.007% | 70ppm | − | − | − | − |
| 0.006% | 60ppm | − | − | − | − |
| 0.005% | 50ppm | − | − | − | − |
| 0.004% | 40ppm | − | − | − | − |
| 0.003% | 30ppm | + | + | + | − |
| 0.002% | 20ppm | + | + | + | + |
| 0.001% | 10ppm | + | + | + | + |
| 0.000% | 0ppm | + | + | + | + |

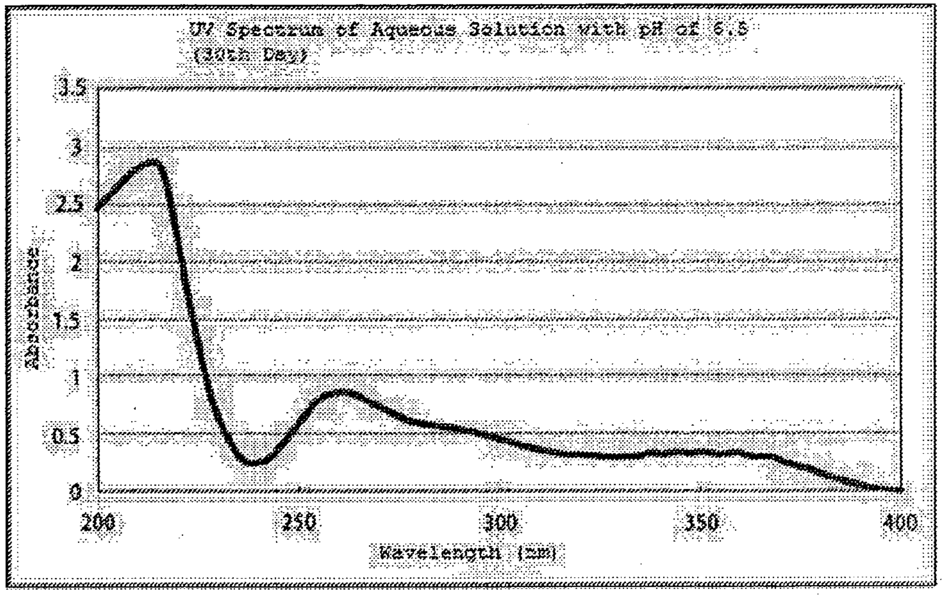

**Sterilizing Effect Examination Chart (pH8.5)**

| Phenol Concentration | | Time of Contact | | | |
|---|---|---|---|---|---|
| % | ppm | 1minute | 5minutes | 10minutes | 15minutes |
| 2.070% | 20000ppm | − | − | − | − |
| 1.500% | 15000ppm | + | − | − | − |
| 1.000% | 10000ppm | + | + | − | − |
| 0.700% | 7000ppm | + | + | + | + |
| 0.500% | 5000ppm | + | + | + | + |
| 0.300% | 3000ppm | + | + | + | + |
| 0.100% | 1000ppm | + | + | + | + |
| 0.010% | 100ppm | + | + | + | + |

| Chlorous Acid Concentration | | Time of Contact | | | |
|---|---|---|---|---|---|
| % | ppm | 1minute | 5minutes | 10minutes | 15minutes |
| 0.015% | 150ppm | − | − | − | − |
| 0.010% | 100ppm | + | − | − | − |
| 0.008% | 80ppm | + | + | − | − |
| 0.007% | 70ppm | + | + | + | + |
| 0.006% | 60ppm | + | + | + | + |
| 0.005% | 50ppm | + | + | + | + |
| 0.004% | 40ppm | + | + | + | + |
| 0.003% | 30ppm | + | + | + | + |
| 0.002% | 20ppm | + | + | + | + |
| 0.001% | 10ppm | + | + | + | + |
| 0.000% | 0ppm | + | + | + | + |

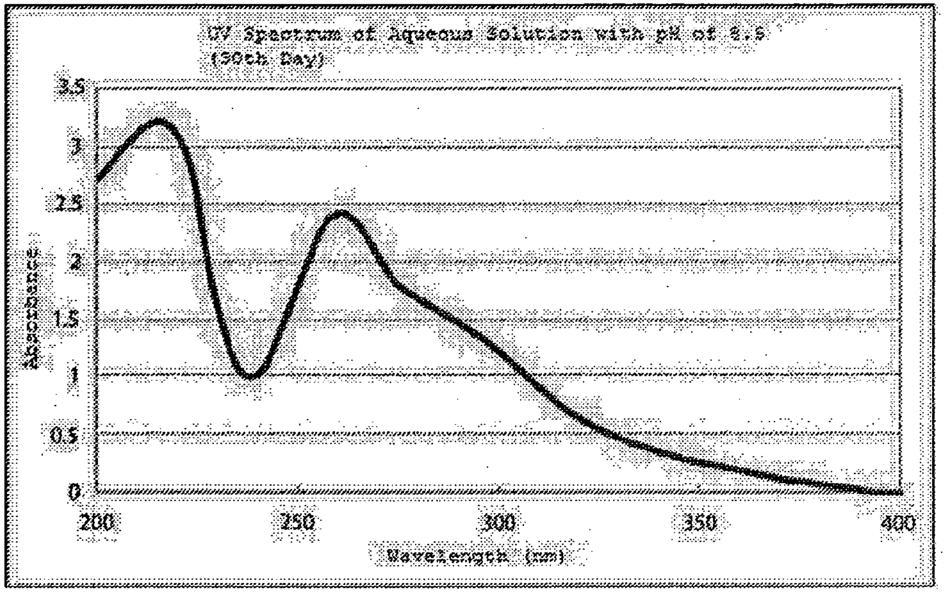

# Fig. 12

Immediately After Manufacture

| pH | Phenol Coefficient |
|---|---|
| 2.0 | 300 |
| 2.5 | 300 |
| 3.0 | 310 |
| 3.5 | 320 |
| 4.0 | 340 |
| 4.5 | 360 |
| 5.0 | 380 |
| 5.5 | 400 |
| 6.0 | 380 |
| 6.5 | 320 |
| 7.0 | 280 |
| 7.5 | 280 |
| 8.0 | 200 |
| 8.5 | 150 |
| 9.0 | 100 |

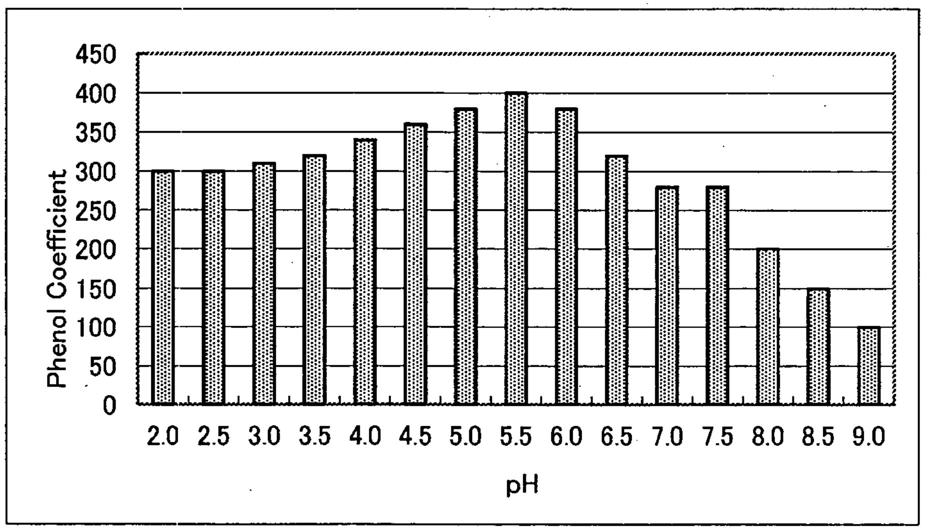


**Effect of pH on Phenol Coefficient**

10th Day

| pH | Phenol Coefficient |
|---|---|
| 2.0 | 0 |
| 2.5 | 0 |
| 3.0 | 0 |
| 3.5 | 100 |
| 4.0 | 180 |
| 4.5 | 320.4 |
| 5.0 | 342 |
| 5.5 | 360 |
| 6.0 | 342 |
| 6.5 | 320 |
| 7.0 | 266 |
| 7.5 | 266 |
| 8.0 | 168 |
| 8.5 | 120 |
| 9.0 | 100 |

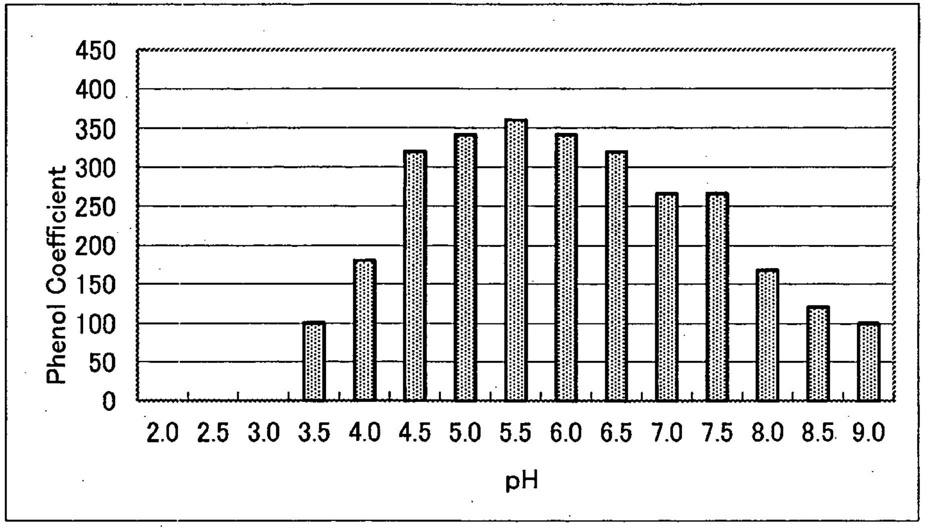


Effect of pH on Phenol Coefficient

30th Day

| pH | Phenol Coefficient |
|---|---|
| 2.0 | 0 |
| 2.5 | 0 |
| 3.0 | 0 |
| 3.5 | 50 |
| 4.0 | 100 |
| 4.5 | 210 |
| 5.0 | 334 |
| 5.5 | 352 |
| 6.0 | 320 |
| 6.5 | 273 |
| 7.0 | 266 |
| 7.5 | 266 |
| 8.0 | 130 |
| 8.5 | 120 |
| 9.0 | 100 |

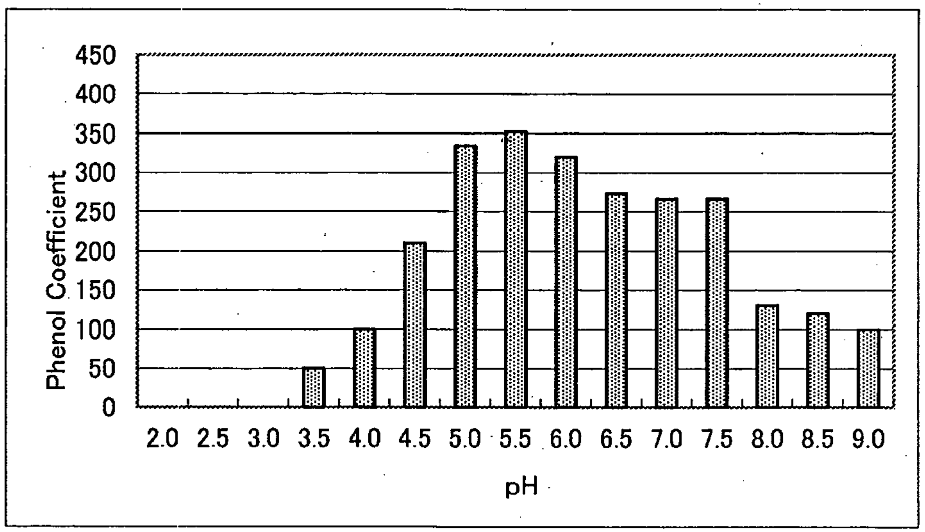


Effect of pH on Phenol Coefficient

Fig.13

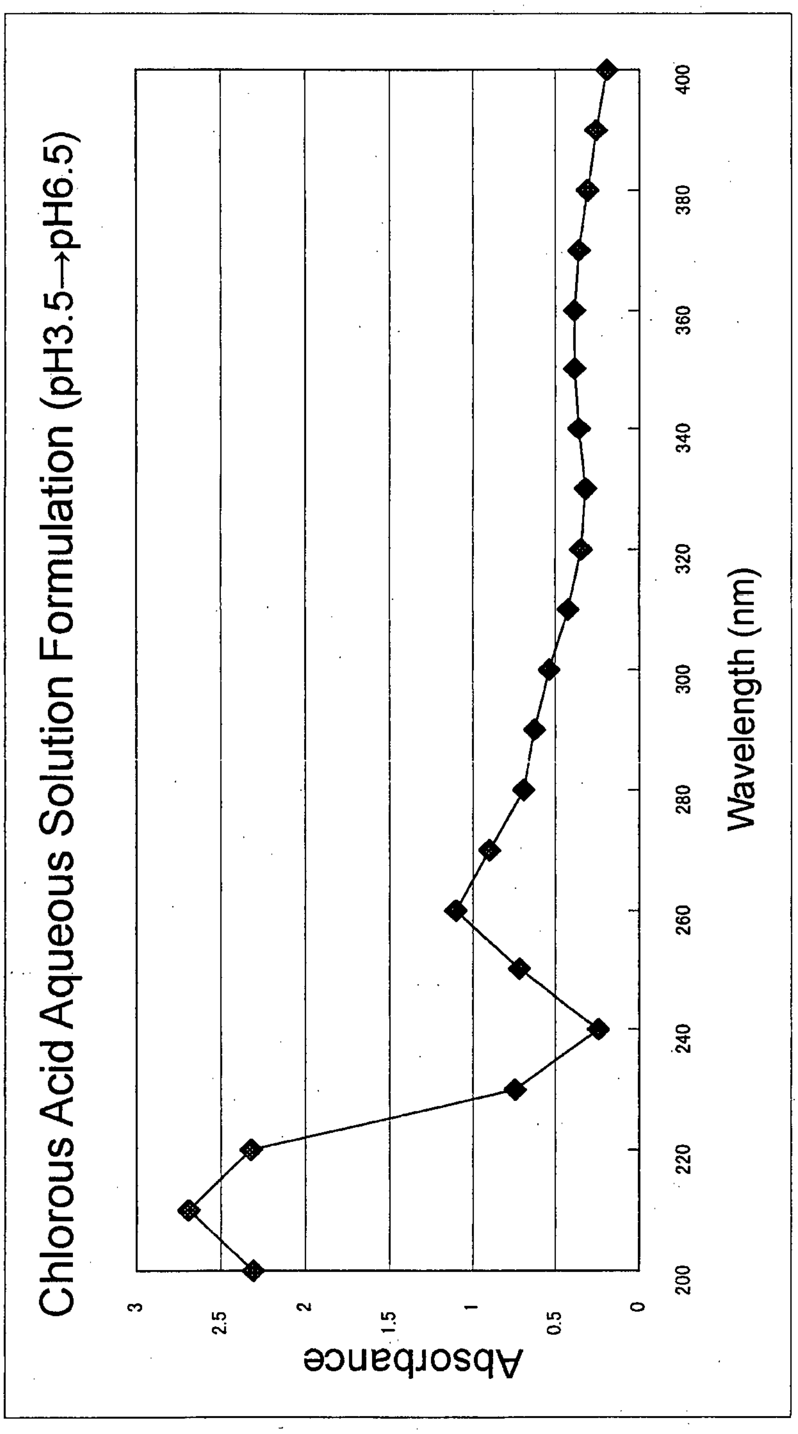

Chlorous Acid Aqueous Solution Formulation (pH3.5→pH6.5)
Absorbance
0
0.5
1
1.5
2
2.5
3
200
220
240
260
280
300
320
340
360
380
400
Wavelength (nm)

Fig.14

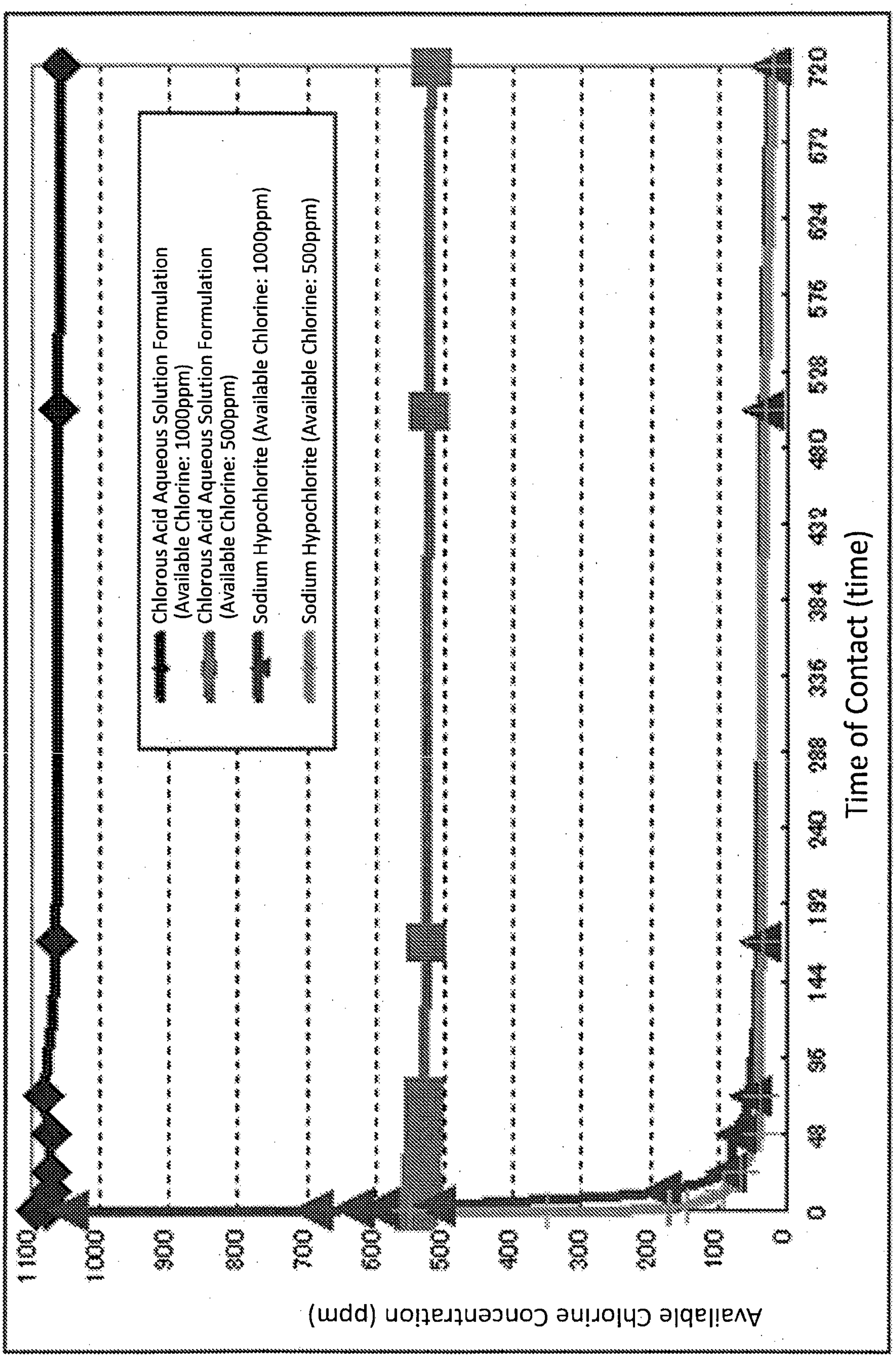

Available Chlorine Concentration (ppm)
1100
1000
900
800
700
600
500
400
300
200
100
0
0
48
96
144
192
240
288
336
384
432
480
528
576
624
672
720
Time of Contact (time)
Chlorous Acid Aqueous Solution Formulation (Available Chlorine: 1000ppm)
Chlorous Acid Aqueous Solution Formulation (Available Chlorine: 500ppm)
Sodium Hypochlorite (Available Chlorine: 1000ppm)
Sodium Hypochlorite (Available Chlorine: 500ppm)

[Fig. 15]

Fig. 15

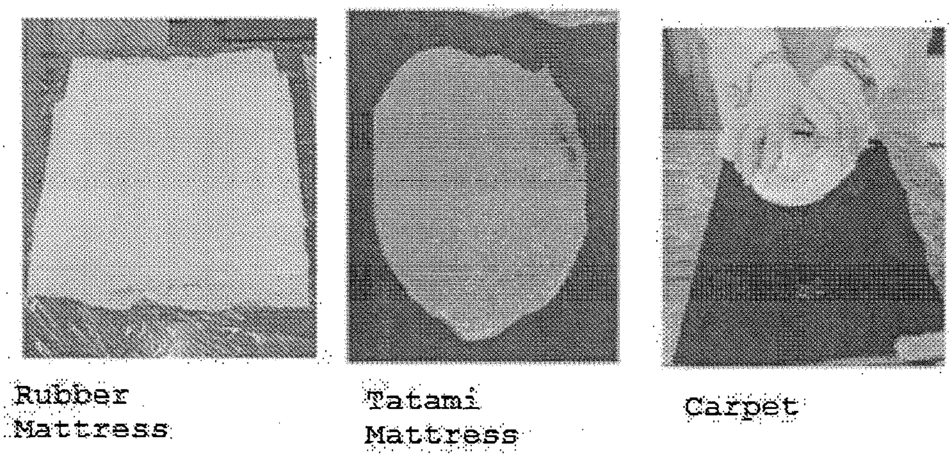


[Fig. 16]

Fig. 16

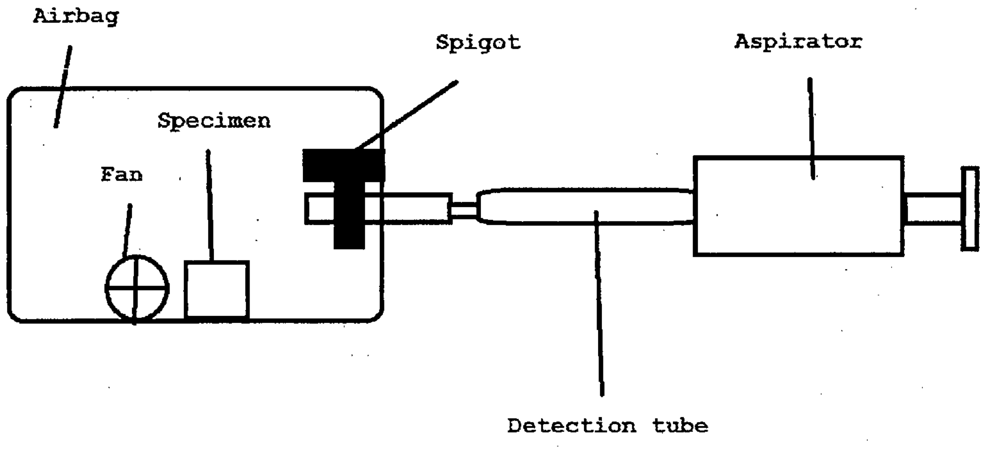

Fig.17

Fig. 17

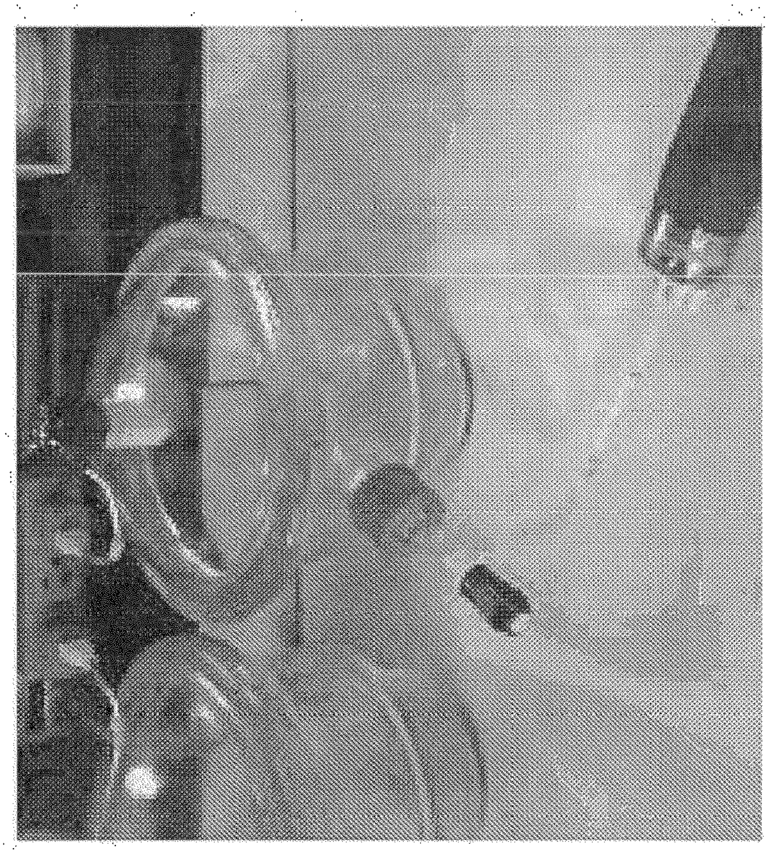

Fig.18

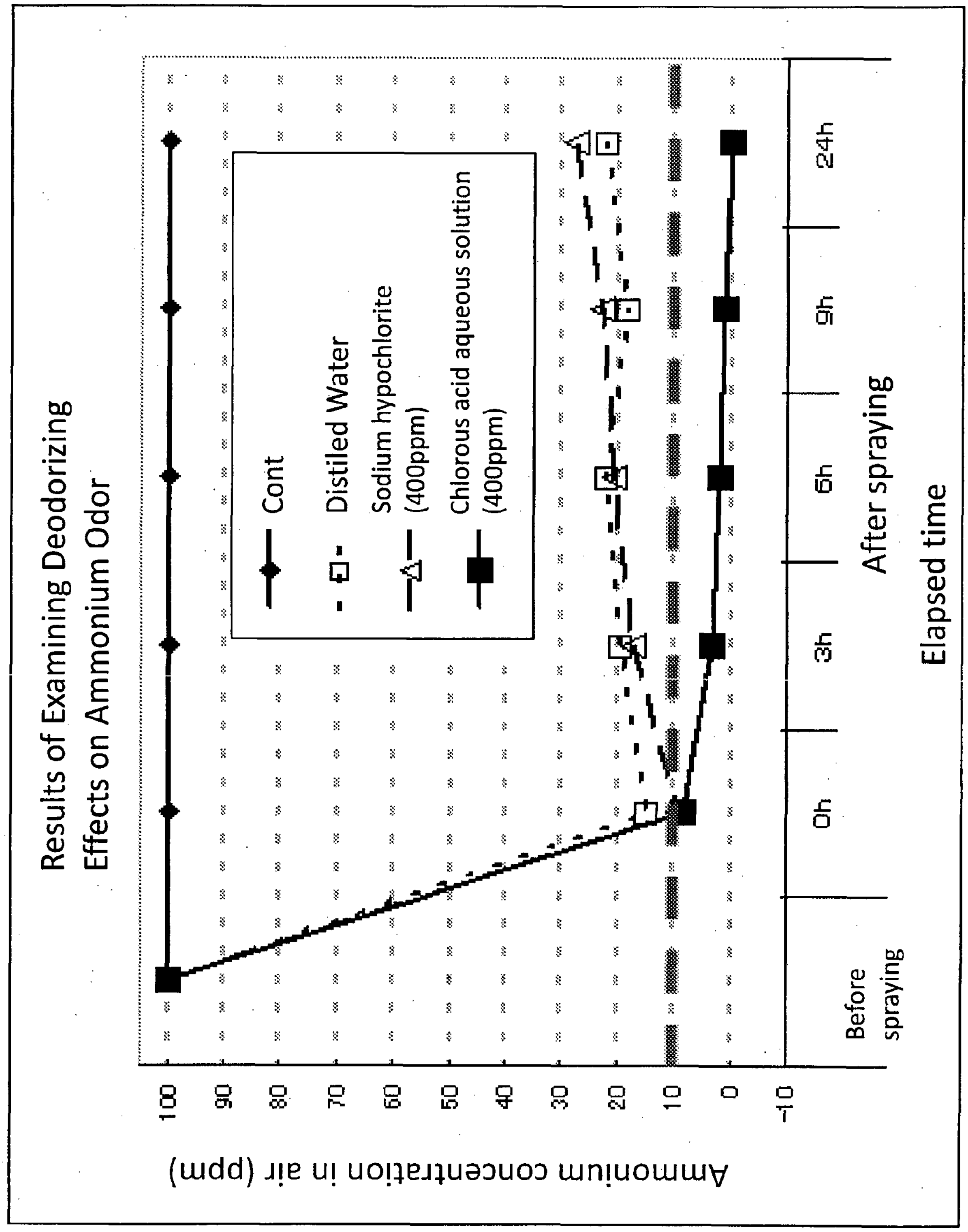
Results of Examining Deodorizing
Effects on Ammonium Odor
Cont
Distiled Water
Sodium hypochlorite
(400ppm)
Chlorous acid aqueous solution
(400ppm)
Ammonium concentration in air (ppm)
100
90
80
70
60
50
40
30
20
10
0
-10
Before
spraying
0h
3h
6h
9h
24h
After spraying
Elapsed time

Fig.19

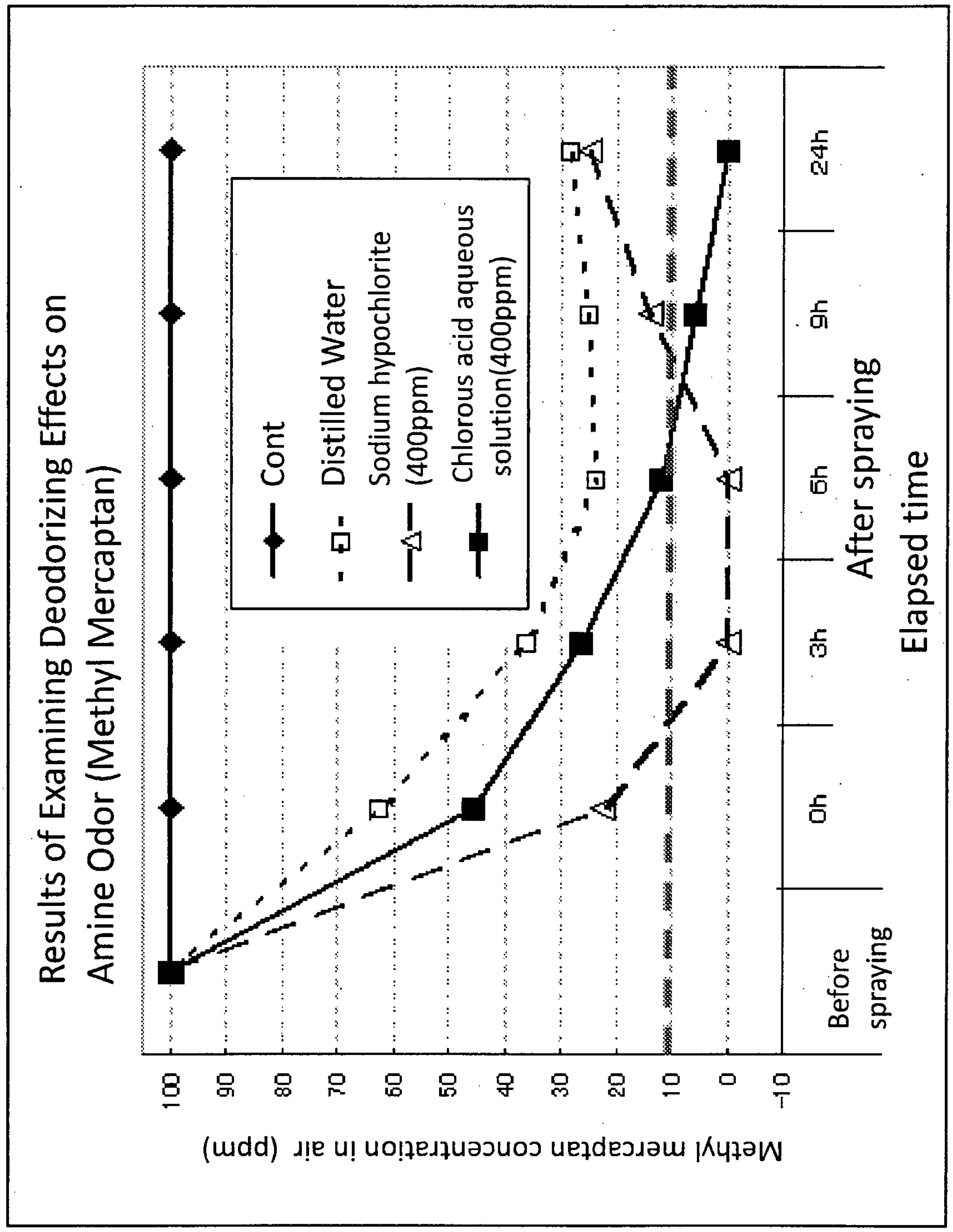
Results of Examining Deodorizing Effects on Amine Odor (Methyl Mercaptan)
Cont
Distilled Water
Sodium hypochlorite (400ppm)
Chlorous acid aqueous solution(400ppm)
Methyl mercaptan concentration in air (ppm)
100
90
80
70
60
50
40
30
20
10
0
-10
Before spraying
0h
3h
6h
9h
24h
After spraying
Elapsed time

Fig.20

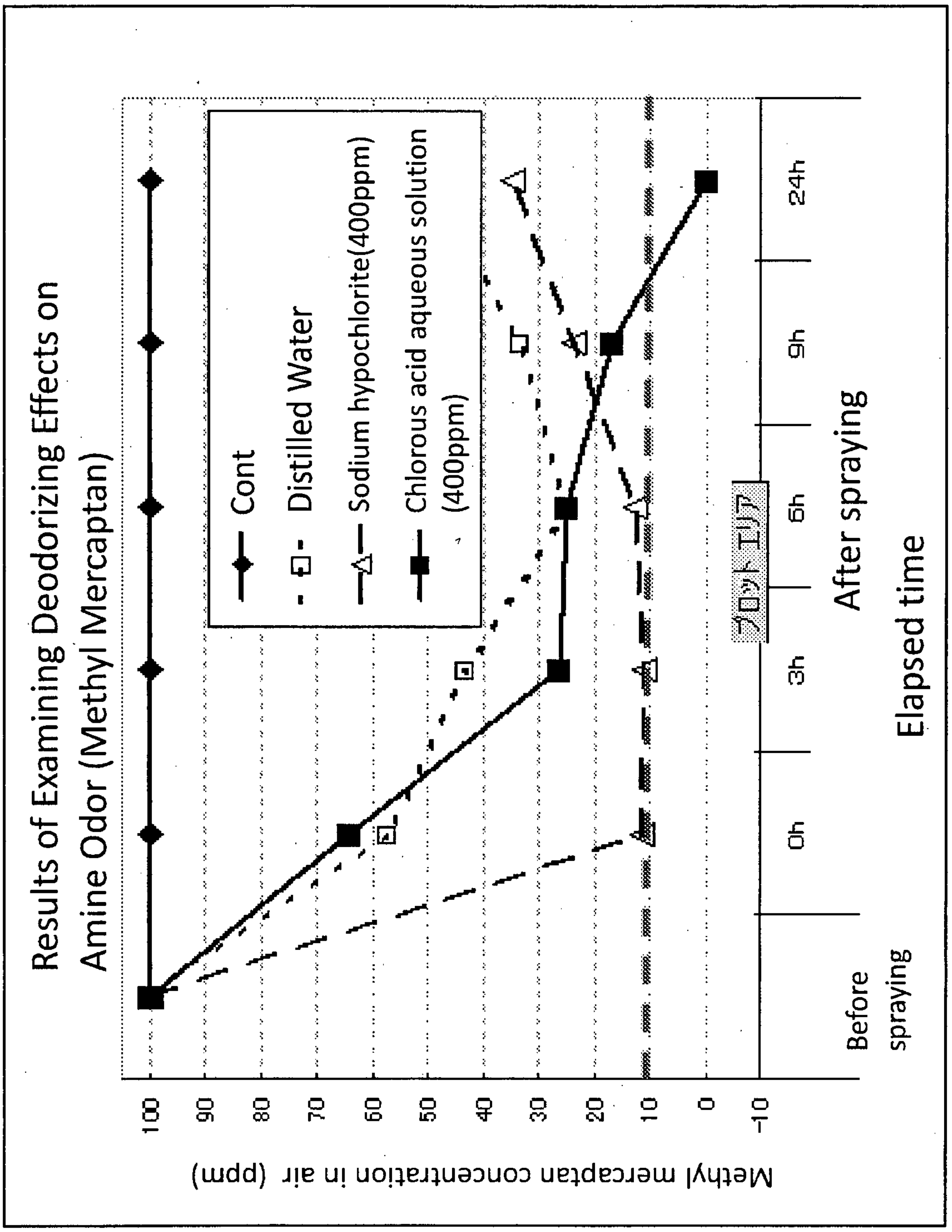
Results of Examining Deodorizing Effects on
Amine Odor (Methyl Mercaptan)
Cont
Distilled Water
Sodium hypochlorite(400ppm)
Chlorous acid aqueous solution
(400ppm)
Methyl mercaptan concentration in air (ppm)
100
90
80
70
60
50
40
30
20
10
0
-10
Before
spraying
0h
3h
6h
9h
24h
After spraying
Elapsed time

Fig.21

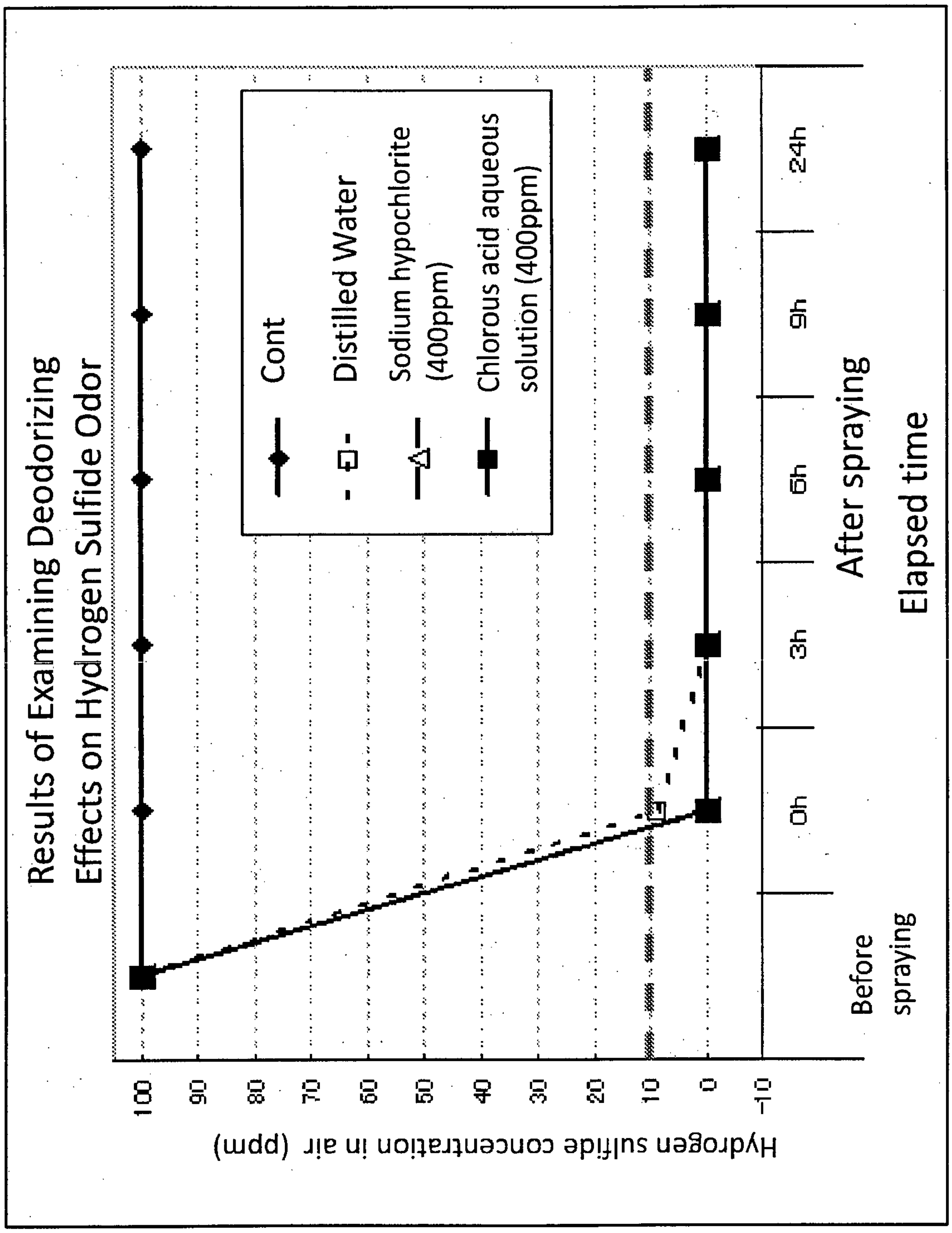
Results of Examining Deodorizing
Effects on Hydrogen Sulfide Odor
Cont
Distilled Water
Sodium hypochlorite
(400ppm)
Chlorous acid aqueous
solution (400ppm)
Hydrogen sulfide concentration in air (ppm)
100
90
80
70
60
50
40
30
20
10
0
-10
Before
spraying
0h
3h
6h
9h
24h
After spraying
Elapsed time

Fig.22

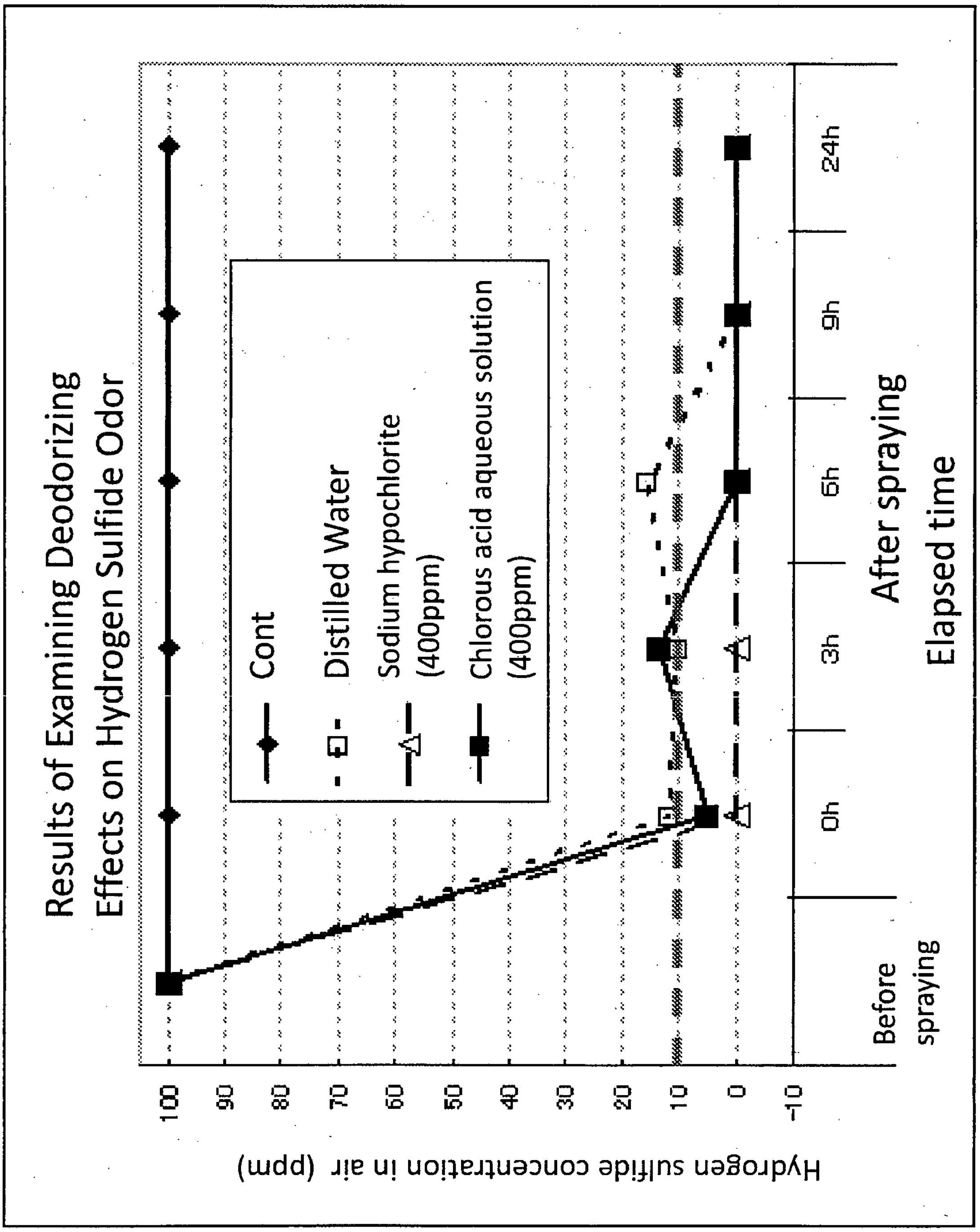
Results of Examining Deodorizing Effects on Hydrogen Sulfide Odor
Cont
Distilled Water
Sodium hypochlorite (400ppm)
Chlorous acid aqueous solution (400ppm)
Hydrogen sulfide concentration in air (ppm)
100
90
80
70
60
50
40
30
20
10
0
-10
Before spraying
0h
3h
6h
9h
24h
After spraying
Elapsed time

Europäisches Patentamt
European Patent Office
Office européen des brevets

# EUROPEAN SEARCH REPORT

Application Number
EP 16 00 1593

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | J. SCHIEL: "Ueber die chlorige Säure", JUSTUS LIEBIGS ANNALEN DER CHEMIE, vol. 109, no. 3, 1 January 1859 (1859-01-01), pages 317-325, XP055319625, WEINHEIM; DE ISSN: 0075-4617, DOI: 10.1002/jlac.18591090312 * page 319, last paragraph - page 320, paragraph first * ----- | 1-10 | INV. A61L2/18 A01N25/00 |
| A | US 2010/178235 A1 (GUPTA AMIT [US] ET AL) 15 July 2010 (2010-07-15) * the whole document * ----- | 1-10 | |
| A | US 2 071 091 A (TAYLOR MAURICE C) 16 February 1937 (1937-02-16) * column 1, line 43 - line 52; claims 1, 5 * * column 2, line 1 - line 4 * ----- | 1-10 | |
| A | EP 2 062 477 A1 (HONBU SANKEI CO LTD [JP]) 27 May 2009 (2009-05-27) * claims 1, 7-9 * ----- | 1-10 | TECHNICAL FIELDS SEARCHED (IPC) A61L |
| A | EP 0 749 275 A1 (ALCIDE CORP [US]) 27 December 1996 (1996-12-27) * paragraph [0021]; claim 1 * ----- | 1-10 | |

The present search report has been drawn up for all claims

1

| Place of search | Date of completion of the search | Examiner |
|---|---|---|
| Munich | 15 November 2016 | Kukolka, Florian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 00 1593

This annex lists the patent family members relating to the patent documents cited in the above-mentioned European search report. The members are as contained in the European Patent Office EDP file on
The European Patent Office is in no way liable for these particulars which are merely given for the purpose of information.

15-11-2016

| Patent document cited in search report | Publication date | Patent family member(s) | Publication date |
|---|---|---|---|
| US 2010178235 A1 | 15-07-2010 | US 2010178235 A1 | 15-07-2010 |
| | | WO 2010083070 A1 | 22-07-2010 |
| US 2071091 A | 16-02-1937 | NONE | |
| EP 2062477 A1 | 27-05-2009 | AU 2007289722 A1 | 06-03-2008 |
| | | CA 2662288 A1 | 06-03-2008 |
| | | CN 101511192 A | 19-08-2009 |
| | | CN 103314996 A | 25-09-2013 |
| | | EP 2062477 A1 | 27-05-2009 |
| | | EP 2633757 A2 | 04-09-2013 |
| | | EP 2984931 A1 | 17-02-2016 |
| | | ES 2583988 T3 | 23-09-2016 |
| | | HK 1134220 A1 | 22-11-2013 |
| | | IL 197328 A | 28-05-2014 |
| | | IL 226395 A | 31-08-2014 |
| | | JP 5201555 B2 | 05-06-2013 |
| | | JP 5823422 B2 | 25-11-2015 |
| | | JP 2013100346 A | 23-05-2013 |
| | | JP 2015145418 A | 13-08-2015 |
| | | KR 20090051233 A | 21-05-2009 |
| | | NZ 575871 A | 31-03-2011 |
| | | US 2010330202 A1 | 30-12-2010 |
| | | US 2013302438 A1 | 14-11-2013 |
| | | US 2015093482 A1 | 02-04-2015 |
| | | US 2015313214 A1 | 05-11-2015 |
| | | WO 2008026607 A1 | 06-03-2008 |
| EP 0749275 A1 | 27-12-1996 | AT 206013 T | 15-10-2001 |
| | | AU 7399494 A | 20-02-1995 |
| | | DE 69428458 D1 | 31-10-2001 |
| | | DE 69428458 T2 | 18-04-2002 |
| | | EP 0749275 A1 | 27-12-1996 |
| | | ES 2160632 T3 | 16-11-2001 |
| | | US 5389390 A | 14-02-1995 |
| | | WO 9502965 A1 | 02-02-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008026607 A